(19) Europäisches Patentamt
European Patent Office
Office européen des brevets

(11) EP 1 686 173 A1

(12) # EUROPEAN PATENT APPLICATION
published in accordance with Art. 158(3) EPC

(43) Date of publication:
**02.08.2006 Bulletin 2006/31**

(21) Application number: **04773766.3**

(22) Date of filing: **08.10.2004**

(51) Int Cl.:
***C12N 5/10*** *(2006.01)* ***C12N 15/13*** *(2006.01)*
***C12P 21/08*** *(2006.01)*

(86) International application number:
**PCT/JP2004/015315**

(87) International publication number:
**WO 2005/035740 (21.04.2005 Gazette 2005/16)**

(84) Designated Contracting States:
**AT BE BG CH CY CZ DE DK EE ES FI FR GB GR HU IE IT LI LU MC NL PL PT RO SE SI SK TR**

(30) Priority: **09.10.2003 JP 2003350166**

(71) Applicant: **KYOWA HAKKO KOGYO CO., LTD.**
**Tokyo 100-8185 (JP)**

(72) Inventors:
- **NAKANO, Ryosuke,**
  **c/o Kyowa Hakko Kogyo Co.,Ltd.**
  **Tokyo 100-8185 (JP)**
- **SATOH, Mitsuo,**
  **c/o Kyowa Hakko Kogyo Co.,Ltd.**
  **Tokyo 100-8185 (JP)**
- **IIDA, Shigeru,**
  **c/o Kyowa Hakko Kogyo Co.,Ltd.**
  **Tokyo 100-8185 (JP)**
- **INOUE, Miho,**
  **c/o Kyowa Hakko Kogyo Co.,Ltd.**
  **Tokyo 100-8185 (JP)**
- **KUSUNOKI, Machi,**
  **c/o Kyowa Hakko Kogyo Co.,Ltd.**
  **Tokyo 100-8185 (JP)**
- **KINOSHITA, Satoko,**
  **c/o Kyowa Hakko Kogyo Co.,Ltd.**
  **Tokyo 100-8185 (JP)**
- **OHNUKI, Naoko,**
  **c/o Kyowa Hakko Kogyo Co.,Ltd.**
  **Tokyo 100-8185 (JP)**

(74) Representative: **Vossius & Partner**
**Siebertstrasse 4**
**81675 München (DE)**

(54) # GENOMICALLY MODIFIED CELL NEUTRALIZED TO SERUM-FREE SYSTEM

(57) Development of a host cell capable of producing a glycoprotein composition such as an antibody composition which is useful in development of medicaments is desired.

The present invention provides a cell in which a genomic gene encoding an enzyme relating to a sugar chain modification in which 1-position of fucose is bound to 6-position of N-acetylglucosamine in the reducing end through α-bond in a complex type N-glycoside-linked sugar chain is knocked out, wherein the cell is naturalized in a serum-free medium and a process for producing a glycoprotein composition using the cell.

EP 1 686 173 A1

Printed by Jouve, 75001 PARIS (FR)

**Description**

Technical Field

The present invention relates to a cell in which a genomic gene encoding an enzyme relating to a sugar chain modification in which 1-position of fucose is bound to 6-position of N-acetylglucosamine in the reducing end through α-bond in a complex type N-glycoside-linked sugar chain is knocked out, wherein the cell is naturalized in a medium containing no serum (hereinafter referred to as "serum-free medium"), and a process for producing a glycoprotein composition, such as an antibody, by using the cell.

Background Art

Hanai *et al.* have reported that addition of fucose to N-acetylglucosamine in the reducing end in N-glycoside-linked sugar chains of an antibody decreases antibody-dependent cellular cytotoxic activity (ADCC activity) of the antibody to fiftieth [WO00/61739, *J. Biol. Chem.,* 278, 3466 (2003)]. These reports show that the sugar chain structure plays a considerably important role in the effector function of human IgG1 subclass antibodies, and that a change in the sugar chain structure results in a change in the pharmacological activity relating to the effector function.

In general, most of glycoproteins including antibodies applicable to medicaments are prepared by using genetic recombination techniques, and are produced by using, as a host cell, an animal cell such as Chinese hamster ovary tissue-derived CHO cell. However, since sugar chain structures of expressed glycoproteins are different depending on the host cell, a sugar chain having the most suitable pharmacological activity is not always added to the glycoprotein under the present situation.

Particularly, when addition of fucose to N-acetylglucosamine in the reducing end in the N-glycoside-linked sugar chain greatly reduces the pharmacological activity like the case of an antibody, it is essential to properly prepare and provide an antibody molecule having a sugar chain structure which is not modified with fucose for the purpose of providing patients with a high quality medical treatment. Accordingly, it has been desired to develop techniques for controlling the sugar chain structure of a glycoprotein.

The sugar chain structure of a glycoprotein is regulated by sugar chain genes which are genes encoding a glycosyltransferase which synthesizes the sugar chain and a glycolytic enzyme which degrades the sugar chain. Also, it is regulated by genes encoding proteins capable of carrying out functions such as biosynthesis of an intracellular sugar nucleotide which becomes the donor of a saccharide to the sugar chain and transfer thereof to the Golgi body. A possibility has been shown that the sugar chain structure of a glycoprotein produced by the host cell can be controlled by introducing a gene relating to the modification of these sugar chains into a host cell, or mutating them.

Attempts have been made to modify the sugar chain structure of a produced glycoprotein by introducing a gene encoding an enzyme relating to the modification of a sugar chain. Specifically, it has been reported that 1) it is possible to produce a protein in which sialic acid is added in large numbers to the non-reducing end of its sugar chain by introducing rat β-galactoside α2,6-sialyltransferase into CHO cell [*J. Biol. Chem.,* 261, 13848 (1989)], 2) it is possible to express an H antigen (Fuc α1-2Gal β1-) in which fucose (hereinafter referred also to as Fuc) is added to the non-reducing end of its sugar chain, by introducing human β-galactoside 2-α-fucosyltransferase into mouse L cell *[Science,* 252, 1668 (1991)], and 3) it is possible to produce an antibody having a high addition ratio ofN-acetylglucosamine at the bisecting ofN-glycoside-linked sugar chain by producing the antibody using CHO cell into which β1,4-N-acetylglucosamine transferase III (GnTIII) is introduced [*Glycobiology*, 5, 813 (1995), WO99/54342]. It has been reported that, when an antibody was expressed using CHO cell into which GnTIII was introduced, it showed 16 times higher ADCC activity than the antibody expressed by the parent cell line. However, over-expression of GnTIII or β1,4-N-acetylglucosamine transferase V (GnTV) showed toxicity upon CHO cell.

The mutants in which the activity of a gene relating to the modification of a sugar chain is changed have been obtained, for example, as clones showing resistance to a lectin such as WGA (wheat-germ agglutinin derived from *T. vulgaris),* ConA (concanavalin A derived from *C. ensiformis),* RIC (a toxin derived from *R communis),* L-PHA (leukoagglutinin derived from *P. vulgaris),* LCA (lentil agglutinin derived from *L. culinaris*), PSA (pea lectin derived from *P. sativum*) or the like [*Somatic Cell Mol. Genet*., 12, 51 (1986)]. A case has been reported in which a glycoprotein in which a produced sugar chain structure is changed is produced by using such a mutant, as the host cell, in which the activity of a gene relating to the modification of a sugar chain was changed. Examples include a report on the production of an antibody having a high mannose type sugar chain structure using a CHO cell mutant clone in which the activity of N-acetylglucosamine transferase I (GnTI) was deleted [*J. Immunol*., 160, 3393 (1998)].

In addition, a case has been reported on expression of an antibody having a sugar chain structure in which sialic acid is not added to the non-reducing end in the sugar chains or expression of an antibody without addition of galactose thereto, using a CMP-sialic acid transporter- or UDP-galactose transporter-deficient clone, but expression of an antibody having effector activity improved to a degree suitable for appliccation to a medicament has not been succeeded [*J. Immunol.,* 160, 3393 (1998)].

Under such a situation, it has been reported that an antibody having high ADCC activity and is highly suitable for application to a medicament can be produced by using, as the host cell, a clone having decreased activity of GDP-mannose 4,6-dehydratase, an enzyme capable of catalyzing a dehydration reaction to convert GDP-mannose into GDP-4-keto,6-deoxy-GDP-mannose [WO00/61739; *J. Biol. Chem.*, 277, 26733 (2002)]. In these reports, as the host cell, a lectin-resistant clone which can recognize a sugar chain structure in which 1-position of fucose is bound to 6-position of N-acetylglucosamine in the reducing end in complex type N-glycoside-linked sugar chains through α-bond, such as clone CHO-AAL which is resistant to AAL (a lectin derived from *Aleuria aurantia*), clone CHO-LCA which is resistant to LCA (lentil agglutinin derived from *L. culinaris*) or clone Lec 13 is used as the host cell. In addition to these, $PL^R1.3$ established as a PSA (pea lectin derived from *P. sativum*) resistant mutant of a mouse leukemia-derived clone BW 5147 is also known as a clone having decreased activity of GDP-mannose 4,6-dehydratase [*J. Biol. Chem.,* 255, 9900 (1980)].

However, since each of these clones is not a complete gene deficient clone, it is difficult to completely make a sugar chain structure, which is a cause of showing high ADCC activity by the antibody. Namely it is difficult to completely inhibit addition of fucose to the N-acetylglucosamine in the reducing end in complex type N-glycoside-linked sugar chains. Particularly, since mutant clones such as $PL^R1.3$ and Lec13 are obtained by randomly introducing mutation through a mutagen treatment, it cannot always be said that they have properties suited as clones to be used in the production of pharmaceutical preparations. On the other hand, there has been no reports in which attempts to produce a glycoprotein such as an antibody using a clone prepared by disrupting a target gene alone on purpose, wherein an enzyme gene concerned in the sugar chain modification of the host cell is used as the target were made.

In addition, when serum is present in the culture medium in producing a physiologically active protein such as a glycoprotein by an animal cell or a recombinant animal cell, the difference in serum lots exerts serious influence upon the cell yield and productivity, and it is necessary to take into consideration a possibility of causing contamination of the final purified product with pathogenic microorganism such as virus or prion. Accordingly, when a physiologically active protein is produced using an animal cell in order to apply it to a medicament, it is preferable to culture the cell using a medium containing no serum.

Disclosure of the Invention

An object of the present invention is to provide a cell in which a genomic gene relating to a sugar chain modification in which 1-position of fucose is bound to 6-position of N-acetylglucosamine in the reducing end through α-bond in a complex type N-glycoside-linked sugar chain is knocked out, wherein the cell is naturalized in a medium containing no serum; a process for producing a glycoprotein composition using the cell; and a glycoprotein composition produced by the process. The cell of the present invention is useful for producing a glycoprotein composition such as an antibody composition which has a modified sugar chain structure and is useful in pharmaceutical development.

The present invention relates to the following (1) to (27):

(1) A cell in which a genomic gene encoding an enzyme relating to a sugar chain modification in which 1-position of fucose is bound to 6-position of N-acetylglucosamine in the reducing end through α-bond in a complex type N-glycoside-linked sugar chain is knocked out, wherein the cell is naturalized in a serum-free medium.

(2) The cell according to the above (1), wherein all of alleles on a genome encoding an enzyme relating to modification of a sugar chain in which 1-position of fucose is bound to 6-position of N-acetylglucosamine in the reducing end through α-bond in a complex type N-glycoside-linked sugar chain are knocked out, and wherein the cell is naturalized in a serum-free medium.

(3) The cell according to the above (1) or (2), wherein an exon region containing an initiation codon of the genomic gene encoding an enzyme relating to modification of a sugar chain in which 1-position of fucose is bound to 6-position ofN-acetylglucosamine in the reducing end through α-bond in a complex type N-glycoside-linked sugar chain is deleted, and wherein the cell is naturalized in a serum-free medium.

(4) The cell according to any one of the above (1) to (3), wherein the enzyme relating to modification of a sugar chain in which 1-position of fucose is bound to 6-position of N-acetylglucosamine in the reducing end through α-bond in a complex type N-glycoside-linked sugar chain is α-1,6-fucosyltransferase.

(5) The cell according to the above (4), wherein the α-1,6-fucosyltransferase is a protein encoded by a DNA selected from the following (a) or (b):

(a) a DNA comprising the nucleotide sequence represented by SEQ ID NO:1;

(b) a DNA which hybridizes with a DNA consisting of the nucleotide sequence represented by SEQ ID NO:1 under stringent conditions and encodes a protein having α-1,6-fucosyltransferase activity.

(6) The cell according to the above (4), wherein the α-1,6-fucosyltransferase is a protein selected from the group

consisting of the following (a), (b) and (c):

(a) a protein comprising the amino acid sequence represented by SEQ ID NO:5;
(b) a protein consisting of an amino acid sequence in which one or more amino acid(s) is/are deleted, substituted, inserted and/or added in the amino acid sequence represented by SEQ ID NO:5 and having α-1,6-fucosyltransferase activity;
(c) a protein consisting of an amino acid sequence which has at least 80% amino acid sequence homology to the amino acid sequence represented by SEQ ID NO:5 and having α-1,6-fucosyltransferase activity.

(7) The cell according to any one of the above (1) to (6), which is resistant to a lectin which recognizes a sugar chain structure in which 1-position of fucose is bound to 6-position of N-acetylglucosamine in the reducing end through α-bond in a complex type N-glycoside-linked sugar chain.
(8) The cell according to the above (7), wherein said resistance is resistance in which the cell survives at a higher ratio than a cell in which the genomic gene has not been knocked out when the cells are cultured in a medium containing the lectin which recognizes a sugar chain structure in which 1-position of fucose is bound to 6-position of N-acetylglucosamine in the reducing end through α-bond in a complex type N-glycoside-linked sugar chain.
(9) The cell according to any one of the above (1) to (7), wherein the serum-free medium is a protein-free medium.
(10) The cell according to any one of the above (1) to (9), which comprises a gene encoding a glycoprotein.
(11) The cell according to the above (10), wherein the glycoprotein is a glycoprotein having no sugar chain structure in which 1-position of fucose is bound to 6-position of N-acetylglucosamine in the reducing end through α-bond in a complex type N-glycoside-linked sugar chain.
(12) The cell according to the above (10) or (11), wherein the glycoprotein is an antibody.
(13) The cell according to the above (12), wherein the antibody belongs to an IgG class.
(14) A process for producing a glycoprotein composition, which comprises using the cell according to any one of the above (1) to (13).
(15) A process for producing a glycoprotein composition, which comprises culturing the cell according to any one of the above (1) to (13) in a medium to form and accumulate the glycoprotein composition in the culture, and recovering and purifying the glycoprotein composition from the culture.
(16) The process for producing a glycoprotein composition according to the above (14) or (15), wherein the process is carried out by batch culture, fed-batch culture or perfusion culture.
(17) The process according to any one of the above (14) to (16), wherein at least one selected from a nutrient factor and a physiologically active substance is added to the medium during culturing.
(18) The process according to the above (17), wherein the nutrient factor is at least one selected from a glucose, an amino acid and a vitamin.
(19) The process according to the above (17), wherein the physiologically active substance is at least one selected from an insulin, an insulin-like growth factor, transferrin and albumin.
(20) The process according to any one of the above (14) to (19), wherein the glycoprotein composition is an antibody composition.
(21) A method for naturalizing a cell in which a genomic gene encoding an enzyme relating to a sugar chain modification in which 1-position of fucose is bound to 6-position of N-acetylglucosamine in the reducing end through α-bond in a complex type N-glycoside-linked sugar chain is knocked out in a seruin-free medium, which comprises inoculating the cell into a medium for naturalization to give a cell density of $1\times10^5$ to $1\times10^6$ cells/ml.
(22) A method for obtaining a clone in which a genomic gene encoding an enzyme relating to a sugar chain modification in which 1-position of fucose is bound to 6-position of N-acetylglucosamine in the reducing end through α-bond in a complex type N-glycoside-linked sugar chain is knocked out, which comprises naturalizing the cell in a serum-free medium by the method according to the above (21), and then cloning the cell.
(23) A cell in which a genomic gene encoding an enzyme relating to a sugar chain modification in which 1-position of fucose is bound to 6-position of N-acetylglucosamine in the reducing end through α-bond in a complex type N-glycoside-linked sugar chain is knocked out, wherein the cell is naturalized in a serum-free medium, which is obtainable by the method according to the above (21).
(24) A clone in which a genomic gene encoding an enzyme relating to a sugar chain modification in which 1-position of fucose is bound to 6-position of N-acetylglucosamine in the reducing end through α-bond in a complex type N-glycoside-linked sugar chain is knocked out, wherein the clone is naturalized in a serum-free medium, which is obtainable by the method according to the above (22).
(25) The method according to the above (21) or (22), wherein the serum-free medium is a protein-free medium,
(26) The cell according to the above (23), wherein the serum-free medium is a protein-free medium.
(27) The clone according to the above (24), wherein the serum-free medium is a protein-free medium.

The present invention is described below in detail. This application is based on the priority of Japanese patent application No. 2003-350166 filed on October 9, 2003, and the entire contents of the specification and the drawings in the patent application are incorporated hereinto by reference.

The cell of the present invention in which a genomic gene encoding an enzyme relating to a sugar chain modification in which 1-position of fucose is bound to 6-position of N-acetylglucosamine in the reducing end through α-bond in a complex type N-glycoside-linked sugar chain is knocked out, wherein the cell is naturalized in a serum-free medium (hereinafter referred to as the cell of the present invention) includes a cell in which a genomic gene is modified so as to delete the activity of an enzyme relating to a sugar chain modification in which 1-position of fucose is bound to 6-position of N-acetylglucosamine in the reducing end through α-bond in a complex type N-glycoside-linked sugar chain (hereinafter referred to as the α-1,6-fucose modifying enzyme).

In the present invention, modification of genome so as to delete the activity of the α-1,6-fucose modifying enzyme means that mutation is introduced into an expression-controlling region of the gene so as to delete the expression of the enzyme, or that mutation is introduced into an amino acid sequence of the gene so as to delete the function of the enzyme. Introduction of the mutation means that modification of a nucleotide sequence such as deletion, substitution, insertion and/or addition is carried out in the nucleotide sequence on the genome. Complete inhibition of the expression or function of the modified genomic gene is called "knocked out". Examples in which a genomic gene is knocked out include a case in which the gene as the target is completely or partially deleted from the genome. Specific examples include deletion of a genomic region of exon containing at least an initiation codon from the chromosome or deletion of all of alleles in the gene encoding the α-1,6-fucose modifying enzyme.

Accordingly, the cell of the present invention includes a cell in which all of alleles on a genome encoding the α-1,6-fucose modifying enzyme are knocked out or a cell in which an exon region containing at least an initiation codon of the enzyme, wherein the cell is naturalized in a serum-free medium.

The α-1,6-fucose modifying enzyme means an enzyme relating to a sugar chain modification in which 1-position of fucose is bound to 6-position of N-acetylglucosamine in the reducing end through α-bond in a complex type N-glycoside-linked sugar chain. The enzyme relating to a sugar chain modification in which 1-position of fucose is bound to 6-position of N-acetylglucosamine in the reducing end through α-bond in a complex type N-glycoside-linked sugar chain also includes an enzyme having influence on the reaction.

Specific examples of the α-1,6-fucose modifying enzyme include α-1,6-fucosyltransferase, α-L-fucosidase and the like.

Furthermore, the enzyme having influence on the reaction in which 1-position of fucose is bound to 6-position of N-acetylglucosamine in the reducing end through α-bond in a complex type N-glycoside-linked sugar chain also includes an enzyme having influence on the above α-1,6-fucose modifying enzyme activity or influence on structure of a substance which is used as a substrate of the enzyme.

In the present invention, the α-1,6-fucosyltransferase includes a protein encoded by a DNA of the following (a), (b), (c), (d), (e), (f), (g) or (h), a protein of the following (i), (j), (k), (l), (m), (n), (o), (p), (q), (r), (s) or (t), and the like:

(a) a DNA comprising the nucleotide sequence represented by SEQ ID NO:1;
(b) a DNA comprising the nucleotide sequence represented by SEQ ID NO:2;
(c) a DNA comprising the nucleotide sequence represented by SEQ ID NO:3;
(d) a DNA comprising the nucleotide sequence represented by SEQ ID NO:4;
(e) a DNA which hybridizes with a DNA consisting of the nucleotide sequence represented by SEQ ID NO: under stringent conditions and encodes a protein having α-1,6-fucosyltransferase activity;
(f) a DNA which hybridizes with a DNA consisting of the nucleotide sequence represented by SEQ ID NO:2 under stringent conditions and encodes a protein having α-1,6-fucosyltransferase activity;
(g) a DNA which hybridizes with a DNA consisting of the nucleotide sequence represented by SEQ ID NO:3 under stringent conditions and encodes a protein having α-1,6-fucosyltransferase activity;
(h) a DNA which hybridizes with a DNA consisting of the nucleotide sequence represented by SEQ ID NO:4 under stringent conditions and encodes a protein having α-1,6-fucosyltransferase activity;
(i) a protein comprising the amino acid sequence represented by SEQ ID NO:5;
(j) a protein comprising the amino acid sequence represented by SEQ ID NO:6;
(k) a protein comprising the amino acid sequence represented by SEQ ID NO:7;
(l) a protein comprising the amino acid sequence represented by SEQ ID NO:8;
(m) a protein consisting of an amino acid sequence in which one or more amino acid(s) is/are deleted, substituted, inserted and/or added in the amino acid sequence represented by SEQ ID NO:5 and having α-1,6-fucosyltransferase activity;
(n) a protein consisting of an amino acid sequence in which one or more amino acid(s) is/are deleted, substituted, inserted and/or added in the amino acid sequence represented by SEQ ID NO:6 and having α-1,6-fucosyltransferase activity;

(o) a protein consisting of an amino acid sequence in which one or more amino acid(s) is/are deleted, substituted, inserted and/or added in the amino acid sequence represented by SEQ ID NO:7 and having α-1,6-fucosyltransferase activity;
(p) a protein consisting of an amino acid sequence in which one or more amino acid(s) is/are deleted, substituted, inserted and/or added in the amino acid sequence represented by SEQ ID NO:8 and having α-1,6-fucosyltransferase activity;
(q) a protein consisting of an amino acid sequence which has 80% or more homology to the amino acid sequence represented by SEQ ID NO:5 and having α-1,6-fucosyltransferase activity;
(r) a protein consisting of an amino acid sequence which has 80% or more homology to the amino acid sequence represented by SEQ ID NO:6 and having α-1,6-fucosyltransferase activity;
(s) a protein consisting of an amino acid sequence which has 80% or more homology to the amino acid sequence represented by SEQ ID NO:7 and having α-1,6-fucosyltransferase activity;
(t) a protein consisting of an amino acid sequence which has 80% or more homology to the amino acid sequence represented by SEQ ID NO:8 and having α-1,6-fucosyltransferase activity.

Also, the DNA encoding the α-1,6-fucosyltransferase includes a DNA comprising the nucleotide sequence represented by SEQ ID NO:1, 2, 3 or 4 and a DNA which hybridizes with the DNA consisting of the nucleotide sequence represented by SEQ ID NO:1, 2, 3 or 4 under stringent conditions and encodes an amino acid sequence having α-1,6-fucosyltransferase activity.

In the present invention, a DNA which hybridizes under stringent conditions is a DNA obtained, e.g., by a method such as colony hybridization, plaque hybridization or Southern blot hybridization using a DNA consisting of the nucleotide sequence represented by SEQ ID NO:1, 2, 3 or 4 or a partial fragment thereof as the probe, and specifically includes a DNA which can be identified by carrying out hybridization at 65°C in the presence of 0.7 to 1.0 M sodium chloride using a filter to which colony- or plaque-derived DNAs are immobilized, and then washing the filter at 65°C using 0.1 to 2 × SSC solution (composition of the 1 × SSC solution comprising 150 mM sodium chloride and 15 mM sodium citrate). The hybridization can be carried out in accordance with the methods described, e.g., in *Molecular Cloning, A Laboratory Manual,* Second Edition, Cold Spring Harbor Laboratory Press (1989) (hereinafter referred to as *"Molecular Cloning,* Second Edition"), *Current Protocols in Molecular Biology,* John Wiley & Sons, 1987-1997 (hereinafter referred to as *"Current Protocols in Molecular Biology"); DNA Cloning 1: Core Techniques, A Practical Approach,* Second Edition, Oxford University (1995); and the like. The hybridizable DNA includes a DNA having at least 60% or more, preferably 70% or more, more preferably 80% or more, still more preferably 90% or more, far more preferably 95% or more, and most preferably 98% or more, of homology with the nucleotide sequence represented by SEQ ID NO:1, 2, 3 or 4.

In the present invention, the protein consisting of an amino acid sequence in which one or more amino acid(s) is/are deleted, substituted, inserted and/or added in the amino acid sequence represented by SEQ ID NO:5, 6, 7 or 8 and having α-1,6-fucosyltransferase activity can be obtained, e.g., by introducing a site-directed mutation into a DNA encoding a protein consisting of the amino acid sequence represented by SEQ ID NO:5, 6, 7 or 8, respectively, using the site-directed mutagenesis described, e.g., in *Molecular Cloning*, Second Edition; *Current Protocols in Molecular Biology*; *Nucleic Acids Research,* 10, 6487 (1982); *Proc. Natl. Acad Sci. USA*, 79, 6409 (1982); *Gene*, 34, 315 (1985); *Nucleic Acids Research,* 13, 4431 (1985); *Proc. Natl. Acad. Sci. USA*, 82, 488 (1985); and the like. The number of amino acids to be deleted, substituted, inserted and/or added is one or more, and the number is not particularly limited, but is a number which can be deleted, substituted or added by a known technique such as the above-described site-directed mutagenesis, e.g., it is 1 to several tens, preferably 1 to 20, more preferably 1 to 10, and most preferably 1 to 5.

Also, in the present invention, the protein consisting of an amino acid sequence which has 80% or more homology to the amino acid sequence represented by SEQ ID NO:5, 6, 7 or 8 and having α-1,6-fucosyltransferase activity is a protein having at least 80% or more homology, preferably 85% or more homology, more preferably 90% or more homology, still more preferably 95% or more homology, far more preferably 97% or more homology, and most preferably 99% or more homology, to the protein consisting of the amino acid sequence represented by SEQ ID NO:5, 6, 7 or 8, when calculated by using an analyzing software such as BLAST [*J. Mol. Biol.*, 215, 403 (1990)], FASTA *[Methods in Enzymology*, 183, 63 (1990)] or the like.

As a method for obtaining the cell of the present invention, any technique can be used, so long as the genome of interest can be modified. However, genetic engineering techniques are preferred. Examples include:

(a) a gene disruption technique which comprises targeting at a gene encoding the α-1,6-fucose modifying enzyme,
(b) a technique for introducing mutation into a gene encoding the α-1,6-fucose modifying enzyme, and the like.

Furthermore, the cell of the present invention can be obtained by using a method for selecting a clone resistant to a lectin which recognizes a sugar chain structure in which 1-position of fucose is bound to 6-position ofN-acetylglucosamine in the reducing end through α-bond in a complex type N-glycoside-linked sugar chain.

The cell which is resistant to the lectin means a cell in which growth is not inhibited in the presence of a lectin at an effective concentration. The effective concentration is equal to or higher than a concentration in which the cell before the genomic gene is knocked out (hereinafter also referred to as the parent cell) cannot normally grow, and is a concentration which is preferably similar to, more preferably 2 to 5 times, still more preferably 10 times, and most preferably 20 times or more, higher than the concentration in which the cell before the genomic gene is knocked out cannot normally grow.

In the present invention, the effective concentration of a lectin which does not inhibit the growth can be decided depending on the cell line, and is generally 10 μg/ml to 10 mg/ml, preferably 0.5 to 2.0 mg/ml.

As the lectin which recognizes a sugar chain structure in which 1-position of fucose is bound to 6-position of N-acetylglucosamine in the reducing end through α-bond in a complex type N-glycoside-linked sugar chain, any lectin can be used, so long as it can recognize the sugar chain structure. Examples include a *Lens culinaris* lectin LCA (lentil agglutinin derived from *Lens culinaris*), a pea lectin PSA (pea lectin derived from *Pisum sativum),* a broad bean lectin VFA (agglutinin derived from *Vicia faba*), an *Aleuria aurantia* lectin AAL (lectin derived from *Aleuria aurantia*) and the like.

The cell of the present invention may be any cell, so long as it can express a glycoprotein. Examples include a yeast, an animal cell, an insect cell, a plant cell and the like, and specific examples include cells described in the item 2 below. The animal cell includes a CHO cell derived from a Chinese hamster ovary tissue, a rat myeloma cell line YB2/3HL.P2.G11.16Ag.20 cell, a mouse myeloma cell line NS0 cell, a mouse myeloma cell line SP2/0-Ag14 cell, a BHK cell derived from a Syrian hamster kidney tissue, an antibody producing-hybridoma cell, a human leukemia cell line Namalwa cell, an embryonic stem cell, a fertilized egg cell and the like. Preferable examples include the above myeloma cell and hybridoma cell used for producing a glycoprotein such as an antibody composition, a host cell for producing a humanized antibody and a human antibody, an embryonic stem cell and fertilized egg cell for preparing a non-human transgenic animal which produces a human antibody, a plant cell for preparing a transgenic plant which produces a humanized antibody and a human antibody, and the like.

The cell before the genomic gene is knocked out is a cell before a method for knocking out a genomic gene encoding the α-1,6-fucose modifying enzyme is applied. The cell before the genomic gene is knocked out is not particularly limited, and includes, as NS0 cell before the genomic gene is knocked out, NS0 cells described in literatures such as *BIO/TECHNOLOGY*, 10, 169 (1992) and *Biotechnol. Bioeng., 73,* 261 (2001). Further examples include NS0 cell line (RCB 0213) registered at RIKEN Cell Bank, The Institute of Physical and Chemical Research, sub-cell lines obtained by naturalizing these cell lines in serum-free media; and the like.

SP2/0-Ag14 cell before the genomic gene is knocked out includes SP2/0-Ag14 cells described in literatures such as *J. Immunol.*, 126, 317 (1981), *Nature*, 276, 269 (1978) and *Human Antibodies and Hybridomas,* 3, 129 (1992). Further examples include SP2/0-Ag14 cell (ATCC CRL-1581) registered at ATCC, sub-cell lines obtained by naturalizing these cell lines in serum-free media (ATCC CRL-1581.1), and the like.

CHO cell derived from Chinese hamster ovary tissue before the genomic gene is knocked out includes CHO cells described in literatures such as *Journal of Experimental Medicine,* 108, 945 (1958), *Proc. Natl. Acad Sci. USA*, 60, 1275 (1968), *Genetics*, 55, 513 (1968), *Chromosoma*, 41, 129 (1973), *Methods in Cell Science,* 18, 115 (1996), *Radiation Research,* 148, 260 (1997), *Proc. Natl. Acad. Sci. USA,* 77, 4216 (1980), *Proc. Natl. Acad. Sci. USA*, 60, 1275 (1968), *Cell,* 6, 121 (1975) and *Molecular Cell Genetics,* Appendix I, II (p. 883-900). Further examples include cell line CHO-K1 (ATCC CCL-61), cell line DUXB11 (ATCC CRL-9096) and cell line Pro-5 (ATCC CRL-1781) registered at ATCC, commercially available cell line CHO-S (Cat # 11619 of Life Technologies), sub-cell lines obtained by naturalizing these cell lines in serum-free media, and the like.

A rat myeloma cell line YB2/3HL.P2.G11.16Ag.20 cell before the genomic gene is knocked out includes cell lines established from Y3/Ag1.2.3 cell (ATCC CRL-1631). Specific examples include YB2/3HL.P2.G11.16Ag.20 cell described in literatures such as *J. Cell. Biol.*, 93, 576 (1982) and *Methods Enzymol.*, 73B, 1 (1981). Further examples include YB2/3HL.P2.G11.16Ag.20 cell (ATCC CRL-1662) registered at ATCC, sub-cell lines obtained by naturalizing these cell lines in serum-free media, and the like.

In the cell of the present invention, among the enzymes relating to the sugar chain structure of a glycoprotein such as an antibody, an enzyme relating to the modification of fucose is inactivated. Accordingly, in the cell of the present invention prepared by introducing a gene encoding a glycoprotein, the produced glycoprotein is not modified with fucose, so that a glycoprotein composition having high physiological activity can be produced stably by serum-free culturing.

The glycoprotein composition having high physiological activity includes a glycoprotein composition having improved affinity with a receptor, a glycoprotein composition having improved half-life in blood, a glycoprotein composition in which its tissue distribution after administration into blood is changed, and a glycoprotein composition in which its interaction with a protein necessary for expressing pharmacological activity is improved.

Accordingly, any glycoprotein composition is included in the glycoprotein composition of the present invention, so long as it is a glycoprotein composition in which the produced protein has a sugar chain structure modified with fucose, when it is produced by a cell before the genomic gene is knocked out. Examples include an antibody, erythropoietin, thrombopoietin, tissue type plasminogen activator, prourokinase, thrombomodulin, antithrombin III, protein C, blood

coagulation factor VII, blood coagulation factor VIII, blood coagulation factor IX, blood coagulation factor X, blood coagulation factor XII, gonadotropic hormone, thyroid-stimulating hormone, epidermal growth factor (EGF), hepatocyte growth factor (HGF), keratinocyte growth factor, activin, bone formation factor, stem cell factor (SCF), interferon α, interferon β, interferon γ, interleukin 2, interleukin 6, interleukin 10, interleukin 11, soluble interleukin 4 receptor, tumor necrosis factor α, DNase I, galactosidase, α-glucosidase, glucocerebrosidase and the like.

Specific examples of the glycoprotein having remarkably improved physiological activity by having a sugar chain structure which is not modified with fucose include an antibody composition.

Accordingly, the cell of the present invention produces an antibody composition having higher ADCC activity than the antibody composition produced by the cell before the genomic gene is knocked out.

Also, the cell of the present invention produces an antibody composition having a higher ratio of sugar chains in which fucose is not bound to N-acetylglucosamine in the reducing end in the sugar chains, among the total complex type N-glycoside-linked sugar chains bound to the Fc region contained in the antibody composition, than the cell before the genomic gene is knocked out.

The antibody composition is a composition which comprises an antibody molecule having a complex type N-glycoside-linked sugar chain in the Fc region.

The antibody is a tetramer in which two molecules of each of two kinds of polypeptide chains, a heavy chain and a light chain, are respectively associated. Each of about a quarter of the N-terminal side of the heavy chain and about a half of the N-terminal side of the light chain (more than 100 amino acids for each) is called variable region which is rich in diversity and directly relates to the binding to an antigen. The greater part of the moiety other than the variable region is called constant region. Based on homology with the constant region, antibody molecules are classified into classes IgG, IgM, IgA, IgD and IgE.

Also, the IgG class is further classified into subclasses IgG1 to IgG4 based on homology with the constant region.

The heavy chain is classified into four immunoglobulin domains VH, CH1, CH2 and CH3 from its N-terminal side, and a highly flexible peptide region called hinge region is present between CH1 and CH2 to divide CH1 and CH2. A structural unit comprising CH2 and CH3 after the hinge region is called Fc region to which N-glycoside-linked sugar chain is bound and is also a region to which an Fc receptor, a complement and the like are bound *(Immunology Illustrated,* the Original, 5th edition, published on February 10, 2000, by Nankodo; *Handbook of Antibody Technology (Kotai Kogaku Nyumon),* 1st edition on January 25, 1994, by Chijin Shokan).

Sugar chains which binds to a glycoprotein such as an antibody are roughly classified into two types, namely a sugar chain which binds to asparagine (N-glycoside-linked sugar chain) and a sugar chain which binds to serine or threonine (O-glycoside-linked sugar chain), based on the binding form to the protein moiety. The N-glycoside-linked sugar chains have a common core structure shown by the following formula (I) [*Biochemical Experimentation Method 23 -Method for Studying Glycoprotein Sugar Chain* (Gakujutsu Shuppan Center), edited by Reiko Takahashi (1989)].

Formula (I):

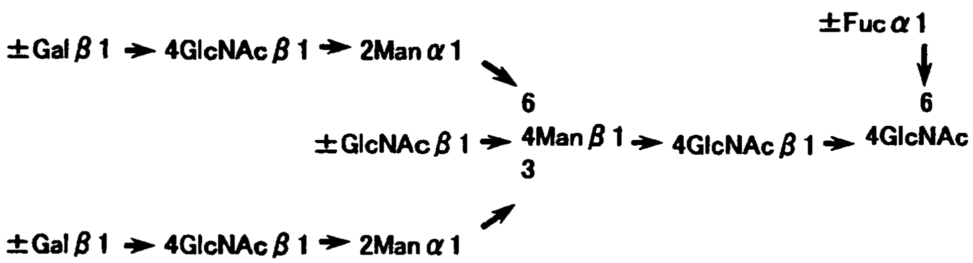

In formula (I), the sugar chain terminus which binds to asparagine is called a reducing end, and the opposite side is called a non-reducing end.

The N-glycoside-linked sugar chain includes a sugar chain having the core structure represented by formula (I) and specifically includes a high mannose type sugar chain in which mannose alone binds to the non-reducing end of the core structure; a complex type sugar chain which the non-reducing end side of the core structure has one or plurality of parallel branches of galactose-N-acetylglucosamine (hereinafter referred to as "Gal-GlcNAc") and the non-reducing end side of Gal-GlcNAc further has a structure of sialic acid, bisecting N-acetylglucosamine or the like; a hybrid type sugar chain in which the non-reducing end side of the core structure comprises branches of both of the high mannose

type and complex type; and the like.

Since the Fc region in the antibody molecule has positions to which N-glycoside-linked sugar chains are separately bound, two sugar chains are bound per one antibody molecule. The N-glycoside-linked sugar chains which bind to antibody molecules may be any sugar chains containing the core structure represented by the above formula (I). A number of combinations of sugar chains are present for the two N-glycoside-linked sugar chains which bind to the antibody.

Accordingly, the antibody composition which is prepared by using the cell of the present invention may comprise an antibody molecule having the same sugar chain structure or an antibody molecule having different sugar chain structures, so long as the effect of the present invention is obtained. The thus prepared antibody composition is preferably an antibody composition having a higher ratio of sugar chains in which fucose is not bound to N-acetylglucosamine in the reducing end in the sugar chains, among the total N-glycoside-linked sugar chains bound to the Fc region contained in the antibody composition, is higher than the antibody composition produced by the parent cell before the genomic gene is knocked out.

The ratio of sugar chains in which fucose is not bound to N-acetylglucosamine in the reducing end in the sugar chain among the total complex type N-glycoside-linked sugar chains bound to the Fc region contained in the antibody composition is a ratio of the number of sugar chains in which fucose is not bound to N-acetylglucosamine in the reducing end in the sugar chains to the total number of the complex type N-glycoside-linked sugar chains bound to the Fc region contained in the composition.

The sugar chain in which fucose is not bound to N-acetylglucosamine in the reducing end in a complex type N-glycoside-linked sugar chain is a sugar chain in which fucose is not bound to N-acetylglucosamine in the reducing end through $\alpha$-bond in a complex type N-glycoside-linked sugar chain. Specifically, it is a sugar chain in which 1-position of fucose is not bound to 6-position of N-acetylglucosamine through $\alpha$-bond in a complex type N-glycoside-linked sugar chain.

The ratio of sugar chains in which fucose is not bound to N-acetylglucosamine in the reducing end in the sugar chain among the total complex type N-glycoside-linked sugar chains bound to the Fc region contained in the antibody composition of the present invention is preferably 20% or more, more preferably 30% or more, still more preferably 40% or more, particularly preferably 50% or more, and most preferably 100%.

The antibody composition having higher ADCC activity than the antibody composition produced by the cell before the genomic gene is knocked out includes those in which, among total complex type N-glycoside-linked sugar chains bound to the Fc region contained in the antibody composition, the ratio of sugar chains in which fucose is not bound to N-acetylglucosamine in the reducing end in the sugar chain is higher than the ratio in an antibody composition produced by the cell before the genomic gene is knocked out. Examples include an antibody composition in which the ratio is at least 2 times, preferably at least 3 times, more preferably at least 5 times, and still more preferably 10 times or higher. An antibody composition in which all of complex type N-glycoside-linked sugar chains bound to the Fc region contained in the antibody composition are sugar chains in which 1-position of fucose is not bound to 6-position ofN-acetylglucosamine in the reducing end in the sugar chain is most preferred.

The antibody composition having a ratio of sugar chains in which fucose are not bound to N-acetylglucosamine in the reducing end in the sugar chain of 100% or the antibody composition in which all of complex type N-glycoside-linked sugar chains bound to the Fc region contained in the antibody composition are sugar chains in which 1-position of the fucose is not bound to 6-position of N-acetylglucosamine in the reducing end contains those in which fucose is in such a degree that it cannot be substantially detected by the sugar chain analysis described in the following item 6. The degree that it cannot be substantially detected means that it is equal to or below the detection limit in the measurement.

In the antibody composition obtained by the present invention, when the ratio of sugar chains in which fucose is not bound to N-acetylglucosamine in the reducing end, among the total compelx type N-glycoside-linked sugar chains bound to the Fc region, is higher than that of an antibody composition produced by the cell before the genomic gene is knocked out, the antibody composition obtained in the present invention has higher ADCC activity than the antibody composition comprising the antibody molecule produced by the parent cell.

The ADCC activity is cytotoxic activity in which an antibody bound to a cell surface antigen existed on a tumor cell and the like in the living body activates an effector cell through binding an Fc receptor existing on the antibody Fc region and effector cell surface and thereby injure the tumor cell and the like [*Monoclonal Antibodies: Principles and Applications,* Wiley-Liss, Inc., Chapter 2.1 (1955)]. The effector cell includes a killer cell, a natural killer cell, an activated macrophage and the like.

The ratio of sugar chains in which fucose is not bound to N-acetylglucosamine in the reducing end in the sugar chain contained in the composition which comprises an antibody molecule having complex type N-glycoside-linked sugar chains in the Fc region can be determined by releasing the sugar chain from the antibody molecule by using a known method such as hydrazinolysis, enzyme digestion or the like *[Biochemical Experimentation Methods 23 - Method for Studying Glycoprotein Sugar Chain* (Japan Scientific Societies Press), edited by Reiko Takahashi (1989)], carrying out fluorescence labeling or radioisotope labeling of the released sugar chain and then separating the labeled sugar chain by chromatography. Also, the released sugar chain can also be analyzed and determined by the HPAED-PAD method

[*J. Liq. Chromatogr.,* 6, 1577 (1983)]. Also, the antibody of the present invention is preferably an antibody which recognizes a tumor-related antigen, an antibody which recognizes an allergy- or inflammation-related antigen, an antibody which recognizes cardiovascular disease-related antigen, an antibody which recognizes an autoimmune disease-related antigen or an antibody which recognizes a viral or bacterial infection-related antigen are exemplified below, and preferably belongs to an IgG class.

The antibody which recognizes a tumor-related antigen includes anti-GD2 antibody [*Anticancer Res.,* 13, 331 (1993)], anti-GD3 antibody [*Cancer Immunol. Immunother.,* 36, 260 (1993)], anti-GM2 antibody [*Cancer Res.,* 54, 1511 (1994)], anti-HER2 antibody *[Proc. Natl. Acad Sci. USA,* 89, 4285 (1992)], anti-CD52 antibody [*Nature*, 332, 323 (1998)], anti-MAGE antibody [*British J. Cancer,* 83, 493 (2000)], anti-HM1.24 antibody *[Molecular Immunol.,* 36, 387 (1999)], anti-parathyroid hormone-related protein (PTHrP) antibody [*Cancer*, 88, 2909 (2000)], anti-FGF8 antibody *[Proc. Natl. Acad. Sci. USA,* 86, 9911 (1989)], anti-basic fibroblast growth factor antibody, anti-FGF8 receptor antibody [*J. Biol. Chem.,* 265, 16455 (1990)], anti-basic fibroblast growth factor receptor antibody, anti-insulin-like growth factor antibody [*J. Neurosci. Res.,* 40, 647 (1995)], anti-insulin-like growth factor receptor antibody [*J. Neurosci. Res.,* 40, 647 (1995)], anti-PMSA antibody [*J. Urology,* 160, 2396 (1998)], anti-vascular endothelial cell growth factor antibody [*Cancer Res.*, 57, 4593 (1997)], anti-vascular endothelial cell growth factor receptor antibody *[Oncogene,* 19, 2138 (2000)], anti-CA125 antibody, anti-17-1A antibody, anti-integrin αvβ3 antibody, anti-CD33 antibody, anti-CD22 antibody, anti-HLA antibody, anti-HLA-DR antibody, anti-CD20 antibody, anti-CD19 antibody, anti-EGF receptor antibody [*Immunology Today,* 21, 403 (2000)], anti-CD 10 antibody *[American Journal of Clinical Pathology,* 113, 374 (2000)] and the like.

The antibody which recognizes an allergy- or inflammation-related antigen includes anti-interleukin 6 antibody [*Immunol. Rev.*, 127, 5 (1992)], anti-interleukin 6 receptor antibody *[Molecular Immunol.*, 31, 371 (1994)], anti-interleukin 5 antibody [*Immunol. Rev.,* 127, 5 (1992)], anti-interleukin 5 receptor antibody and anti-interleukin 4 antibody [*Cytokine*, 3, 562 (1991)], anti-interleukin 4 receptor antibody [*J. Immunol. Meth.*, 217, 41 (1998)], anti-tumor necrosis factor antibody *[Hybridoma,* 13, 183 (1994)], anti-tumor necrosis factor receptor antibody *[Molecular Pharmacol.*, 58, 237 (2000)], anti-CCR4 antibody *[Nature,* 400, 776 (1999)], anti-chemokine antibody [*J. Immuno. Meth.*, 174, 249 (1994)], anti-chemokine receptor antibody [*J. Exp. Med.*, 186, 1373 (1997)], anti-IgE antibody, anti-CD23 antibody, anti-CD11a antibody [*Immunology Today*, 21, 403 (2000)], anti-CRTH2 antibody [*J Immunol.*, 162, 1278 (1999)], anti-CCR8 antibody (WO99/25734), anti-CCR3 antibody (US6207155) and the like.

The antibody which recognizes a cardiovascular disease-related antigen includes anti-GpIIb/IIIa antibody [*J. Immunol.*, 152, 2968 (1994)], anti-platelet-derived growth factor antibody *[Science,* 253, 1129 (1991)], anti-platelet-derived growth factor receptor antibody [*J. Biol. Chem.,* 272, 17400 (1997)], anti-blood coagulation factor antibody [*Circulation*, 101, 1158 (2000)] and the like.

The antibody which recognizes an antigen relating to autoimmune diseases (psoriasis, rheumarthritis, Crohn's diseases, colitis ulcerosa, systemic erythematodes, multiple sclerosis, *etc.)* includes an anti-auto-DNA antibody [*Immunol. Letters,* 72, 61 (2000)], anti-CD11a antibody, anti-ICAM3 antibody, anti-CD80 antibody, anti-CD2 antibody, anti-CD3 antibody, anti-CD4 antibody, anti-integrin α4β7 antibody, anti-CD40L antibody, anti-IL-2 receptor antibody [*Immunology Today,* 21, 403 (2000)], and the like.

The antibody which recognizes a viral or bacterial infection-related antigen includes anti-gp120 antibody [*Structure*, 8, 385 (2000)], anti-CD4 antibody [*J. Rheumatology,* 25, 2065 (1998)], anti-CCR4 antibody, anti-Vero toxin antibody [*J. Clin. Microbiol.,* 37, 396 (1999)], , and the like.

The antibody molecule may be any antibody molecule, so long as it comprises the Fc region of an antibody. Examples include an antibody, an antibody fragment, a fusion protein comprising an Fc region, and the like.

The antibody is a protein which is produced in the living body by immune reaction as a result of exogenous antigen stimulation and has an activity to specifically bind to the antigen. Examples include an antibody secreted by a hybridoma cell prepared from a spleen cell of an animal immunized with an antigen; an antibody prepared by a genetic recombination technique, namely an antibody obtained by introducing an antibody gene-inserted antibody expression vector into a host cell; and the like. Specific examples include an antibody produced by a hybridoma, a humanized antibody, a human antibody and the like.

A hybridoma is a cell which is obtained by cell fusion between a B cell obtained by immunizing a non-human mammal with an antigen and a myeloma cell derived from mouse, rat or the like and can produce a monoclonal antibody having the desired antigen specificity.

The humanized antibody includes a human chimeric antibody, a human CDR-grafted antibody and the like.

A human chimeric antibody is an antibody which comprises a heavy chain variable region (hereinafter referred to as "HV" or "VH", as the variable region being referred to as V region) and a light chain variable region (hereinafter referred to as "LV" or "VL", as the light region being referred to as L region), both of a non-human animal antibody, a human antibody heavy chain constant region (hereinafter also referred to as "CH") and a human antibody light chain constant region (hereinafter also referred to as "CL"). The non-human animal may be any animal such as mouse, rat, hamster, rabbit or the like, so long as a hybridoma can be prepared therefrom.

The human chimeric antibody can be produced by obtaining cDNAs encoding VH and VL from a monoclonal

antibody-producing hybridoma, inserting them into an expression vector for host cell having genes encoding human antibody CH and human antibody CL to thereby construct a vector for expression of human chimeric antibody, and then introducing the vector into a host cell to express the antibody.

The CH of human chimeric antibody may be any CH, so long as it belongs to human immunoglobulin (hereinafter referred to as "hIg"). Those belonging to the hIgG class are preferred and any one of the subclasses belonging to the hIgG class, such as hIgG1, hIgG2, hIgG3 and hIgG4, can be used. Also, as the CL of human chimeric antibody, any CL can be used, so long as it belongs to the hIg class, and those belonging to the κ class or λ class can also be used.

A human CDR-grafted antibody is an antibody in which amino acid sequences of CDRs of VH and VL of a non-human animal antibody are grafted into appropriate positions of VH and VL of a human antibody.

The human CDR-grafted antibody can be produced by constructing cDNAs encoding V regions in which CDRs of VH and VL of a non-human animal antibody are grafted into CDRs of VH and VL of a human antibody, respectively inserting them into an expression vector for host cell having genes encoding human antibody CH and human antibody CL to thereby construct a human CDR-grafted antibody expression vector, and then introducing the expression vector into a host cell to express the human CDR-grafted antibody.

The CH of human CDR-grafted antibody may be any CH, so long as it belongs to the hIg. Those belonging to the hIgG class are preferred and any one of the subclasses belonging to the hIgG class, such as hIgG1, hIgG2, hIgG3 and hIgG4, can be used. Also, as the CL of human CDR-grafted antibody, any CL can be used, so long as it belongs to the hIg class, and those belonging to the κ class or λ class can also be used.

A human antibody is originally an antibody naturally existing in the human body, but it also includes antibodies obtained from a human antibody phage library, a human antibody-producing transgenic non-human animal and a human antibody-producing transgenic plant, which are prepared based on the recent advance in genetic engineering, cell engineering and developmental engineering techniques.

Regarding the antibody existing in the human body, a lymphocyte capable of producing the antibody can be cultured by isolating a human peripheral blood lymphocyte, immortalizing it by infecting with EB virus or the like and then cloning it, and the antibody can be purified from the culture.

The human antibody phage library is a library in which antibody fragments such as Fab, single chain antibody and the like are expressed on the phage surface by inserting a gene encoding an antibody prepared from a human B cell into a phage gene. A phage expressing an antibody fragment having the desired antigen binding activity can be recovered from the library based on its binding activity to an antigen-immobilized substrate. The antibody fragment can be converted further into a human antibody molecule comprising two full H chains and two full L chains by genetic engineering techniques.

A human antibody-producing transgenic non-human animal is a non-human animal in which a human antibody gene is introduced into cells. Specifically, a human antibody-producing transgenic animal can be prepared by introducing a human antibody gene into embryonic stem cell of a mouse, transplanting the embryonic stem cell into an early stage embryo of other mouse and then developing it. The human antibody-producing transgenic non-human animal can also be prepared by introducing a human antibody gene into a fertilized egg of an animal and developing it. Regarding the preparation method of a human antibody from the human antibody-producing transgenic non-human animal, the human antibody can be formed and accumulated in a culture by obtaining a human antibody-producing hybridoma by a hybridoma preparation method usually carried out in non-human mammals and then culturing it.

The transgenic non-human animal includes cattle, sheep, goat, pig, horse, mouse, rat, fowl, monkey, rabbit and the like.

Also, in the present invention, it is preferred that the antibody is an antibody which recognizes a tumor-related antigen, an antibody which recognizes an allergy- or inflammation-related antigen, an antibody which recognizes cardiovascular disease-related antigen, an antibody which recognizes an autoimmune disease-related antigen or an antibody which recognizes a viral or bacterial infection-related antigen, and a human antibody which belongs to the IgG class is preferred.

An antibody fragment is a fragment which comprises at least a part of Fc region of the above antibody. The Fc region is CH2 region and CH3 region, which is a region at the C-terminal of H chain of an antibody, and includes a natural type and a mutant type. The part of Fc region is preferably a fragment which comprises CH2 region, and more preferably region which comprises aspartic acid at position 1 existing in the CH2 region.. The Fc region of the IgG class is from Cys at position 226 to the C-terminal or from Pro at position 230 to the C-terminal according to the numbering of EU Index of Kabat *et al*. [*Sequences of Proteins of Immunological Interest,* 5th Ed., Public Health Service, National Institutes of Health, Bethesda, MD. (1991)]. The antibody fragment includes an H chain monomer, an H chain dimer and the like.

A fusion protein comprising a part of Fc region may be any fusion protein so long as it is a protein in which an antibody comprising the Fc region of an antibody or the antibody fragment is fused with a protein such as an enzyme or a cytokine (hereinafter referred to as "Fc fusion protein").

The present invention is explained below in detail.

1. Preparation of cell of the present invention

The cell of the present invention can be prepared by the following techniques.

(1) Gene disruption technique which comprises targeting gene encoding enzyme

The cell of the present invention can be prepared by using a gene disruption technique by targeting a genomic gene encoding the α-1,6-fucose modifying enzyme. The α-1-,6-fucose modifying enzyme includes α-1,6-fucosyltransferase, α-L-fucosidase and the like.
The gene disruption method may be any method, so long as it can disrupt the gene of the target enzyme. Examples include a homologous recombination method, an RNA-DNA oligonucleotide (RDO) method, a method using retrovirus, a method using transposon, and the like. The methods are specifically described below.

(a) Preparation of the cell of the present invention by homologous recombination

The cell of the present invention can be produced by modifying a target gene on chromosome through a homologous recombination technique for targeting at a gene encoding the α-1,6-fucose modifying enzyme.
The target gene on the chromosome can be modified by using a chromosome engineering method described in *Manipulating the Mouse Embryo, A Laboratory Manual,* Second Edition, Cold Spring Harbor Laboratory Press (1994) (hereinafter referred to as "*Manipulating the Mouse Embryo, A Laboratory Manual"); Gene Targeting, A Practical Approach,* IRL Press at Oxford University Press (1993); *Biomanual Series 8, Gene Targeting, Preparation of Mutant Mice using ES Cells,* Yodo-sha (1995) (hereinafter referred to as "*Preparation of Mutant Mice using ES Cells");* or the like, for example, as follows.
A cDNA encoding the α-1,6-fucose modifying enzyme is prepared.
Based on the nucleotide sequence of the obtained cDNA, a genomic DNA encoding the α-1,6-fucose modifying enzyme is prepared.
Based on the nucleotide sequence of the genomic DNA, a target vector is prepared for homologous recombination of a target gene to be modified (e.g., structural gene of the α-1,6-fucose modifying enzyme, or a promoter gene).
The cell of the present invention can be produced by introducing the prepared target vector into a host cell and selecting a cell in which homologous recombination generated between the target gene and target vector.
As the host cell, any cell such as yeast, an animal cell, an insect cell, a plant cell or the like can be used, so long as it has the gene targeting at the α-1,6-fucose modifying enzyme. Examples include cells described in the following item 2.
The method for obtaining a cDNA or a genomic DNA encoding the α-1,6-fucose modifying enzyme includes the method described below.

<u>Preparation method of cDNA:</u>

A total RNA or mRNA is prepared from various host cells.
A cDNA library is prepared from the prepared total RNA or mRNA.
Degenerative primers are produced based on the known amino acid sequence of the α-1,6-fucose modifying enzyme, e.g., known amino acid sequences such as human amino acid sequence, and a gene fragment encoding the α-1,6-fucose modifying enzyme is obtained by PCR using the prepared cDNA library as the template.
A cDNA encoding the α-1,6-fucose modifying enzyme can be obtained by screening the cDNA library by using the obtained gene fragment as a probe.
As the mRNA of various host cells, a commercially available product (e.g., manufactured by Clontech) may be used or may be prepared from various host cells as follows. The method for preparing a total RNA from various host cells includes the guanidine thiocyanate-cesium trifluoroacetate method [*Methods in Enzymology*, <u>154</u>, 3 (1987)], the acidic guanidine thiocyanate phenol chloroform (AGPC) method [*Analytical Biochemistry*, <u>162,</u> 156 (1987); *Experimental Medicine (Jikken Igaku),* <u>9</u>, 1937 (1991)] and the like.
Furthermore, the method for preparing mRNA as poly(A)$^+$ RNA from a total RNA includes the oligo(dT)-immobilized cellulose column method *(Molecular Cloning,* Second Edition) and the like.
In addition, mRNA can be prepared by using a kit such as Fast Track mRNA Isolation Kit (manufactured by Invitrogen), Quick Prep mRNA Purification Kit (manufactured by Pharmacia) or the like.
A cDNA library is prepared from the prepared mRNA of various host cells. The method for preparing cDNA libraries includes the methods described in *Molecular Cloning,* Second Edition; *Current Protocols in Molecular Biology; A Laboratory Manual,* Second Edition (1989); and the like, or methods using commercially available kits such as SuperScript Plasmid System for cDNA Synthesis and Plasmid Cloning (manufactured by Life Technologies), ZAP-cDNA

Synthesis Kit (manufactured by STRATAGENE) and the like.

As the cloning vector for the preparation of the cDNA library, any vector such as a phage vector, a plasmid vector or the like can be used, so long as it is autonomously replicable in *Escherichia coli* K12. Examples include ZAP Express [manufactured by STRATAGENE, *Strategies*, 5, 58 (1992)], pBluescript II SK(+) *[Nucleic Acids Research,* 17, 9494 (1989)], Lambda ZAP II (manufactured by STRATAGENE), λgt10 and λgt11 [DNA Cloning, A Practical Approach, 1, 49 (1985)], λTriplEx (manufactured by Clontech), λExCell (manufactured by Pharmacia), pT7T318U (manufactured by Pharmacia), pcD2 [*Mol. Cell. Biol.*, 3, 280 (1983)], pUC18 [*Gene*, 33, 103 (1985)] and the like.

Any microorganism can be used as the host microorganism for preparing the cDNA library, and *Escherichia coli* is preferably used. Examples include *Escherichia coli* XL1-Blue MRF' [manufactured by STRATAGENE, *Strategies*, 5, 81 (1992)], *Escherichia coli* C600 [*Genetics*, 39, 440 (1954)], *Escherichia coli* Y1088 *[Science,* 222, 778 (1983)], *Escherichia coli* Y1090 *[Science,* 222, 778 (1983)], *Escherichia coli* NM522 [*J. Mol. Biol.*, 166, 1 (1983)], *Escherichia coli* K802 [*J. Mol. Biol.,* 16, 118 (1966)], *Escherichia coli* JM105 *[Gene,* 38, 275 (1985)] and the like.

The cDNA library can be used as such in the subsequent analysis, and in order to obtain a full length cDNA as efficiently as possible by decreasing the ratio of a cDNA fragment containing a partial coding sequence, a cDNA library prepared by using the oligo cap method developed by Sugano *et al. [Gene,* 138, 171 (1994); *Gene,* 200, 149 (1997); *Protein, Nucleic Acid, Enzyme*, 41, 603 (1996); *Experimental Medicine (Jikken Igaku)*, 11, 2491 (1993); *cDNA Cloning* (Yodo-sha) (1996); *Methods for Preparing Gene Libraries* (Yodo-sha) (1994)] can be used in the following analysis.

Based on the amino acid sequence of the α-1,6-fucose modifying enzyme, degenerative primers specific for the 5'-terminal and 3'-terminal nucleotide sequences of a nucleotide sequence presumed to encode the amino acid sequence are prepared, and DNA is amplified by PCR [*PCR Protocols,* Academic Press (1990)] using the prepared cDNA library as the template to obtain a gene fragment encoding the α-1,6-fucose modifying enzyme.

It can be confirmed that the obtained gene fragment is a DNA encoding the α-1,6-fucose modifying enzyme by a method generally used for analyzing a nucleotide sequence, such as the dideoxy method of Sanger *et al. [Proc. Natl. Acad. Sci. USA,* 74, 5463 (1977)], a nucleotide sequence analyzer such as ABI PRISM 377 DNA Sequencer (manufactured by PE Biosystems) or the like.

A DNA encoding the α-1,6-fucose modifying enzyme can be obtained by carrying out colony hybridization or plaque hybridization *(Molecular Cloning,* Second Edition) to the cDNA or cDNA library synthesized from the mRNA contained in the various host cells, using the gene fragment as a DNA probe.

Also, a DNA encoding the α-1,6-fucose modifying enzyme can also be obtained by carrying out screening by PCR using the cDNA or cDNA library synthesized from the mRNA contained in the various host cells as the template and using the primers used for obtaining the gene fragment encoding the α-1,6-fucose modifying enzyme.

The nucleotide sequence of the obtained DNA encoding the α-1,6-fucose modifying enzyme is analyzed from its terminus and determined by a method generally used for analyzing a nucleotide sequence, such as the dideoxy method of Sanger *et al. [Proc. Natl. Acad. Sci. USA,* 74, 5463 (1977)], a nucleotide sequence analyzer such as ABI PRISM 377 DNA Sequencer (manufactured by PE Biosystems) or the like.

A gene encoding the α-1,6-fucose modifying enzyme can also be determined from genes in data bases by searching nucleotide sequence data bases such as GenBank, EMBL and DDBJ by using a homology retrieving program such as BLAST based on the determined cDNA nucleotide sequence.

The nucleotide sequence of the gene encoding the α-1,6-fucose modifying enzyme includes the nucleotide sequence represented by SEQ ID NO:1, 2, 3 or 4.

The cDNA encoding the α-1,6-fucose modifying enzyme can also be obtained by chemically synthesizing it with a DNA synthesizer such as DNA Synthesizer model 392 manufactured by Perkin Elmer by using the phosphoamidite method, based on the determined DNA nucleotide sequence.

As an example of the method for preparing a genomic DNA encoding the α-1,6-fucose modifying enzyme, the method described below is exemplified.

Preparation method of genomic DNA:

The method for preparing genomic DNA includes known methods described in *Molecular Cloning,* Second Edition; *Current Protocols in Molecular Biology;* and the like. In addition, a genomic DNA encoding the α-1,6-fucose modifying enzyme can also be isolated by using a kit such as Genome DNA Library Screening System (manufactured by Genome Systems) or Universal GenomeWalker™ Kits (manufactured by CLONTECH).

The nucleotide sequence of the genomic DNA encoding the α-1,6-fucose modifying enzyme obtained above includes the nucleotide sequence represented by SEQ ID NO:9.

The target vector used in the homologous recombination of the target gene can be prepared in accordance with a method described in *Gene Targeting*, *A Practical Approach,* IRL Press at Oxford University Press (1993); *Biomanual Series 8, Gene Targeting, Preparation of Mutant Mice using ES Cells,* Yodo-sha (1995); or the like. The target

vector can be used as both a replacement type and an insertion type.

For introducing the target vector into various host cells, the methods for introducing recombinant vectors suitable for various cells described in the following item 2 can be used.

The method for efficiently selecting a homologous recombinant includes a method such as the positive selection, promoter selection, negative selection or polyA selection described in *Gene Targeting, A Practical Approach,* IRL Press at Oxford University Press (1993); *Biomanual Series 8, Gene Targeting, Preparation of Mutant Mice using ES Cells,* Yodo-sha (1995); or the like. The method for selecting the homologous recombinant of interest from the selected clones includes the Southern hybridization method for genomic DNA *(Molecular Cloning,* Second Edition), PCR [*PCR Protocols,* Academic Press (1990)], and the like.

A homologous recombinant can be obtained based on the change of the activity of the α-1,6-kcose modifying enzyme. The following method is exemplified as a method for selecting a transformant.

<u>Method for selecting transformant:</u>

The method for selecting a cell in which the genomic gene encoding the α-1,6-fucose modifying enzyme is knocked out includes biochemical methods or genetic engineering techniques described in literatures such as *New Biochemical Experimentation Series 3-Saccharides I, Glycoprotein* (Tokyo Kagaku Dojin), edited by Japanese Biochemical Society (1988); *Cell Engineering, Supplement, Experimental Protocol Series, Glycobiology Experimental Protocol, Glycoprotein, Glycolipid and Proteoglycan* (Shujun-sha), edited by Naoyuki Taniguchi, Akemi Suzuki, Kiyoshi Furukawa and Kazuyuki Sugawara (1996); *Molecular Cloning,* Second Edition; *Current Protocols in Molecular Biology*; and the like. The biochemical method includes a method in which the enzyme activity is evaluated by using an enzyme-specific substrate and the like. The genetic engineering technique includes the Northern analysis, RT-PCR and the like which measures the amount of mRNA of a gene encoding the enzyme.

Furthermore, the method for selecting a cell based on morphological change caused by knocking out the genomic gene encoding the α-1,6-fucose modifying enzyme includes a method for selecting a transformant based on the sugar chain structure of a produced antibody molecule, a method for selecting a transformant based on the sugar chain structure of a glycoprotein on a cell membrane, and the like. The method for selecting a transformant using the sugar chain structure of an antibody-producing molecule includes method described in the following item 6. The method for selecting a transformant based on the sugar chain structure of a glycoprotein on a cell membrane includes a method selecting a clone resistant to a lectin which recognizes a sugar chain structure wherein 1-position of fucose is bound to 6-position of N-acetylglucosamine in the reducing end through α-bond in an N-glycoside-linked sugar chain. Examples include a method using a lectin described in *Somatic Cell Mol. Genet.,* <u>12,</u> 51 (1986).

As the lectin, any lectin can be used, so long as it is a lectin which recognizes a sugar chain structure in which 1-position of fucose is bound to 6-position of N-acetylglucosamine in the reducing end through α-bond in an N-glycoside-linked sugar chain. Examples include a *Lens culinaris* lectin LCA (lentil agglutinin derived from *Lens culinaris),* a pea lectin PSA (pea lectin derived from *Pisum sativum*), a broad bean lectin VFA (agglutinin derived from *Vicia faba*), an *Aleuria aurantia* lectin AAL (lectin derived from *Aleuria aurantia*) and the like.

Specifically, the cell of the present invention can be selected by culturing cells for 1 day to 2 weeks, preferably 3 days to 1 week, in a medium comprising the above-described lectin at a concentration of several ten μg/ml to several mg/ml, preferably 0.5 to 2.0 mg/ml, subculturing surviving cells or picking up a colony and transferring it into another culture vessel, and subsequently continuing the culturing in the lectin-containing medium.

(b) Preparation of the cell of the present invention by RDO method

The cell of the present invention can be prepared by an RDO method by targeting a gene encoding the α-1,6-fucose modifying enzyme, for example, as follows.

A cDNA or a genomic DNA encoding the α-1,6-fucose modifying enzyme is prepared.

The nucleotide sequence of the prepared cDNA or genomic DNA is determined.

Based on the determined DNA sequence, an RDO construct of an appropriate length comprising a part encoding the α-1,6-fucose modifying enzyme, a part of an untranslated region or a part of intron is designed and synthesized.

The cell of the present invention can be obtained by introducing the synthesized RDO into a host cell and then selecting a transformant in which a mutation is generated in the target enzyme, i.e., the α-1,6-fucose modifying enzyme.

As the host cell, any cell such as yeast, an animal cell, an insect cell and a plant cell can be used, so long as it has a gene encoding the target α-1,6-fucose modifying enzyme. Examples include the host cells described in the following item 2.

The method for introducing RDO into various host cells includes the methods for introducing recombinant vectors suitable for various host cells, described in the following item 2.

The method for preparing cDNA encoding the α-1,6-fucose modifying enzyme includes the "Preparation method

of cDNA" described in the item 1(1)(a) and the like.

The method for preparing a genomic DNA encoding the α-1,6-fucose modifying enzyme includes the "Preparation method of genomic DNA" described in the item 1(1)(a) and the like.

The nucleotide sequence of the DNA can be determined by digesting it with appropriate restriction enzymes, cloning the DNA fragments into a plasmid such as pBluescript SK(-) (manufactured by Stratagene), subjecting the clones to the reaction generally used as a method for analyzing a nucleotide sequence such as the dideoxy method of Sanger *et al.* [*Proc. Natl. Acad Sci. USA,* 74, 5463 (1977)] or the like, and then analyzing the clones by using an automatic nucleotide sequence analyzer such as A.L.F. DNA Sequencer (manufactured by Pharmacia) or the like.

The RDO can be prepared by a usual method or using a DNA synthesizer.

The method for selecting a cell in which a mutation occurred, by introducing the RDO into the host cell, in the target enzyme, i.e., the gene encoding the α-1,6-fucose modifying enzyme, includes the methods for directly detecting mutations in chromosomal genes described in *Molecular Cloning,* Second Edition, *Current Protocols in Molecular Biology* and the like.

Furthermore, "Method for selecting transformant" described in the item 1(1)(a) based on the change of the activity of the α-1,6-fucose modifying enzyme can also be used.

The construct of the RDO can be designed in accordance with the methods described in *Science,* 273, 1386 (1996); *Nature Medicine,* 4, 285 (1998); *Hepatology,* 25, 1462 (1997); *Gene Therapy*, 5, 1960 (1999); *J. Mol. Med.,* 75, 829 (1997); *Proc. Natl. Acad Sci. USA*, 96, 8774 (1999); *Proc. Natl. Acad. Sci. USA*, 96, 8768 (1999); *Nuc. Acids. Res.,* 27, 1323 (1999); *Invest. Dematol.,* 111, 1172 (1998); *Nature Biotech.,* 16, 1343 (1998); *Nature Biotech.,* 18, 43 (2000); *Nature Biotech.,* 18, 555 (2000); and the like.

(c) Preparation of the cell of the present invention by method using transposon

The cell of the present invention can be prepared by using a transposon system described in *Nature Genet.,* 25, 35 (2000) or the like, and by selecting a mutant based on the activity of the α-1,6-fucose modifying enzyme, or the sugar chain structure of a produced antibody molecule or of a glycoprotein on the cell membrane.

The transposon system is a system in which a mutation is induced by randomly inserting an exogenous gene into chromosome, wherein an exogenous gene interposed between transposons is generally used as a vector for inducing a mutation, and a transposase expression vector for randomly inserting the gene into chromosome is introduced into the cell at the same time.

Any transposase can be used, so long as it is suitable for the sequence of the transposon to be used.

As the exogenous gene, any gene can be used, so long as it can induce a mutation in the DNA of a cell.

As the cell, any cell such as yeast, an animal cell, an insect cell or a plant cell can be used, so long as it has a gene encoding the target α-1,6-fucose modifying enzyme. Examples include the host cells described in the following item 2.

For introducing a gene into the cell, the method for introducing recombinant vectors suitable for various host cells described in the following item 2 can be used.

The method for selecting a mutant based on the activity of the α-1,6-fucose modifying enzyme includes "Method for selecting transformant" described in the above item 1(1)(a) based on change of the activity of the α-1,6-fucose modifying enzyme.

(2) Method for introducing mutation into enzyme

The cell of the present invention can be prepared by introducing a mutation into a gene encoding the α-1,6-fucose modifying enzyme, and then by selecting a clone of interest in which the mutation is generated in the enzyme.

The α-1,6-fucose modifying enzyme includes α-1,6-fucosyltransferase, α-L-fucosidase and the like.

The method includes 1) a method in which a desired clone is selected from mutants obtained by a mutation-inducing treatment of a parent cell line or spontaneously generated mutants based on the change of the activity of the α-1,6-fucose modifying enzyme, 2) a method in which a desired clone is selected from mutants obtained by a mutation-inducing treatment of a parent cell line or spontaneously generated mutants based on the sugar chain structure of a produced antibody molecule and 3) a method in which a desired clone is selected from mutants obtained by a mutation-inducing treatment of a parent cell line or spontaneously generated mutants based on the sugar chain structure of a glycoprotein on the cell membrane of the cell.

As the mutation-inducing treatment, any treatment can be used, so long as it can induce a point mutation, a deletion or frame shift mutation in the DNA of the parent cell line. Examples include treatment with ethyl nitrosourea, nitrosoguanidine, benzopyrene or an acridine pigment and treatment with radiation. Also, various alkylating agents and carcinogens can be used as mutagens. The method for allowing a mutagen to' act upon cells includes the methods described in *Tissue Culture Techniques,* 3rd edition (Asakura Shoten), edited by Japanese Tissue Culture Association

(1996), *Nature Genet.,* 24, 314 (2000) and the like.

The spontaneously generated mutant includes mutants which are spontaneously formed by continuing subculture under general cell culture conditions without applying special mutation-inducing treatment.

The method for selecting a clone of interest based on the change of the activity of the α-1,6-fucose modifying enzyme, the method for selecting a clone of interest based on the sugar chain structure of a prepared antibody molecule and the method for selecting a clone of interest based on the sugar chain structure of a glycoprotein on the cell membrane include "Method for selecting transformant" described in the above item 1(1)(a) based on change of the activity of the α-1,6-fucose modifying enzyme.

2. Process for producing glycoprotein based on antibody composition as an example

A process for producing a glycoprotein using the cell of the present invention is explained below based on production of an antibody composition as a specific example.

The antibody composition can be obtained by expressing it in a host cell to which a gene encoding an antibody molecule is introduced, by using the methods described in *Molecular Cloning,* Second Edition; *Current Protocols in Molecular Biology; Antibodies, A Laboratory Manual,* Cold Spring Harbor Laboratory, 1988 (hereinafter referred to as *"Antibodies"); Monoclonal Antibodies: Principles and Practice,* Third Edition, Acad. Press, 1993 (hereinafter referred to as *"Monoclonal Antibodies*"); and *Antibody Engineering, A Practical Approach,* IRL Press at Oxford University Press, 1996 (hereinafter referred to as "*Antibody Engineering"),* for example, as follows.

A cDNA of an antibody molecule is prepared.

Based on the prepared full length cDNA of an antibody molecule, an appropriate length of a DNA fragment comprising a moiety encoding the protein is prepared, if necessary.

A recombinant vector is prepared by inserting the DNA fragment or the full length cDNA into a site downstream of a promoter in an appropriate expression vector.

A transformant which produces the antibody composition of the present invention can be obtained by introducing the recombinant vector into a host cell suitable for the expression vector.

The cDNA can be prepared from a human or non-human animal tissue or cell using, e.g., a probe primer specific for the antibody molecule of interest, in accordance with the methods described in "Preparation method of cDNA" in the item 1(1)(a).

When yeast is used as the host cell, the expression vector includes YEP13 (ATCC 37115), YEp24 (ATCC 37051), YCp50 (ATCC 37419) and the like.

Any promoter can be used, so long as it can be expressed in yeast. Examples include a promoter of a gene of the glycolytic pathway such as a hexose kinase gene, PHO5 promoter, PGK promoter, GAP promoter, ADH promoter, gal 1 promoter, gal 10 promoter, heat shock protein promoter, MF α1 promoter, CUP 1 promoter and the like.

The host cell includes microorganisms belonging to the genus *Saccharomyces,* the genus *Schizosaccharomyces,* the genus *Kluyveromyces*, the genus *Trichosporon*, the genus *Schwanniomyces* and the like, such as *Saccharomyces cerevisiae, Schizosaccharomyces pombe, Kluyveromyces lactis, Trichosporon pullulans* and *Schwanniomyces alluvius*.

As the method for introducing the recombinant vector, any method can be used, so long as it can introduce DNA into yeast. Examples include electroporation [*Methods in Enzymology*, 194, 182 (1990)], the spheroplast method [*Proc. Natl. Acad Sci. USA*, 84, 1929 (1978)], the lithium acetate method [*J. Bacteriol*., 153, 163 (1983)], the method described in *Proc. Natl. Acad. Sci. USA*, 75, 1929 (1978) and the like.

When an animal cell is used as the host cell, the expression vector includes pcDNAI, pcDM8 (available from Funakoshi), pAGE107 [Japanese Published Unexamined Patent Application No. 22979/91; *Cytotechnology,* 3, 133 (1990)], pAS3-3 (Japanese Published Unexamined Patent Application No. 227075/90), pCDM8 [*Nature*, 329, 840 (1987)], pcDNAI/Amp (manufactured by Invitrogen), pREP4 (manufactured by Invitrogen), pAGE103 [*J. Biochemistry*, 101, 1307 (1987)], pAGE210 and the like.

Any promoter can be used, so long as it can be expressed in an animal cell. Examples include a promoter of IE (immediate early) gene of cytomegalovirus (CMV), an early promoter of SV40, a promoter of retrovirus, a promoter of metallothionein, a heat shock promoter, an SRα promoter and the like. Also, an enhancer of the IE gene of human CMV may be used together with the promoter.

The host cell includes a human cell such as Namalwa cell, a monkey cell such as COS cell, a Chinese hamster cell such as CHO cell or HBT5637 (Japanese Published Unexamined Patent Application No. 299/88), a rat myeloma cell, a mouse myeloma cell, a cell derived from Syrian hamster kidney, an embryonic stem cell, a fertilized egg cell and the like.

As the method for introducing the recombinant vector, any method can be used, so long as it can introduce DNA into an animal cell. Examples include electroporation [*Cytotechnology,* 3, 133 (1990)], the calcium phosphate method (Japanese Published Unexamined Patent Application No. 227075/90), the lipofection method [*Proc. Natl. Acad*

*Sci. USA*, 84, 7413 (1987)], the injection method [*Manipulating the Mouse Embryo, A Laboratory Manual,* Second Edition], a method using particle gun (gene gun) (Japanese Patent No. 2606856, Japanese Patent No. 2517813), the DEAE-dextran method *[Biomanual Series 4-Gene Transfer and Expression Analysis* (Yodo-sha), edited by Takashi Yokota and Kenichi Arai (1994)], the virus vector method (*Manipulating Mouse Embryo,* Second Edition) and the like.

When an insect cell is used as the host, the protein can be expressed by the method described in *Current Protocols in Molecular Biology, Baculovirus Expression Vectors, A Laboratory Manual,* W.H. Freeman and Company, New York (1992), *Bio/Technology*, 6, 47 (1988) or the like.

The protein can be expressed by co-infecting a recombinant gene introducing vector and a baculovirus into an insect cell to obtain a recombinant virus in an insect cell culture supernatant and then infecting the insect cell with the recombinant virus.

The gene introducing vector used in the method includes pVL1392, pVL1393, pBlueBacIII (all manufactured by Invitrogen) and the like.

The baculovirus includes *Autographa californica* nuclear polyhedrosis virus which is infected by an insect of the family *Barathra.*

The insect cell includes *Spodoptera frugiperda* oocytes Sf9 and Sf21 [*Current Protocols in Molecular Biology, Baculovirus Expression Vectors, A Laboratory Manual,* W.H. Freeman and Company, New York (1992)], a *Trichoplusiani* oocyte High 5 (manufactured by Invitrogen) and the like.

The method for co-introducing the above recombinant gene-introducing vector and the above baculovirus into an insect cell for preparing the recombinant virus includes the calcium phosphate method (Japanese Published Unexamined Patent Application No. 227075/90), the lipofection method [*Proc. Natl. Acad Sci. USA*, 84, 7413 (1987)] and the like.

When a plant cell is used as the host cell, the expression vector includes Ti plasmid, tobacco mosaic virus vector and the like.

As the promoter, any promoter can be used, so long as it can function in a plant cell. Examples include cauliflower mosaic virus (CaMV) 35S promoter, rice actin 1 promoter and the like.

The host cell includes plant cells of tobacco, potato, tomato, carrot, soybean, rape, alfalfa, rice, wheat, barley and the like.

As the method for introducing the recombinant vector, any method can be used, so long as it can introduce DNA into a plant cell. Examples include a method using *Agrobacterium* (Japanese Published Unexamined Patent Application No. 140885/84, Japanese Published Unexamined Patent Application No. 70080/85, WO94/00977), electroporation (Japanese Published Unexamined Patent Application No. 251887/85), a method using a particle gun (gene gun) (Japanese Patent No. 2606856, Japanese Patent No. 2517813) and the like.

As the method for expressing a gene, secretion production, expression of a fusion protein of the Fc region with other protein and the like can be carried out in accordance with the method described in *Molecular Cloning,* Second Edition or the like, in addition to the direct expression.

When a gene is expressed by yeast, an animal cell, an insect cell, a plant cell or the like into which a gene relating to the synthesis of a sugar chain is introduced, an antibody molecule to which a sugar or a sugar chain is added by the introduced gene can be obtained.

An antibody composition can be obtained by culturing the obtained transformant in a medium to form and accumulate the antibody molecule in the culture and then recovering it from the resulting culture. The method for culturing the transformant using a medium can be carried out in accordance with a general method which is used for the culturing of host cells.

As the medium for culturing the transformant obtained by using yeast as a host, either a natural medium or a synthetic medium can be used, so long as it comprises carbon sources, nitrogen sources, inorganic salts and the like which can be assimilated by the organisms and culturing of the transformant can be carried out efficiently.

As the carbon source, those which can be assimilated by the organism can be used. Examples include carbohydrates such as glucose, fructose, sucrose, molasses containing them, starch and starch hydrolysate; organic acids such as acetic acid and propionic acid; alcohols such as ethanol and propanol; and the like.

The nitrogen source includes ammonia; ammonium salts of inorganic acid or organic acid such as ammonium chloride, ammonium sulfate, ammonium acetate and ammonium phosphate; other nitrogen-containing compounds; peptone; meat extract; yeast extract, corn steep liquor; casein hydrolysate; soybean meal; soybean meal hydrolysate; various fermented cells and hydrolysates thereof; and the like.

The inorganic material includes potassium dihydrogen phosphate, dipotassium hydrogen phosphate, magnesium phosphate, magnesium sulfate, sodium chloride, ferrous sulfate, manganese sulfate, copper sulfate, calcium carbonate, and the like.

The culturing is carried out generally under aerobic conditions such as shaking culture or submerged-aeration stirring culture. The culturing temperature is preferably 15 to 40°C, and the culturing time is generally 16 hours to 7 days. During the culturing, the pH is maintained at 3.0 to 9.0. The pH is adjusted using an inorganic or organic acid, an alkali

solution, urea, calcium carbonate, ammonia or the like.

If necessary, antibiotics such as ampicillin or tetracycline may be added to the medium during the culturing.

When a microorganism transformed with a recombinant vector obtained by using an inducible promoter as the promoter is cultured, an inducer may be added to the medium, if necessary. For example, when a microorganism transformed with a recombinant vector obtained by using *lac* promoter is cultured, isopropyl-β-D-thiogalactopyranoside or the like may be added to the medium, and when a microorganism transformed with a recombinant vector obtained by using *trp* promoter is cultured, indoleacrylic acid may be added to the medium.

When a transformant obtained by using an animal cell as the host is cultured, the medium includes generally used RPMI 1640 medium [*The Journal of the American Medical Association,* 199, 519 (1967)], Eagle's MEM medium [*Science,* 122, 501 (1952)], Dulbecco's modified MEM medium [*Virology,* 8, 396 (1959)], 199 medium [*Proceeding of the Society for the Biological Medicine,* 73, 1 (1950)] and Whitten's medium [*Developmental Engineering Experimentation Manual-Preparation of Transgenic Mice* (Kodan-sha), edited by M. Katsuki (1987)], the media to which insulin, an insulin-like growth factor, transferrin, albumin, *etc.* is added, and the like.

The culturing is carried out generally at a pH of 6 to 8 and 30 to 40°C for 1 to 7 days in the presence of 5% $CO_2$ The culturing can be carried out for 1 day to several months by a culturing method such as fed-batch culture and hollow-fiber culture.

If necessary, antibiotics such as kanamycin or penicillin may be added to the medium during the culturing.

The medium for the culturing of a transformant obtained by using an insect cell as the host includes generally used TNM-FH medium (manufactured by Pharmingen), Sf-900 II SFM medium (manufactured by Life Technologies), ExCell 400 and ExCell 405 (both manufactured by JRH Biosciences), Grace's Insect Medium [*Nature*, 195, 788 (1962)] and the like.

The culturing is carried out generally at a pH of 6 to 7 and 25 to 30°C for 1 to 5 days.

In addition, antibiotics such as gentamicin may be added to the medium during the culturing, if necessary.

A transformant obtained by using a plant cell as the host can be cultured as a cell or after differentiating it into a plant cell or organ. The medium for culturing the transformant includes generally used Murashige and Skoog (MS) medium and White medium, the media to which a plant hormone such as auxin, cytokinin, *etc.* is added, and the like.

The culturing is carried out generally at a pH of 5 to 9 and 20 to 40°C for 3 to 60 days.

If necessary, antibiotics such as kanamycin or hygromycin may be added to the medium during the culturing.

Accordingly, an antibody composition can be produced by culturing a transformant derived from a yeast, an animal cell or a plant cell, which comprises a recombinant vector into which a DNA encoding an antibody molecule is inserted, in accordance with a general culturing method, to thereby form and accumulate the antibody composition, and then recovering the antibody composition from the culture.

The method for producing an antibody composition includes a method of intracellular expression in a host cell, a method of extracellular secretion from a host cell, and a method of producing it on a host cell membrane outer envelope. The method can be selected by changing the host cell used or the structure of the antibody composition produced.

When the antibody composition is produced in a host cell or on a host cell membrane outer envelope, it can be positively secreted extracellularly in accordance with the method of Paulson *et al*. [*J. Biol. Chem.,* 264, 17619 (1989)], the method of Lowe *et al*. [*Proc. Natl. Acad. Sci. USA,* 86, 8227 (1989), *Genes Develop.,* 4, 1288 (1990)], the methods described in Japanese Published Unexamined Patent Application No. 336963/93 and WO94/23021 and the like.

That is, an antibody molecule of interest can be positively secreted extracellularly from a host cell by inserting a DNA encoding the antibody molecule and a DNA encoding a signal peptide suitable for the expression of the antibody molecule into an expression vector using a gene recombination technique, introducing the expression vector into the host cell and then expressing the antibody molecule.

Also, its production amount can be increased in accordance with the method described in Japanese Published Unexamined Patent Application No. 227075/90 by using a gene amplification system using a dihydrofolate reductase gene and the like.

In addition, the antibody composition can also be produced by using a gene-introduced animal individual (transgenic non-human animal) or a plant individual (transgenic plant) which is constructed by the redifferentiation of an animal or plant cell into which the gene is introduced.

When the transformant is an animal individual or a plant individual, the antibody composition can be produced in accordance with a general method by rearing or cultivating it to thereby form and accumulate the antibody composition and then recovering the antibody composition from the animal or plant individual.

The method for producing an antibody composition by using an animal individual includes a method in which the antibody composition of interest is produced in an animal constructed by introducing a gene in accordance with a known method [*American Journal of Clinical Nutrition*, 63, 639S (1996); *American Journal of Clinical Nutrition*, 63, 627S (1996); *Bio/Technology*, 9, 830 (1991)].

In the case of an animal individual, an antibody composition can be produced by rearing a transgenic non-human animal into which a DNA encoding an antibody molecule is introduced to thereby form and accumulate the

antibody composition in the animal, and then recovering the antibody composition from the animal. The place in the animal where the composition is formed and accumulated includes milk (Japanese Published Unexamined Patent Application No. 309192/88), eggs of the animal and the like. As the promoter used in this case, any promoter can be used, so long as it can function in an animal. Preferred examples include mammary gland cell-specific promoters such as α casein promoter, β casein promoter, β lactoglobulin promoter, whey acidic protein promoter and the like.

The method for producing an antibody composition by using a plant individual includes a method in which an antibody composition is produced by cultivating a transgenic plant into which a DNA encoding an antibody molecule is introduced by a known method [*Tissue Culture,* 20 (1994); *Tissue Culture,* 21 (1995); *Trends in Biotechnology,* 15, 45 (1997)] to form and accumulate the antibody composition in the plant, and then recovering the antibody composition from the plant.

Regarding an antibody composition produced by a transformant into which a gene encoding an antibody molecule is introduced, for example, when the antibody composition is intracellularly expressed in a dissolved state, the cells after culturing are recovered by centrifugation, suspended in an aqueous buffer and then disrupted with ultrasonic oscillator, French press, Manton Gaulin homogenizer, dynomill or the like to obtain a cell-free extract. A purified product of the antibody composition can be obtained from a supernatant obtained by centrifuging the cell-free extract, by using a general enzyme isolation purification techniques such as solvent extraction; salting out and desalting with ammonium sulfate, and the like; precipitation with an organic solvent; anion exchange chromatography using a resin such as diethylaminoethyl (DEAE)-Sepharose or DIAION HPA-75 (manufactured by Mitsubishi Chemical); cation exchange chromatography using a resin such as S-Sepharose FF (manufactured by Pharmacia); hydrophobic chromatography using a resin such as butyl-Sepharose, phenyl-Sepharose; gel filtration using a molecular sieve; affinity chromatography; chromatofocusing; electrophoresis such as isoelectric focusing; and the like which may be used alone or in combination.

Also, when the antibody composition is expressed intracellularly by forming an insoluble body, the cells are recovered, disrupted and centrifuged in the same manner, and the insoluble body of the antibody composition is recovered as a precipitation fraction. The recovered insoluble body of the antibody composition is solubilized using a protein denaturing agent. The antibody composition is made into a normal three-dimensional structure by diluting or dialyzing the solubilized solution, and then a purified preparation of the antibody composition is obtained by the same isolation purification method as above.

When the antibody composition is secreted extracellularly, the antibody composition can be recovered from the culture supernatant. That is, the culture is treated by a technique such as centrifugation as described above to obtain a soluble fraction, and a purified preparation of the antibody composition can be obtained from the soluble fraction by the same isolation purification method as above.

The thus obtained antibody composition includes an antibody, the fragment of the antibody, a fusion protein comprising the Fc region of the antibody, and the like.

As an example for obtaining the antibody composition, methods for producing a humanized antibody composition and an Fc fusion protein composition is described below in detail, but glycoproteins of other antibody compositions and the like can also be obtained in a manner similar to the above-described method or the method.

A. Preparation of humanized antibody composition

(1) Construction of vector for expression of humanized antibody

A vector for expression of humanized antibody is an expression vector for animal cell into which genes encoding the heavy chain (H chain) C region and light (L chain) C region of a human antibody are inserted, which can be constructed by cloning each of genes encoding the H chain C region and L chain C region of a human antibody into an expression vector for animal cell.

The C regions of a human antibody may be the H chain C region and L chain C region of any human antibody. Examples include the C region belonging to IgG1 subclass in the H chain of a human antibody (hereinafter referred to as "hCγ1"), the C region belonging to κ class in the L chain of a human antibody (hereinafter referred to as "hCκ"), and the like.

As the genes encoding the H chain C region and L chain C region of a human antibody, a chromosomal DNA comprising an exon and an intron can be used, and a cDNA can also be used.

As the expression vector for animal cell, any vector can be used, so long as a gene encoding the C region of a human antibody can be inserted thereinto and expressed therein. Examples include pAGE107 [*Cytotechnology*, 3, 133 (1990)], pAGE103 [*J. Biochem.*, 101, 1307 (1987)], pHSG274 [*Gene*, 27, 223 (1984)], pKCR [*Proc. Natl. Acad. Sci. USA*, 78, 1527 (1981), pSG1 β d2-4 [*Cytotechnology,* 4, 173 (1990)] and the like. The promoter and enhancer used in the expression vector for animal cell includes SV40 early promoter and enhancer [*J. Biochem.,* 101, 1307 (1987)], Moloney mouse leukemia virus LTR promoter [*Biochem. Biophys. Res. Commun.,* 149, 960 (1987)], immunoglobulin H chain promoter [*Cell,* 41, 479 (1985)] and enhancer [*Cell*, 33, 717 (1983)] and the like.

The vector for expression of humanized antibody may be either of a type in which genes encoding the H chain and L chain of an antibody exist on separate vectors or of a type in which both genes exist on the same vector (hereinafter referred to as "tandem type"). In respect of easiness of construction of a humanized antibody expression vector, easiness of introduction into animal cells, and balance between the expression amounts of the H and L chains of an antibody in animal cells, a tandem type of the vector for expression of humanized antibody is more preferred [*J. Immunol. Methods,* 167, 271 (1994)].

The constructed vector for expression of humanized antibody can be used for expression of a human chimeric antibody and a human CDR-grafted antibody in animal cells.

(2) Obtaining of cDNA encoding V region of non-human animal antibody

cDNAs encoding the H chain C region and L chain V region of a non-human animal antibody, such as a mouse antibody, can be obtained in the following manner.

A cDNA is synthesized from mRNA extracted from a hybridoma cell which produces the mouse antibody of interest. The synthesized cDNA is cloned into a vector such as a phage or a plasmid to obtain a cDNA library. Each of a recombinant phage or recombinant plasmid comprising a cDNA encoding the H chain V region and a recombinant phage or recombinant plasmid comprising a cDNA encoding the L chain V region is isolated from the library by using a C region part or a V region part of an existing mouse antibody as the probe. Full nucleotide sequences of the H chain V region and L chain V region of the mouse antibody of interest on the recombinant phage or recombinant plasmid are determined, and full length amino acid sequences of the H chain V region and L chain V region are deduced from the nucleotide sequences.

As the non-human animal, any animal such as mouse, rat, hamster or rabbit can be used so long as a hybridoma cell can be produced therefrom.

The method for preparing total RNA from a hybridoma cell includes the guanidine thiocyanate-cesium trifluoroacetate method [*Methods in Enzymology,* 154, 3 (1987)] and the like, and the method for preparing mRNA from total RNA includes an oligo(dT)-immobilized cellulose column method [*Molecular Cloning, A Laboratory Manual,* Cold Spring Harbor Lab. Press New York (1989)] and the like. In addition, examples of a kit for preparing mRNA from a hybridoma cell include Fast Track mRNA Isolation Kit (manufactured by Invitrogen), Quick Prep mRNA Purification Kit (manufactured by Pharmacia) and the like.

The method for synthesizing cDNA and preparing a cDNA library includes the usual methods [*Molecular Cloning, A Laboratory Manual,* Cold Spring Harbor Lab. Press New York (1989), *Current Protocols in Molecular Biology,* Supplement 1-34], methods using a commercially available kit such as SuperScript™, Plasmid System for cDNA Synthesis and Plasmid Cloning (manufactured by GIBCO BRL) or ZAP-cDNA Synthesis Kit (manufactured by Stratagene) and the like.

In preparing the cDNA library, the vector into which a cDNA synthesized by using mRNA extracted from a hybridoma cell as the template is inserted may be any vector so long as the cDNA can be inserted. Examples include ZAP Express [*Strategies,* 5, 58 (1992)], pBluescript II SK(+) [*Nucleic Acids Research,* 17, 9494 (1989)], λzapII (manufactured by Stratagene), λgt10 and λgt11 [*DNA Cloning; A Practical Approach,* I, 49 (1985)], Lambda BlueMid (manufactured by Clontech), λExCell, pT7T3 18U (manufactured by Pharmacia), pcD2 [*Mol. Cell. Biol.,* 3, 280 (1983)], pUC18 [*Gene,* 33, 103 (1985)] and the like.

As *Escherichia coli* into which the cDNA library constructed from a phage or plasmid vector is introduced, any *Escherichia coli* can be used, so long as the cDNA library can be introduced, expressed and maintained. Examples include XL1-Blue MRF' [*Strategies,* 5, 81 (1992)], C600 [*Genetics,* 39, 440 (1954)], Y1088 and Y1090 [*Science,* 222, 778 (1983)], NM522 [*J. Mol. Biol.,* 166, 1 (1983)], K802 [*J. Mol. Biol.,* 16, 118 (1966)], JM105 [*Gene,* 38, 275 (1985)] and the like.

As the method for selecting a cDNA clone encoding the H chain V region and L chain V region of a non-human animal antibody from the cDNA library, a colony hybridization or a plaque hybridization using an isotope- or fluorescence-labeled probe can be used *[Molecular Cloning, A Laboratory Manual,* Cold Spring Harbor Lab. Press New York(1989)].

The cDNA encoding the H chain V region and L chain V region can also be prepared by preparing primers and carrying out polymerase chain reaction [hereinafter referred to as "PCR"; *Molecular Cloning, A Laboratory Manual,* Cold Spring Harbor Lab. Press New York (1989); *Current Protocols in Molecular Biology,* Supplement 1-34] using a cDNA synthesized from mRNA or a cDNA library as the template.

The nucleotide sequences of the cDNAs can be determined by digesting the selected cDNAs according to the above methods with appropriate restriction enzymes, cloning the DNA fragments into a plasmid such as pBluescript SK (-) (manufactured by Stratagene), carrying out the reaction of a generally used nucleotide sequence analyzing method such as the dideoxy method of Sanger *et al. [Proc. Natl. Acad. Sci. USA, 74,* 5463 (1977)] or the like and then analyzing the clones using an automatic nucleotide sequence analyzer such as A.L.F. DNA Sequencer (manufactured by Pharmacia) or the like.

Whether or not the obtained cDNAs encode the full length amino acid sequences of the H chain V region and L chain V region of the antibody comprising a secretory signal sequence can be confirmed by deducing the full length amino acid sequences of the H chain V regionand L chain V region from the determined nucleotide sequence and comparing them with the full length amino acid sequences of the H chain V region and L chain V region of known antibodies [*Sequences of Proteins of Immunological Interest*, US Dept. Health and Human Services (1991)].

(3) Analysis of amino acid sequence of V region of non-human animal antibody

Regarding the full length amino acid sequences of the H chain V region and L chain V region of the antibody comprising a secretory signal sequence, the length of the secretory signal sequence and the N-terminal amino acid sequences can be deduced and subgroups to which they belong can also be found, by comparing them with the full length amino acid sequences of the H chain V region and L chain V region of known antibodies [*Sequences of Proteins of Immunological Interest,* US Dept. Health and Human Services, (1991)]. In addition, the amino acid sequences of each CDR of the H chain V region and L chain V region can also be found by comparing them with the amino acid sequences of the H chain V region and L chain V region of known antibodies [*Sequences of Proteins of Immunological Interest,* US Dept. Health and Human Services, (1991)].

(4) Construction of human chimeric antibody expression vector

A human chimeric antibody expression vector can be constructed by cloning cDNAs encoding the H chain V region and L chain V region of a non-human animal antibody into a site upstream of genes encoding the H chain C region and L chain C region of a human antibody in the vector for expression of humanized antibody described in the item 2.A.(1). For example, a human chimeric antibody expression vector can be constructed by linking each of cDNAs encoding the H chain V region and L chain V region of a non-human animal antibody to a synthetic DNA comprising nucleotide sequences at the 3'-terminals of the H chain V region and L chain V region of a non-human animal antibody and nucleotide sequences at the 5'-terminals of the H chain C region and L chain C region of a human antibody and also having a recognition sequence of an appropriate restriction enzyme at both terminals, and by appropriately cloning them into a site upstream of genes encoding the H chain C region and L chain C region of a human antibody contained in the vector for expression of humanized antibody described in the item 2.A.(1).

(5) Construction of cDNA encoding V region of human CDR-grafted antibody

cDNAs encoding the H chain V region and L chain V region of a human CDR-grafted antibody can be obtained as follows. First, amino acid sequences of the frameworks (hereinafter referred to as "FR") of the H chain V region and L chain V region of a human antibody for grafting CDR of the H chain V region and L chain V region of a non-human animal antibody of interest is selected. As the amino acid sequences of FRs of the H chain V region and L chain V region of a human antibody, any amino acid sequences can be used so long as they are derived from a human antibody. Examples include amino acid sequences of FRs of the H chain V region and L chain V region of human antibodies registered at databases such as Protein Data Bank, amino acid sequences common in each subgroup of FRs of the H chain V region and L chain V region of human antibodies [*Sequences of Proteins of Immunological Interest,* US Dept. Health and Human Services (1991)] and the like. In order to prepare a human CDR-grafted antibody having sufficient activities, it is preferred to select an amino acid sequence having a homology as high as possible (at least 60% or more) with amino acid sequences of FRs of the H chain V region and L chain V region of a non-human animal antibody of interest.

Next, the amino acid sequences of CDRs of the H chain V region and L chain V region of the non-human animal antibody of interest are grafted to the selected amino acid sequences of FRs of the H chain V region and L chain V region of a human antibody to design amino acid sequences of the H chain V region and L chain V region of the human CDR-grafted antibody. The designed amino acid sequences are converted into DNA sequences by considering the frequency of codon usage found in nucleotide sequences of antibody genes [*Sequences of Proteins of Immunological Interest*, US Dept. Health and Human Services (1991)], and the DNA sequences encoding the amino acid sequences of the H chain V region and L chain V region of the human CDR-grafted antibody are designed. Based on the designed DNA sequences, several synthetic DNAs having a length of about 100 bases are synthesized, and PCR is carried out by using them. In this case, it is preferred in each of the H chain and the L chain that 6 synthetic DNAs are designed in view of the reaction efficiency of PCR and the lengths of DNAs which can be synthesized.

Also, they can be easily cloned into the vector for expression of humanized antibody described in the item 2.A. (1) by introducing recognition sequences of an appropriate restriction enzyme into the 5'-terminals of the synthetic DNA on both terminals. After the PCR, the amplified product is cloned into a plasmid such as pBluescript SK(-) (manufactured by Stratagene) and the nucleotide sequences are determined by the method in the item 2.A.(2) to thereby obtain a plasmid having DNA sequences encoding the amino acid sequences of the H chain V region and L chain V region of

the desired human CDR-grafted antibody.

(6) Modification of amino acid sequence of V region of human CDR-grafted antibody

It is known that a human CDR-grafted antibody in which CDRs in the H chain V region and L chain V region of a non-human animal antibody of interest are simply grafted with FRs in the H chain V region and L chain V region of a human antibody has lowered antigen-binding activity than the original non-human animal antibody [*BIO/TECHNOLOGY*, 9, 266 (1991)]. As the reason, it is considered that several amino acid residues of FRs other than CDRs directly or indirectly relate to antigen-binding activity in the H chain V region and L chain V region of the original non-human animal antibody, and that they are changed to different amino acid residues of FRs in the H chain V region and L chain V region of a human antibody as they are grafted with CDRs. In order to solve the problem, in human CDR-grafted antibodies, among the amino acid sequences of FRs in H chain V region and L chain V region of a human antibody, an amino acid residue which directly relates to an antigen binding, or an amino acid residue which interacts with CDR, or an amino acid residue which indirectly relates to an antigen binding by maintaining the three-dimensional structure of an antibody is identified and modified to an amino acid residue which is found in the original non-human animal antibody to thereby increase the antigen binding activity which has been decreased [*BIO/TECHNOLOGY*, 9, 266 (1991)].

In the production of a human CDR-grafted antibody, the most important point is how to efficiently identify the amino acid residues relating to the antigen binding activity in FR. Therefore, the three-dimensional structure of an antibody is constructed and analyzed by X-ray crystallography [*J. Mol. Biol.*, 112, 535 (1977)], computer-modeling [*Protein Engineering,* 7, 1501 (1994)] or the like. Although the information of the three-dimensional structure of antibodies has been useful in the production of a human CDR-grafted antibody, method for producing a human CDR-grafted antibody which can be applied to all antibodies has not been established yet. Therefore, various attempts must be made. Such attempts include producing several modified antibodies of each antibody and examining the relationship between each of the modified antibodies and its antigen binding activity.

The modification of the amino acid residue of FRs in H chain V region and L chain V region of a human antibody can be accomplished using various synthetic DNA for modification according to PCR as described in the item 2.A.(5). With regard to the amplified product obtained by the PCR, the nucleotide sequence is determined according to the method as described in the item 2.A.(2) to thereby confirm whether the objective modification has been carried out.

(7) Construction of human CDR-grafted antibody expression vector

A human CDR-grafted antibody expression vector can be constructed by cloning the cDNAs encoding the H chain V region and L chain V region of the human CDR-grafted antibody constructed in the items 2.A.(5) and (6) into a site upstream of the gene encoding the H chain C region and L chain C region of a human antibody in the vector for expression of humanized antibody described in the item 2.A.(1). For example, recognizing sequences of an appropriate restriction enzyme are introduced into the 5'-terminals of both terminals of a synthetic DNA fragment, among the synthetic DNAs which are used in the items 2.A.(5) and (6) for constructing the H chain V region and L chain V region of the human CDR-grafted antibody, so that they are cloned into a site upstream of the genes encoding the H chain C region and L chain C region of a human antibody in the vector for expression of humanized antibody described in the item 2.A.(1) in such a manner that they can be expressed in a suitable form, to thereby construct the human CDR-grafted antibody expression vector.

(8) Stable production of humanized antibody

A transformant capable of stably producing a human chimeric antibody and a human CDR-grafted antibody (both hereinafter referred to as "humanized antibody") can be obtained by introducing the humanized antibody expression vector described in the items 2.A.(4) and (7) into an appropriate animal cell.

The method for introducing a humanized antibody expression vector into an animal cell includes electroporation [Japanese Published Unexamined Patent Application No. 257891/90, *Cytotechnology,* 3, 133 (1990)] and the like.

As the animal cell into which a humanized antibody expression vector is introduced, any cell can be used so long as it is an animal cell capable of producing the humanized antibody.

Examples include mouse myeloma cells such as NS0 cell and SP2/0 cell, Chinese hamster ovary cells such as CHO/dhfr- cell and CHO/DG44 cell, rat myeloma such as YB2/0 cell and IR983F cell, BHK cell derived from a Syrian hamster kidney, a human myeloma cell such as Namalwa cell, and the like, and a Chinese hamster ovary cell CHO/DG44 cell, a rat myeloma YB2/0 cell and the cells described in the item 1 and the like are preferred.

After introduction of the humanized antibody expression vector, a transformant capable of stably producing the humanized antibody can be selected by using a medium for animal cell culture comprising an agent such as G418 sulfate (hereinafter referred to as "G418"; manufactured by SIGMA) and the like in accordance with the method described in

Japanese Published Unexamined Patent Application No. 257891/90. The medium to culture animal cells includes RPMI 1640 medium (manufactured by Nissui Pharmaceutical), GIT medium (manufactured by Nihon Pharmaceutical), EX-CELL 302 medium (manufactured by JRH), IMDM medium (manufactured by GIBCO BRL), Hybridoma-SFM medium (manufactured by GIBCO BRL), media obtained by adding various additives such as insulin, an insulin-like growth factor, transferrin and albumin to these media, and the like. The humanized antibody can be formed and accumulated in the culture supernatant by culturing the obtained transformant in a medium. The amount of production and antigen binding activity of the humanized antibody in the culture supernatant can be measured by a method such as enzyme-linked immunosorbent assay [hereinafter referred to as "ELISA"; *Antibodies: A Laboratory Manual*, Cold Spring Harbor Laboratory, Chapter 14 (1998), *Monoclonal Antibodies: Principles and Practice*, Academic Press Limited (1996)] or the like. Also, the amount of the humanized antibody produced by the transformant can be increased by using a DHFR gene amplification system in accordance with the method described in Japanese Published Unexamined Patent Application No. 257891/90.

The humanized antibody can be purified from a culture supernatant of the transformant using a protein A column [*Antibodies, A Laboratory Manual,* Cold Spring Harbor Laboratory, Chapter 8 (1988), *Monoclonal Antibodies: Principles and Practice,* Academic Press Limited (1996)]. In addition, purification methods generally used for the purification of proteins can also be used. For example, the purification can be carried out through the combination of gel filtration, ion exchange chromatography, ultrafiltration and the like. The molecular weight of the H chain, L chain or antibody molecule as a whole of the purified humanized antibody can be measured, e.g., by polyacrylamide gel electrophoresis [hereinafter referred to as "SDS-PAGE"; *Nature*, 227, 680 (1970)], Western blotting [*Antibodies, A Laboratory Manual,* Cold Spring Harbor Laboratory, Chapter 12, (1988), *Monoclonal Antibodies: Principles and Practice*, Academic Press Limited (1996)] or the like.

B. Preparation of Fc fusion protein

(1) Construction of vector for expression of Fc fusion protein

A vector for expression Fc fusion protein is a vector for expression of animal cell into which genes encoding the Fc region of a human antibody and a protein to be fused are inserted, which can be constructed by cloning each of genes encoding the Fc region of a human antibody and the protein to be fused into a vector for expression of animal cell.

The Fc region of a human antibody includes those comprising a part of a hinge region and/or CH1 in addition to regions comprising CH2 and CH3 regions. Also, it can be any Fc region so long as at least one amino acid of CH2 or CH3 is deleted, substituted, added or inserted, and substantially has the binding activity to the Fcγ receptor.

As the genes encoding the Fc region of a human antibody and the protein to be fused, a chromosomal DNA comprising an exon and an intron can be used, and a cDNA can also be used. The method for linking the genes and the Fc region includes PCR using each of the gene sequences as the template *(Molecular Cloning*, Second Edition; *Current Protocols in Molecular Biology,* Supplement 1-34).

As the vector for expression of animal cell, any vector can be used, so long as a gene encoding the C region of a human antibody can be inserted thereinto and expressed therein. Examples include pAGE107 *[Cytotechnology,* 3, 133 (1990)], pAGE103 [*J. Biochem.,* 101, 1307 (1987)], pHSG274 *[Gene,* 27, 223 (1984)], pKCR [*Proc. Natl. Acad. Sci. USA*, 78, 1527 (1981), pSG1 β d2-4 *[Cytotechnology,* 4, 173 (1990)] and the like. The promoter and enhancer in the vector for expression of animal cell include SV40 early promoter and enhancer [*J. Biochem.,* 101, 1307 (1987)], Moloney mouse leukemia virus LTR promoter *[Biochem. Biophys. Res. Commun.,* 149, 960 (1987)], immunoglobulin H chain promoter *[Cell,* 41, 479 (1985)] and enhancer [*Cell*, 33, 717 (1983)], and the like.

(2) Obtaining of DNA encoding Fc region of human antibody and protein to be fused

A DNA encoding the Fc region of a human antibody and the protein to be fused can be obtained in the following manner.

A cDNA is synthesized from mRNA extracted from a cell or tissue which expresses the protein of interest to be fused with Fc. The synthesized cDNA is cloned into a vector such as a phage or a plasmid to obtain a cDNA library. A recombinant phage or recombinant plasmid comprising cDNA encoding the protein of interest is isolated from the library by using the gene sequence part of the protein of interest as the probe. A full nucleotide sequence of the protein of interest on the recombinant phage or recombinant plasmid is determined, and a full length amino acid sequence is deduced from the nucleotide sequence.

As the non-human animal, any animal such as mouse, rat, hamster or rabbit can be used so long as a cell or tissue can be removed therefrom.

The method for preparing a total RNA from a cell or tissue includes the guanidine thiocyanate-cesium trifluoroacetate method *[Methods in Enzymology,* 154, 3 (1987)] and the like, and the method for preparing mRNA from total

RNA includes an oligo (dT)-immobilized cellulose column method *(Molecular Cloning,* Second Edition) and the like. In addition, a kit for preparing mRNA from a cell or tissue includes Fast Track mRNA Isolation Kit (manufactured by Invitrogen), Quick Prep mRNA Purification Kit (manufactured by Pharmacia) and the like.

The method for synthesizing a cDNA and preparing a cDNA library includes the usual methods *(Molecular Cloning,* Second Edition; *Current Protocols in Molecular Biology,* Supplement 1-34); methods using a commercially available kit such as SuperScript™, Plasmid System for cDNA Synthesis and Plasmid Cloning (manufactured by GIBCO BRL) or ZAP-cDNA Synthesis Kit (manufactured by Stratagene); and the like.

In preparing the cDNA library, the vector into which a cDNA synthesized by using mRNA extracted from a cell or tissue as the template is inserted may be any vector so long as the cDNA can be inserted. Examples include ZAP Express *[Strategies,* 5, 58 (1992)], pBluescript II SK(+) *[Nucleic Acids Research,* 17, 9494 (1989)], λzapII (manufactured by Stratagene), λgt10 and λgt11 [*DNA Cloning, A Practical Approach,* I, 49 (1985)], Lambda BlueMid (manufactured by Clontech), λExCell, pT7T3 18U (manufactured by Pharmacia), pcD2 [*Mol. Cell. Biol*., 3, 280 (1983)], pUC18 [*Gene*, 33, 103 (1985)] and the like.

As *Escherichia coli* into which the cDNA library constructed from a phage or plasmid vector is introduced, any *Escherichia coli* can be used, so long as the cDNA library can be introduced, expressed and maintained. Examples include XL1-Blue MRF' *[Strategies,* 5, 81 (1992)], C600 [*Genetics,* 39, 440 (1954)], Y1088 and Y1090 [*Science,* 222, 778 (1983)], NM522 [*J. Mol. Biol.,* 166, 1 (1983)], K802 [*J. Mol. Biol.,* 16, 118 (1966)], JM105 [*Gene*, 38, 275 (1985)] and the like.

As the method for selecting a cDNA clone encoding the protein of interest from the cDNA library, a colony hybridization or a plaque hybridization using an isotope- or fluorescence-labeled probe can be used *(Molecular Cloning,* Second Edition). The cDNA encoding the protein of interest can also be prepared by preparing primers and using a cDNA synthesized from mRNA or a cDNA library as the template according to PCR.

The method for fusing the protein of interest with the Fc region of a human antibody includes PCR. For example, synthesized oligo DNAs (primers) are designed at the 5'-terminal and 3'-terminal of the gene sequence encoding the protein of interest, and PCR is carried out using the primers to prepare a PCR product. In the same manner, primers are designed for the gene sequence encoding the Fc region of a human antibody to be fused to prepare a PCR product. At this time, the primers are designed in such a manner that the same restriction enzyme site or the same gene sequence is present in the 3'-terminal of the PCR product of the protein to be fused and the 5'-terminal of the PCR product of the Fc region. When it is necessary to modify the amino acids around the linked site, mutation is introduced by using the primer into which the mutation is introduced. PCR is further carried out by using the two kinds of the obtained PCR fragments to link the genes. Also, they can be linked by carrying out ligation after treatment with the same restriction enzyme.

The nucleotide sequence of the DNA can be determined by digesting the gene sequence linked by the above method with appropriate restriction enzymes, cloning the DNA fragments into a plasmid such as pBluescript SK(-) (manufactured by Stratagene), carrying out analysis by using a generally used nucleotide sequence analyzing method such as the dideoxy method of Sanger *et al*. [*Proc. Natl. Acad Sci. USA*, 74, 5463 (1977)] or a nucleotide sequence analyzer such as ABI PRISM 377 DNA Sequencer (manufactured by PE Biosystems).

Whether or not the obtained cDNA encodes the full length amino acid sequences of the Fc fusion protein comprising a secretory signal sequence can be confirmed by deducing the full length amino acid sequence of the Fc fusion protein from the determined nucleotide sequence and comparing it with the amino acid sequence of interest.

(3) Stable production of Fc fusion protein

A transformant capable of stably producing an Fc fusion protein can be obtained by introducing the Fc fusion protein expression vector described in the item (1) into an appropriate animal cell.

The method for introducing the Fc fusion protein expression vector into an animal cell include electroporation [Japanese Published Unexamined Patent Application No. 257891/90, *Cytotechnology,* 3, 133 (1990)] and the like.

As the animal cell into which the Fc fusion protein expression vector is introduced, any cell can be used, so long as it is an animal cell which can produce the Fc fusion protein.

Examples include mouse myeloma cells such as NS0 cell and SP2/0 cell, Chinese hamster ovary cells such as CHO/dhfr- cell and CHO/DG44 cell, rat myeloma such as YB2/0 cell and IR983F cell, BHK cell derived from a Syrian hamster kidney, a human myeloma cell such as Namalwa cell, and the like, and preferred are a Chinese hamster ovary cell CHO/DG44 cell, a rat myeloma YB2/0 cell, the host cells used in the method of the present invention described in the item 1 and the like.

After introduction of the Fc fusion protein expression vector, a transformant capable of stably producing the Fc fusion protein can be selected by using a medium for animal cell culture comprising an agent such as G418 in accordance with the method described in Japanese Published Unexamined Patent Application No. 257891/90. The medium to culture animal cells includes RPMI 1640 medium (manufactured by Nissui Pharmaceutical), GIT medium (manufactured by

Nihon Pharmaceutical), EX-CELL 302 medium (manufactured by JRH), IMDM medium (manufactured by GIBCO BRL), Hybridoma-SFM medium (manufactured by GIBCO BRL), media obtained by adding various additives such as insulin, an insulin-like growth factor, thransferrin and albumin to these media, and the like. The Fc fusion protein can be formed and accumulated in the culture supernatant by culturing the obtained transformant in a medium. The amount of production and antigen binding activity of the Fc fusion protein in the culture supernatant can be measured by a method such as ELISA. Also, the amount of the Fc fusion protein produced by the transformant can be increased by using a *dhfr* gene amplification system and the like in accordance with the method described in Japanese Published Unexamined Patent Application No. 257891/90.

The Fc fusion protein can be purified from a culture supernatant culturing the transformant by using a protein A column or a protein G column (*Antibodies*, Chapter 8; *Monoclonal Antibodies*). In addition, purification methods generally used for the purification of proteins can also be used. For example, the purification can be carried out through the combination of a gel filtration, an ion exchange chromatography, an ultrafiltration and the like. The molecular weight as a whole of the purified Fc fusion protein molecule can be measured by SDS-PAGE *[Nature,* 227, 680 (1970)], Western blotting (*Antibodies*, Chapter 12, *Monoclonal Antibodies*) or the like.

Thus, the processes for producing an antibody and Fc fusion protein composition using an animal cell as the host have been described, but, as described above, the antibody and the Fc fusion protein composition can also be produced by a yeast, an insect cell, a plant cell, an animal individual or a plant individual.

When a cell has the ability to express a glycoprotein such as an antibody molecule innately, the antibody composition or glycoprotein composition of the present invention can be produced by preparing a glycoprotein producing cell using the method described in the item 1, culturing the cell and then purifying the antibody composition or glycoprotein composition of interest from the resulting culture.

3. Method for producing the glycoprotein composition of the present invention by serum-free culturing

It is necessary to further naturalize the cell of the present invention in a serum-free medium. A glycoprotein composition in a serum-free or protein-free medium can be produced by producing a glycoprotein using the cell of the present invention.

The naturalization method in a serum-free medium in the present invention includes a method wherein a cell in which a genomic gene encoding the α-1,6-fucose modifying enzyme is knocked out is sub-cultured in a medium containing serum and then directly naturalized or continuously naturalized in a commercially available serum-free medium or the like (Cell & Tissue Culture: Laboratory Procedures, JOHN WILEY & SONS 2C:1) and the like. Specific examples are described below.

During the serum-free naturalization, there is a case in which the survival rate of the cell temporarily decreases, causing death of the cell. Thus, in order to restore the survival rate of the cell or keep it at a high level, it is preferable to inoculate the cell to give a cell density of $1\times10^5$ to $10\times10^5$ cells/ml, preferably $4\times10^5$ to $6\times10^5$ cells/ml, at the time when the cell is inoculated into a serum-free naturalization medium. For example, in the case of a direct naturalization method, the cell is inoculated into a medium and cultured at 37°C in a 5% $CO_2$ incubator using a conventional animal cell culture method such as batch culture, and when the cell density reaches $10\times10^5$ to $40\times10^5$ cells/ml, the cells are inoculated into a serum-free medium and cultured under similar conditions.

The cell in which a genomic gene encoding the α-1,6-fucose modifying enzyme is knocked out is inoculated into a serum-free medium to give a density of $1\times10^5$ to $10\times10^5$ cells/ml, preferably $4\times10^5$ to $6\times10^5$ cells/ml, and 4 to 7 days after culturing according to a conventional animal cell culture method, the cells which reach a cell density of $10\times10^5$ to $40\times10^5$ cells/ml are selected as cells to be naturalized in a serum-free medium.

Subculture of the cells naturalized in the serum-free medium can be carried out by inoculating them into a medium to be used in the batch culture, which is described later, to give a density of $10\times10^5$ to $30\times10^5$ cells/ml, and culturing them for 3 to 5 days under the culture conditions to be used in the batch culture, which is described later. In this connection, it is preferable to keep the survival rate of the cells naturalized in the serum-free medium at 90% or more during the sub-culturing. In addition, regarding the cell in which a genomic gene encoding the α-1,6-fucose modifying enzyme is knocked out, such as a cell in which the genomic gene of α-1,6-fucosyltransferase was knocked out or a transformed cell of the cell, albumin may be added to the serum-free medium preferably in an amount of 0.1 to 10 g/l, more preferably 0.5 to 3 g/l, for the purpose of keeping productivity of a desired glycoprotein by the cells naturalized in the serum-free medium.

After the naturalization of the cell using the method of the present invention for its naturalization in a serum-free medium, a cloned (single cell) cell line can be prepared by using a limiting dilution method by a 96-well plate, a colony forming method or the like.

A method for preparing a cloned cell line by limiting dilution method is described below.

A cell suspension is diluted, inoculated into a plate at such a density that the cells are dispensed at a frequency of one or less cell per well, and cultured at 30 to 40°C for several weeks in a 5% $CO_2$ incubator using commercially

available serum-free medium and the like. After completion of the culturing, the concentration of a desired glycoprotein in cell culture supernatants where cell growth is observed is examined, and a cell having high productivity of said glycoprotein is selected.

The cloning method using a colony forming method is as follows.

In the case of adherent cells, cells are inoculated into a plate by diluting a cell suspension and cultured, and then formation of colonies is confirmed. The colonies are separated using a ring such as a penicillin cap, the cells are separated using an enzyme such as trypsin and transferred to appropriate culture devices, and then productivity of the desired glycoprotein is examined to select a cell having high productivity of the glycoprotein.

In the case of suspension cells, a cell suspension is diluted, cells thereof are inoculated into a soft agar and cultured, and the formed colonies are picked up under a microscope and returned to a static culturing, and then productivity of the desired glycoprotein is examined to select a cell having high productivity.

By repeatedly carrying out the above-described method, a clone in which a genomic gene encoding the α-1,6-fucose modifying enzyme is knocked out, wherein it has characteristics of interest and is naturalized in the serum-free medium, can be selected.

By the above-described method, a clone in which a genomic gene of an α-1,6-fucose modifying enzyme is knocked out, wherein it is naturalized in the serum-free medium, can be obtained.

The above-described clone in which a genomic gene encoding the α-1,6-fucose modifying enzyme is knocked out, wherein it is naturalized in the serum-free medium, can be sub-cultured by a method in which the above-described cell naturalized in the serum-free medium is sub-cultured. The cell in which a genomic gene encoding the α-1,6-fucose modifying enzyme is knocked out, wherein it is naturalized in the serum-free medium, using the naturalization method to a serum-free medium in the present invention includes cells prepared by naturalizing WK704-2B8P (FERM BP-8337), WK704-2871 (FERM BP-8336) and WK704-2760 (FERM BP-8335) in a serum-free medium, and the like.

In this connection, the naturalization method of a cell in a protein-free medium in the present invention can also be carried out a method similar to the above-described naturalization method of a cell in a serum-free medium.

As the culturing method of a cell in the present invention, any one of the generally used animal cell culturing methods can be used, so long as it is a method which can efficiently produce a desired glycoprotein composition. Examples include batch culture, repeat batch culture, fed-batch culture, perfusion culture and the like, and fed-batch culture and perfusion culture are preferable for the purpose of increasing productivity of the desired glycoprotein.

(1) Batch culture

As the serum-free medium used in the culturing method of a cell in the present invention, those in which various physiologically active substances and nutrient factors are added, instead of serum, to a basal medium which is used in the general animal cell culturing, and is also supplemented with carbon sources, nitrogen sources and the like which can be assimilated by animal cells.

Examples include RPMI 1640 medium [*The Journal of the American Medical Association*, 199, 519 (1967)], Eagle's MEM medium *[Science,* 122, 501 (1952)], Dulbecco's modified MEM medium [*Virology*, 8, 396 (1959)], 199 medium [*Proceeding of the Society for the Biological Medicine,* 73, 1 (1950)], F12 medium *[Proc. Natl. Acad Sci. USA*, 53, 288 (1965)], IMDM medium [*J. Experimental Medicine,* 147, 923 (1978)] and the like, and DMEM medium, F12 medium, IMDM medium and the like are preferably used.

If necessary, a nutrient factor, a physiologically active substance and the like which are necessary for the growth of animal cells are added to the serum-free medium. These additives are contained in the medium in advance before the culturing.

The nutrient factor includes glucose, an amino acid, a vitamin and the like.

The amino acid includes L-alanine, L-arginine, L-asparagine, L-aspartic acid, L-cystine, L-glutamic acid, L-glutamine, glycine, L-histidine, L-isoleucine, L-leucine, L-lysine, L-methionine, L-phenylalanine, L-proline, L-serine, L-threonine, L-tryptophan, L-tyrosine, L-valine and the like, which are used alone or in combination of two or more.

The vitamin includes d-biotin, D-pantothenic acid, choline, folic acid, myo-inositol, niacinamide, pyridoxal, riboflavin, thiamin, cyanocobalamin, DL-α-tocopherol and the like, which are used alone or in combination of two or more.

The physiologically active substance includes insulin, an insulin-like growth factor, transferrin, albumin and the like.

As the concentrations of the nutrient factors, the concentration of glucose is 200 to 6000 mg/l, preferably 3000 to 5000 mg/l.

As the concentrations of the amino acids, for example, 1 to 160 mg/l (preferably 3 to 120 mg/l) of L-alanine, 10 to 1000 mg/l (preferably 30 to 800 mg/l) of L-arginine monohydrochloride, 10 to 200 mg/l (preferably 20 to 150 mg/l) of L-asparagine monohydrate, 5 to 100 mg/l (preferably from 10 to 75 mg/l) of L-aspartic acid, 10 to 200 mg/l (preferably 20 to 150 mg/l) of L-cystine dihydrochloride, 5 to 200 mg/l (preferably 10 to 150 mg/l) of L-glutamic acid, 50 to 2000 mg/l (preferably 100 to 1500 mg/l) of L-glutamine, 2 to 100 mg/l (preferably 5 to 75 mg/l) of glycine, 5 to 200 mg/l (preferably

10 to 150 mg/l) of L-histidine monohydrochloride dihydrate, 2 to 300 mg/l (preferably 4 to 200 mg/l) of L-isoleucine, 5 to 300 mg/l (preferably 10 to 200 mg/l) of L-leucine, 10 to 300 mg/l (preferably 20 to 250 mg/l) of L-lysine monohydrochloride, 5 to 100 mg/l (preferably 10 to 75 mg/l) of L-methionine, 5 to 200 mg/l (preferably 10 to 150 mg/l) of L-phenylalanine, 5 to 200 mg/l (preferably 10 to 150 mg/l) of L-proline, 5 to 200 mg/l (preferably 10 to 150 mg/l) of L-serine, 5 to 200 mg/l (preferably 10 to 150 mg/l) of L-threonine, 1 to 40 mg/l (preferably 2 to 30 mg/l) of L-tryptophan, 2 to 300 mg/l (preferably 4 to 200 mg/l) of L-tyrosine disodium dihydrate and 5 to 300 mg/l (preferably 10 to 200 mg/l) of L-valine are cited.

As the concentrations of the vitamins, for example, 0.001 to 0.4 mg/l (preferably 0.002 to 0.3 mg/l) of d-biotin, 0.001 to 10.0 mg/l (preferably 0.002 to 7.5 mg/l) of calcium D-pantothenate, 0.1 to 20.0 mg/l (preferably 0.2 to 15.0 mg/l) of choline chloride, 0.005 to 20.0 mg/l (preferably 0.01 to 15.0 mg/l) of folic acid, 0.01 to 300 mg/l (preferably 0.05 to 200 mg/l) of myo-inositol, 0.01 to 20.0 mg/l (preferably 0.02 to 15.0 mg/l) of niacinamide, 0.01 to 15.0 mg/l (preferably 0.02 to 10.0 mg/l) of pyridoxal monohydrochloride, 0.005 to 2.0 mg/l (preferably 0.01 to 1.5 mg/l) of riboflavin, 0.005 to 20.0 mg/l (preferably 0.01 to 15.0 mg/l) of thiamin monohydrochloride and 0.001 to 5.0 mg/l (preferably 0.002 to 3.0 mg/l) of cyanocobalamin are cited.

As the concentrations of the physiologically active substances, for example, 10 to 500 mg/l, preferably 50 to 300 mg/l, of insulin; 10 to 500 mg/l, preferably 50 to 300 mg/l, of an insulin-like growth factor; 10 to 500 mg/l, preferably 50 to 300 mg/l, of transferrin; and 200 to 6000 mg/l, preferably 700 to 4000 mg/l, of albumin are cited.

The batch culture is carried out generally under conditions of, for example, pH 6 to 8 and 30 to 40°C, for 3 to 12 days. In addition, if necessary, antibiotics such as streptomycin and penicillin may be added to the medium during the culturing. In this connection, dissolved oxygen concentration control, pH control, temperature control, agitation and the like can be carried out in accordance with the methods generally used in the culturing of animal cells.

(2) Fed-batch culture

As the serum-free medium to be used in the culturing method of a cell in the present invention, those in which various physiologically active substances and nutrient factors are added, instead of serum, to a basal medium which is used in the general animal cell culturing, and is also supplemented with carbon sources, nitrogen sources and the like which can be assimilated by animal cells. Examples include RPMI 1640 medium [*The Journal of the American Medical Association*, 199, 519 (1967)], Eagle's MEM medium *[Science,* 122, 501 (1952)], Dulbecco's modified MEM medium [*Virology*, 8, 396 (1959)], 199 medium [*Proceeding of the Society for the Biological Medicine,* 73, 1 (1950)], F12 medium *[Proc. Natl. Acad. Sci. USA,* 53, 288 (1965)], IMDM medium [*J. Experimental Medicine,* 147, 923 (1978)] and the like, and DMEM medium, F12 medium, IMDM medium and the like are preferably used. In addition to the media described in the above, the serum-free medium described in the batch culture may also be used.

If necessary, a physiologically active substance, a nutrient factor and the like which are necessary for the growth of animal cells are added to the serum-free medium. These additives are contained in the medium in advance before the culturing, or if necessary, added optionally to the culture liquid during the culturing.

The nutrient factor includes glucose, an amino acid, a vitamin and the like.

The amino acid includes L-alanine, L-arginine, L-asparagine, L-aspartic acid, L-cystine, L-glutamic acid, L-glutamine, glycine, L-histidine, L-isoleucine, L-leucine, L-lysine, L-methionine, L-phenylalanine, L-proline, L-serine, L-threonine, L-tryptophan, L-tyrosine, L-valine and the like, which are used alone or in combination of two or more.

The vitamin includes d-biotin, D-pantothenic acid, choline, folic acid, myo-inositol, niacinamide, pyridoxal, riboflavin, thiamin, cyanocobalamin, DL-α-tocopherol and the like, which are.used alone or in combination of two or more.

As the physiologically active substances, insulin, insulin-like growth factor, transferrin, albumin and the like can be exemplified.

As the final concentrations of the nutrient factors to be added to the medium or culture liquid, the concentration of glucose is 200 to 6000 mg/l, preferably 1000 to 5000 mg/l.

As the amino acids, for example, 1 to 960 mg/l (preferably 1 to 640 mg/l) of L-alanine, 10 to 6000 mg/l (preferably 11 to 4000 mg/l) of L-arginine monohydrochloride, 10 to 1200 mg/l (preferably 11 to 800 mg/l) of L-asparagine monohydrate, 5 to 600 mg/l (preferably 5 to 400 mg/l) of L-aspartic acid, 10 to 1200 mg/l (preferably 11 to 800 mg/l) of L-cystine dihydrochloride, 5 to 1200 mg/l (preferably 5 to 800 mg/l) of L-glutamic acid, 53 to 12000 mg/l (preferably 55 to 8000 mg/l) of L-glutamine, 2 to 600 mg/l (preferably 2 to 400 mg/l) of glycine, 5 to 1200 mg/l (preferably 5 to 800 mg/l) of L-histidine monohydrochloride dihydrate, 4 to 1800 mg/l (preferably 4 to 1200 mg/l) of L-isoleucine, 3 to 1800 mg/l (preferably 14 to 1200 mg/l) of L-leucine, 10 to 1800 mg/l (preferably 11 to 1200 mg/l) of L-lysine monohydrochloride, 4 to 600 mg/l (preferably 5 to 400 mg/l) of L-methionine, 5 to 1200 mg/l (preferably 5 to 800 mg/l) ofL-phenylalanine, 5 to 1200 mg/l (preferably 5 to 800 mg/l) of L-proline, 5 to 1200 mg/l (preferably 5 to 800 mg/l) of L-serine, 5 to 1200 mg/l (preferably 5 to 800 mg/l) of L-threonine, 1 to 240 mg/l (preferably 1 to 160 mg/l) of L-tryptophan, 8 to 1800 mg/l (preferably 8 to 1200 mg/l) of L-tyrosine disodium dihydrate, and 12 to 1800 mg/l (preferably 12 to 1200 mg/l) of L-valine are cited.

As the vitamin, for example, 0.001 to 2.4 mg/l (preferably 0.001 to 1.6 mg/l) of d-biotin, 0.011 to 60 mg/l (preferably 0.011 to 40 mg/l) of calcium D-pantothenate, 0.11 to 90 mg/l (preferably 0.11 to 60 mg/l) of choline chloride,

0.01 to 120 mg/l (preferably 0.01 to 80 mg/l) of folic acid, 0.05 to 1800 mg/l (preferably 0.05 to 1200 mg/l) of myo-inositol, 0.02 to 120 mg/l (preferably 0.03 to 80 mg/l) of niacinamide, 0.02 to 90 mg/l (preferably 0.03 to 60 mg/l) of pyridoxal monohydrochloride, 0.01 to 12 mg/l (preferably 0.01 to 98 mg/l) of riboflavin, 0.01 to 120 mg/l (preferably 0.01 to 80 mg/l) of thiamin monohydrochloride and 0.001 to 30 mg/l (preferably 0.001 to 20 mg/l) of cyanocobalamin are cited.

As the final amounts of the physiologically active substances to be added to the medium or culture liquid, for example, 10 to 3000 mg/l, preferably 11 to 2000 mg/l of insulin; 10 to 3000 mg/l, preferably 11 to 2000 mg/l of an insulin-like growth factor; 10 to 3000 mg/l, preferably 11 to 2000 mg/l of transferrin; and 200 to 36000 mg/l, preferably 220 to 24000 mg/l of albumin are cited.

According to the culturing method of a cell in the present invention, the "final amounts of substances to be added" is calculated after final completion of the addition of the concentrated culture liquid to be added during the fed-batch culturing and expressed as a value obtained by dividing the sum total of the weight of said substance contained in the medium and the weight of said substance added to the culture liquid by the sum total of the amount of the medium and the amount of the added concentrated culture liquid.

In carrying out the fed-batch culturing, it is preferable to add each of the physiologically active substances, nutrient factors and the like at a concentration higher than the generally used concentration. For example, 1/30 to 1/3, preferably 1/20 to 1/5, of the culture liquid volume is added as a portion of the feeding. When added to the culture liquid, it is preferable to supplementarily add it during the period of culturing continuously or separately for several times to 10 and several times. According to the fed-batch culture method described in the above in which the physiologically active substances, nutrient factors and the like are added continuously or intermittently in small portions, it has high metabolic efficiency of cells, decrease of achieving cell density of the cultured cells due to accumulation of waste matter in the culture liquid can be prevented, and concentration of the glycoprotein of interest in the recovered culture liquid is high in comparison with the batch culture method, so that separation and purification of the glycoprotein becomes easy, and production of the glycoprotein per medium can be increased in comparison with the batch culture.

The fed-batch culture is carried out generally at pH 6 to 8 and 30 to 40°C for 3 to 12 days. In addition, if necessary, antibiotics such as streptomycin and penicillin may be added to the medium during the culturing. In this connection, dissolved oxygen concentration control, pH control, temperature control, agitation and the like can be carried out in accordance with the methods generally used in the culturing of animal cells.

(3) Perfusion culture

As the serum-free medium to be used in the culturing method of a cell in the present invention, those in which various physiologically active substances and nutrient factors are added, instead of serum, to a basal medium which is used in the general animal cell culturing, and is also supplemented with carbon sources, nitrogen sources and the like which can be assimilated by animal cells in general. Examples include RPMI 1640 medium [*The Journal of the American Medical Association*, 199, 519 (1967)], Eagle's MEM medium [*Science*, 122, 501 (1952)], Dulbecco's modified MEM medium [*Virology*, 8, 396 (1959)], 199 medium [*Proceeding of the Society for the Biological Medicine,* 73, 1 (1950)], F12 medium [*Proc. Natl. Acad. Sci. USA,* 53, 288 (1965)], IMDM medium [*J. Experimental Medicine,* 147, 923 (1978)] and the like, and DMEM medium, F12 medium, IMDM medium and the like are preferably used. In addition to the media described above, the serum-free medium described in the batch culture may also be used.

If necessary, a physiologically active substance, a nutrient factor and the like which are necessary for the growth of animal cells are added to the serum-free medium. These additives may be contained in the medium before the culturing or in the medium to be fed into the culture liquid.

The nutrient factor includes glucose, an amino acid, a vitamin and the like.

The amino acid includes L-alanine, L-arginine, L-asparagine, L-aspartic acid, L-cystine, L-glutamic acid, L-glutamine, glycine, L-histidine, L-isoleucine, L-leucine, L-lysine, L-methionine, L-phenylalanine, L-proline, L-serine, L-threonine, L-tryptophan, L-tyrosine, L-valine and the like, which are used alone or in combination of two or more.

The vitamin includes d-biotin, D-pantothenic acid, choline, folic acid, myo-inositol, niacinamide, pyridoxal, riboflavin, thiamin, cyanocobalamin, DL-$\alpha$-tocopherol and the like, which are used alone or in combination of two or more.

The physiologically active substance includes insulin, insulin-like growth factor, transferrin, albumin and the like.

As the concentrations of the nutrient factors, the concentration of glucose is controlled to 500 to 6000 mg/l, preferably 1000 to 2000 mg/l.

The nutrient factors include an amino acid, a vitamin and the like. As the amounts of other physiologically active substances or nutrient factors to be added, for example, 4 to 560 mg/ml, preferably from 20 to 360 mg/ml, of insulin; 4 to 560 mg/ml, preferably 20 to 360 mg/ml, of an insulin-like growth factor; 4 to 560 mg/ml, preferably 20 to 360 mg/ml, of transferrin; and 80 to 6500 mg/ml, preferably 280 to 4500 mg/ml of albumin are cited.

The amino acid includes L-alanine, L-arginine, L-asparagine, L-aspartic acid, L-cystine, L-glutamic acid, L-glutamine, glycine, L-histidine, L-isoleucine, L-leucine, L-lysine, L-methionine, L-phenylalanine, L-proline, L-serine, L-threonine, L-tryptophan, L-tyrosine, L-valine and the like, which are used alone or in combination of two or more. As the

concentrations of the amino acids, for example, 1 to 200 mg/l (preferably 2 to 160 mg/l) of L-alanine, 10 to 1140 mg/l (preferably 30 to 940 mg/l) of L-arginine monohydrochloride, 10 to 250 mg/l (preferably 20 to 200 mg/l) of L-asparagine monohydrate, 5 to 148 mg/l (preferably 10 to 120 mg/l) of L-aspartic acid, 10 to 350 mg/l (preferably 20 to 300 mg/l) of L-cystine dihydrochloride, 5 to 320 mg/l (preferably 10 to 270 mg/l) of L-glutamic acid, 50 to 3300 mg/l (preferably 100 to 1800 mg/l) of L-glutamine, 2 to 148 mg/l (preferably 5 to 123 mg/l) of glycine, 5 to 270 mg/l (preferably 10 to 220 mg/l) of L-histidine monohydrochloride dihydrate, 4 to 470 mg/l (preferably 4 to 370 mg/l) of L-isoleucine, 10 to 470 mg/l (preferably 13 to 370 mg/l) of L-leucine, 10 to 530 mg/l (preferably 20 to 480 mg/l) of L-lysine monohydrochloride, 4 to 150 mg/l (preferably 4 to 120 mg/l) of L-methionine, 4 to 310 mg/l (preferably 4 to 260 mg/l) of L-phenylalanine, 5 to 270 mg/l (preferably 10 to 210 mg/l) of L-proline, 5 to 270 mg/l (preferably 10 to 220 mg/l) of L-serine, 5 to 350 mg/l (preferably 10 to 300 mg/l) of L-threonine, 1 to 65 mg/l (preferably 2 to 55 mg/l) of L-tryptophan, 4 to 470 mg/l (preferably 8 to 370 mg/l) of L-tyrosine disodium dihydrate, and 10 to 450 mg/l (preferably 11 to 350 mg/l) of L-valine are cited.

The vitamin includes d-biotin, D-pantothenic acid, choline, folic acid, myo-inositol, niacinamide, pyridoxal, riboflavin, thiamin, cyanocobalamin, DL-$\alpha$-tocopherol and the like, which are used alone or in combination of two or more. As the final amounts of the vitamins to be added, for example, 0.001 to 0.44 mg/l (preferably 0.02 to 0.34 mg/l) of d-biotin, 0.01 to 16 mg/l (preferably 0.02 to 14 mg/l) of calcium D-pantothenate, 0.1 to 21 mg/l (preferably 30.2 to 16 mg/l) of choline chloride, 0.01 to 26 mg/l (preferably 0.01 to 21 mg/l) of folic acid, 0.05 to 310 mg/l (preferably 0.05 to 211 mg/l) of myo-inositol, 0.02 to 26 mg/l (preferably 0.02 to 21 mg/l) of niacinamide, 0.02 to 21 mg/l (preferably 0.02 to 16 mg/l) of pyridoxal monohydrochloride, 0.01 to 2.6 mg/l (preferably 0.01 to 2.6 mg/l) of riboflavin, 0.01 to 26 mg/l (preferably 0.01 to 21 mg/l) of thiamin monohydrochloride, and 0.001 to 5 mg/l (preferably 0.002 to 3 mg/l) of cyanocobalamin are cited.

In the culturing method of a cell in the present invention, the culture is efficiently separated by a generally used device for separating a culture and cells, the concentrated cell suspension is returned to the original culture vessel, and the fresh medium corresponding to the reduced volume is newly supplemented. By this, the proper culturing environment is constantly maintained.

In the case of the cell of the present invention, apart from the medium exchange ratio with fresh medium, productivity of the glycoprotein of interest can be increased by stabilizing the culturing system through discharge of the growing cells into outside of the culturing system in response to the growth rate of the cells. For example, a culturing having high productivity becomes possible when 2/5 to 3/5 of the total cells present in the culture vessel are discharged outside of the system at the doubling time of the cells, by adjusting the rate of discharging the cells outside of the system to the cell growth rate in such a manner that the intended cell density can be maintained.

According to the present invention, the culturing is carried out generally under conditions of, for example, pH 6 to 8 and 30 to 40°C, for 10 to 40 days. In addition, if necessary, antibiotics such as streptomycin and penicillin may be added to the medium during the culturing. In this connection, dissolved oxygen concentration control, pH control, temperature control, agitation and the like can be carried out in accordance with the methods generally used in the culturing of animal cells.

As described above, the glycoprotein of interest can be produced by culturing the cell of the present invention in which a genomic gene encoding the $\alpha$-1,6-fucose modifying enzyme is knocked out to thereby form and accumulate the desired glycoprotein, and recovering the glycoprotein from the culture.

When the cell is grown by the culturing method of a cell in the present invention, it is desirable to carry out the culturing by keeping the insulin concentration in the culture liquid at 10 mg/l or more, preferably 20 mg/l or more. On the other hand, when the glycoprotein of interest is produced, it is desirable to carry out the culturing, for example, by keeping the insulin concentration in the culture liquid at 10 mg/l or less, preferably 5 mg/l or less. In this connection, when insulin is contained in the preculture medium, insulin may not be added thereto for the purpose of increasing productivity of the antibody, but it is desirable in general to keep the insulin concentration in the medium at a level of 0.01 to 10 mg/l, preferably 0.01 to 5 mg/l.

The method for adjusting insulin concentration in the culture is suitably used in culturing which can adjust the concentration of insulin, such as fed-bath culture or perfusion culture.

In this connection, the method for culturing a cell can also be carried out in accordance with the above-described method by employing a method similar to the culturing method that uses a serum-free medium, using a medium which is free from proteins such as serum, insulin, insulin-like growth factor, transferrin and albumin. The glycoprotein composition of interest can be produced by the culturing method.

4. Activity evaluation of glycoprotein composition

Methods for measuring a protein amount of the purified glycoprotein composition, affinity to the receptor, half-life in blood, distribution in tissue after administer into blood and change of protein interaction necessary for expression of pharmacological activity are measured by known methods described in *Current Protocols In Protein Science,* John Wiley & Sons Inc., (1995); *New Biochemical Experimentation Series 19-Animal Experimental Test,* Tokyo Kagaku Dojin, edited by Japanese Biochemical Society (1991); *New Biochemical Experimentation Series 8-Intracellular Information*

*and Cell Response,* Tokyo Kagaku Dojin, edited by Japanese Biochemical Society (1990); *New Biochemical Experimentation Series 9-Hormone I, Peptide hormone,* Tokyo Kagaku Dojin, edited by Japanese Biochemical Society (1991); *Experimental Biology Course 3-Isotope Experimental Test,* Maruzen (1982); *Monoclonal Antibodies: Principles and Applications*, Wiley-Liss, Inc., (1995); *Enzyme-Linked Immuno Adsorbent Assay,* 3rd Ed., Igaku Shoin (1987); *Revised Enzyme Immunoassay,* Gakusai Kikaku (1985); and the like.

Specific examples include a method in which a purified glycoprotein composition is labeled with a compound such as a radioisotope and binding activity to a receptor of the labeled glycoprotein composition or an interacted protein is quantitatively determined. Furthermore, interaction between the proteins can be measured by using various apparatus such as BIAcore Series manufactured by Biacore *(J. Immnunol. Methods,* 145, 229 (1991); *Experimental Medicine Supplement, Biomanual UP Series, Experimental Test of Intermolecular Interaction Experimental Test,* Yodo-sha (1996)).

By administration of the labeled glycoprotein into the living body, the half-life in blood and the distribution of the glycoprotein in tissue after administered into the living body can be known. Detection of the labeled material is preferably carried out by a detection method in which a method for detecting a labeled substance is combined with an antigen-antibody reaction specific to the glycoprotein which is to be detected.

5. Activity evaluation of antibody composition

When the glycoprotein composition is an antibody composition, known methods described in *Monoclonal Antibodies, Antibody Engineering* and the like can be used as the methods for measuring the protein amount of the purified antibody composition, the binding activity to an antigen and the effector function.

For example, when the antibody composition is a humanized antibody, the binding activity to an antigen and the binding activity to an antigen-positive cultured clone can be measured by methods such as ELISA and an immunofluorescent method *[Cancer Immunol. Immunother.,* 36, 373 (1993)]. The cytotoxic activity against an antigen-positive cultured clone can be evaluated by measuring CDC activity, ADCC activity [*Cancer Immunol. Immunother.,* 36, 373 (1993)] and the like.

Also, safety and therapeutic effect of the antibody composition in human can be evaluated by using an appropriate model of animal species relatively close to human, such as *Macaca fascicularis*.

6. Analysis of sugar chains in glycoprotein composition

The sugar chain structure in the glycoprotein composition expressed in various cells can be analyzed in accordance with the general analysis of the sugar chain structure of a glycoprotein. For example, the sugar chain which is bound to IgG molecule in the antibody composition comprises a neutral sugar such as galactose, mannose or fucose, an amino sugar such as N-acetylglucosamine and an acidic sugar such as sialic acid, and can be analyzed by a method, such as a sugar chain structure analysis, using sugar composition analysis, two dimensional sugar chain mapping or the like.

(1) Analysis of neutral sugar and amino sugar compositions

The sugar chain composition in the glycoprotein composition can be analyzed by carrying out acid hydrolysis of sugar chains with trifluoroacetic acid or the like to release a neutral sugar or an amino sugar and analyzing the composition ratio.

Examples include a method using a sugar composition analyzer (BioLC) manufactured by Dionex. The BioLC is an apparatus which analyzes a sugar composition by HPAEC-PAD (high performance anion-exchange chromatography-pulsed amperometric detection) [*J. Liq. Chromatogr.*, 6, 1577 (1983)].

The composition ratio can also be analyzed by a fluorescence labeling method using 2-aminopyridine. Specifically, the composition ratio can be calculated in accordance with a known method [*Agric. Biol. Chem.*, 55(1), 283-284 (1991)], by labeling an acid-hydrolyzed sample with a fluorescence with 2-aminopyridylation and then analyzing the composition by HPLC.

(2) Analysis of sugar chain structure

The sugar chain structure of the glycoprotein composition can be analyzed by the two dimensional sugar chain mapping method [*Anal. Biochem.,* 171, 73 (1988), *Biochemical Experimentation Methods 23 - Methods for Studying Glycoprotein Sugar Chains* (Japan Scientific Societies Press) edited by Reiko Takahashi (1989)]. The two dimensional sugar chain mapping method is a method for deducing a sugar chain structure by, e.g., plotting the retention time or elution position of a sugar chain by reverse phase chromatography as the X axis and the retention time or elution position

of the sugar chain by normal phase chromatography as the Y axis, respectively, and comparing them with those of known sugar chains.

Specifically, sugar chains are released from a glycoprotein composition by hydrazinolysis, and the released sugar chain is subjected to fluorescence labeling with 2-aminopyridine (hereinafter referred to as "PA") [*J. Biochem.,* 95, 197 (1984)], and then the sugar chains are separated from an excess PA-treating reagent and the like by gel filtration, and subjected to reverse phase chromatography. Thereafter, each peak of the separated sugar chains are subjected to normal phase chromatography. The sugar chain structure can be deduced by plotting the results on a two dimensional sugar chain map and comparing them with the spots of a sugar chain standard (manufactured by Takara Shuzo Co., Ltd.) or a literature *[Anal. Biochem.,* 171, 73 (1988)].

The structure deduced by the two dimensional sugar chain mapping method can be confirmed by further carrying out mass spectrometry such as MALDI-TOF-MS of each sugar chain.

7. Application of the glycoprotein composition

Since the glycoprotein composition produced by the present invention has a sugar chain structure to which fucose is not bound, for example, effects, such as improvement of affinity with a receptor, improvement of serum half-life, improvement of tissue distribution after administration into blood and improvement of its interaction with a protein necessary for pharmacological activity, can be expected and high physiological activity can be obtained. Particularly, when it is an antibody composition, it has high effector function, namely ADCC activity. The above glycoprotein composition having high physiological activity, particularly the antibody composition having high ADCC activity, is useful for preventing and treating various diseases including cancers, inflammatory diseases, immune diseases such as autoimmune diseases, allergies and the like, cardiovascular diseases and various diseases which are caused by viral and bacterial infections.

In the case of cancers, namely malignant tumors, cancer cells grow. General anti-tumor agents inhibit the growth of cancer cells. In contrast, an antibody having high ADCC activity can treat cancers by injuring cancer cells through its cell killing effect, and therefore, it is more effective as a therapeutic agent than the general anti-tumor agents. At present, in the therapeutic agent for cancers, an anti-tumor effect of an antibody medicament alone is not sufficient, so that combination therapy with chemotherapy has been carried out [*Science,* 280, 1197 (1998)]. Since the antibody composition produced by the present invention having high anti-tumor effect, the dependency on chemotherapy is decreased and side effects is reduced.

In immune diseases such as inflammatory diseases, autoimmune diseases and allergies, *in vivo* reactions of the diseases are induced by the release of a mediator molecule by immunocytes. For example, the allergy reaction can be suppressed by eliminating immunocytes using an antibody having high ADCC activity.

The cardiovascular diseases include arteriosclerosis and the like. The arteriosclerosis is treated using balloon catheter at present, but cardiovascular diseases can be prevented and treated by inhibiting growth of arterial cells in restricture after balloon catheter treatment using an antibody having high ADCC activity.

Various diseases including viral and bacterial infections can be prevented and treated by inhibiting proliferation of cells infected with a virus or bacterium using an antibody having high ADCC activity.

An antibody which recognizes a tumor-related antigen, an antibody which recognizes an allergy- or inflammation-related antigen, an antibody which recognizes cardiovascular disease-related antigen, an antibody which recognizes autoimmune disease-related antigen and an antibody which recognizes a viral or bacterial infection-related antigen are exemplified below.

The antibody which recognizes a tumor-related antigen includes anti-GD2 antibody [*Anticancer Res.,* 13, 331 (1993)], anti-GD3 antibody [*Cancer Immunol. Immunother.,* 36, 260 (1993)], anti-GM2 antibody [*Cancer Res.,* 54, 1511 (1994)], anti-HER2 antibody [*Proc. Natl. Acad. Sci. USA*, 89, 4285 (1992)], anti-CD52 antibody [*Nature,* 332, 323 (1998)], anti-MAGE antibody [*British J. Cancer,* 83, 493 (2000)], anti-HM1.24 antibody [*Molecular Immunol.*, 36, 387 (1999)], anti-parathyroid hormone-related protein (PTHrP) antibody [*Cancer,* 88, 2909 (2000)], anti-FGF8 antibody [*Proc. Natl. Acad Sci. USA*, 86, 9911 (1989)], anti-basic fibroblast growth factor antibody, anti-FGF8 receptor antibody [*J. Biol. Chem.*, 265, 16455 (1990)], anti-basic fibroblast growth factor receptor antibody, anti-insulin-like growth factor antibody [*J. Neurosci. Res.,* 40, 647 (1995)], anti-insulin-like growth factor receptor antibody [*J. Neurosci. Res.,* 40, 647 (1995)], anti-PMSA antibody [*J. Urology,* 160, 2396 (1998)], anti-vascular endothelial cell growth factor antibody [*Cancer Res.,* 57, 4593 (1997)], anti-vascular endothelial cell growth factor receptor antibody [*Oncogene,* 19, 2138 (2000)], anti-CA125 antibody, anti-17-1A antibody, anti-integrin $\alpha v\beta 3$ antibody, anti-CD33 antibody, anti-CD22 antibody, anti-HLA antibody, anti-HLA-DR antibody, anti-CD20 antibody, anti-CD 19 antibody, anti-EGF receptor antibody [*Immunology Today,* 21, 403 (2000)], anti-CD10 antibody [*American Journal of Clinical Pathology,* 113, 374 (2000)] and the like.

The antibody which recognizes an allergy- or inflammation-related antigen includes anti-interleukin 6 antibody [*Immunol. Rev.,* 127, 5 (1992)], anti-interleukin 6 receptor antibody [*Molecular Immunol.*, 31, 371 (1994)], anti-interleukin 5 antibody [*Immunol. Rev.,* 127, 5 (1992)], anti-interleukin 5 receptor antibody and anti-interleukin 4 antibody [*Cytokine*,

3, 562 (1991)], anti-interleukin 4 receptor antibody [*J. Immunol. Meth.*, 217, 41 (1998)], anti-tumor necrosis factor antibody [*Hybridoma,* 13, 183 (1994)], anti-tumor necrosis factor receptor antibody [*Molecular Pharmacol.*, 58, 237 (2000)], anti-CCR4 antibody [*Nature*, 400, 776 (1999)], anti-chemokine antibody [*J. Immuno. Meth.*, 174, 249 (1994)], anti-chemokine receptor antibody [*J. Exp. Med.*, 186, 1373 (1997)], anti-IgE antibody, anti-CD23 antibody, anti-CD11a antibody [*Immunology Today,* 21, 403 (2000)], anti-CRTH2 antibody [*J Immunol.*, 162, 1278 (1999)], anti-CCR8 antibody (WO99/25734), anti-CCR3 antibody (US6207155) and the like.

The antibody which recognizes a cardiovascular disease-related antigen includes anti-GpIIb/IIIa antibody [*J. Immunol.*, 152, 2968 (1994)], anti-platelet-derived growth factor antibody [*Science,* 253, 1129 (1991)], anti-platelet-derived growth factor receptor antibody [*J. Biol. Chem.,* 272, 17400 (1997)], anti-blood coagulation factor antibody [*Circulation,* 101, 1158 (2000)] and the like.

The antibody which recognizes an antigen relating to autoimmune diseases (for example, psoriasis, rheumarthritis, Crohn's diseases, colitis ulcerosa, systemic erythematodes, disseminated sclerosis and the like) includes an anti-auto-DNA antibody [*Immunol. Letters,* 72, 61 (2000)], anti-CD11a antibody, anti-ICAM3 antibody, anti-CD80 antibody, anti-CD2 antibody, anti-CD3 antibody, anti-CD4 antibody, anti-integrin $\alpha 4\beta 7$ antibody, anti-CD40L antibody, anti-IL-2 receptor antibody [*Immunology Today*, 21, 403 (2000)], and the like.

The antibody which recognizes a viral or bacterial infection-related antigen includes anti-gp120 antibody [*Structure,* 8, 385 (2000)], anti-CD4 antibody [*J. Rheumatology,* 25, 2065 (1998)], anti-CCR4 antibody, anti-Vero toxin antibody [*J. Clin. Microbiol.,* 37, 396 (1999)], and the like.

These antibodies can be obtained from public organizations such as ATCC (The American Type Culture Collection), RIKEN Gene Bank at The Institute of Physical and Chemical Research, and National Institute of Bioscience and Human Technology, Agency of Industrial Science and Technology, or private reagent sales companies such as Dainippon Pharmaceutical, R & D SYSTEMS, PharMingen, Cosmo Bio and Funakoshi.

The medicament comprising the glycoprotein composition in the present invention can be administered as a therapeutic agent alone, but generally, it is preferred to provide it as a pharmaceutical formulation prepared by an arbitrary method well known in the technical field of pharmaceuticals, by mixing it with one or more pharmaceutically acceptable carriers.

It is desirable to select a route of administration which is most effective for treatment. Examples include oral administration and parenteral administration, such as buccal, tracheal, rectal, subcutaneous, intramuscular or intravenous. In the case of a glycoprotein antibody preparation, intravenous administration is preferred.

The dosage form includes sprays, capsules, tablets, granules, syrups, emulsions, suppositories, injections, ointments, tapes and the like.

The pharmaceutical preparation suitable for oral administration includes emulsions, syrups, capsules, tablets, powders, granules and the like.

Liquid preparations, such as emulsions and syrups, can be produced using, as additives, water; sugars such as sucrose, sorbitol and fructose; glycols such as polyethylene glycol and propylene glycol; oils such as sesame oil, olive oil and soybean oil; antiseptics such as p-hydroxybenzoic acid esters; flavors such as strawberry flavor and peppermint; and the like.

Capsules, tablets, powders, granules and the like can be produced using, as additive, excipients such as lactose, glucose, sucrose and mannitol; disintegrating agents such as starch and sodium alginate; lubricants such as magnesium stearate and talc; binders such as polyvinyl alcohol, hydroxypropylcellulose and gelatin; surfactants such as fatty acid ester; plasticizers such as glycerine; and the like.

The pharmaceutical preparation suitable for parenteral administration includes injections, suppositories, sprays and the like.

Injections can be prepared using a carrier, such as a salt solution, a glucose solution, a mixture of both thereof or the like. Also, powdered injections can be prepared by freeze-drying the glycorprotein composition in the usual way and adding sodium chloride thereto.

Suppositories can be prepared using a carrier such as cacao butter, hydrogenated fat, carboxylic acid or the like.

Sprays can be prepared using the compound as such or using the glycorprotein composition together with a carrier which does not stimulate the buccal or airway mucous membrane of the patient and can facilitate absorption of the glycorprotein composition by dispersing it as fine particles.

The carrier includes lactose, glycerol and the like. Depending on the properties of the glycorprotein composition and the carrier used, it is possible to produce pharmaceutical preparations such as aerosols, dry powders and the like. In addition, the components exemplified as additives for oral preparations can also be added to the parenteral preparations.

Although the clinical dose or the frequency of administration varies depending on the objective therapeutic effect, administration method, treating period, age, body weight and the like, it is usually 10 μg/kg to 20 mg/kg per day and per adult.

Also, as the method for examining antitumor effect of the antibody composition against various tumor cells, *in*

*vitro* tests include CDC activity measuring method, ADCC activity measuring method and the like, and *in vivo* tests include antitumor experiments using a tumor system in an experimental animal such as a mouse, and the like.

CDC activity and ADCC activity and antitumor experiments can be carried out in accordance with the methods described in *Cancer Immunology Immunotherapy,* 36, 373 (1993); *Cancer Research,* 54, 1511 (1994) and the like.

## Brief Description of the Drawings

Fig. 1 shows construction of a plasmid pKOFUT8Neo.
Fig. 2 shows construction of a plasmid pBs-2B8L.
Fig. 3 shows construction of a plasmid pBs-2B8H and a plasmid pBs-2B8Hm.
Fig. 4 shows construction of a plasmid pKANTEX2B8P.
Fig. 5 shows ADCC activities of an anti-CD20 chimeric antibody purified from a FUT8 gene double knockout clone derived from CHO/DG44 cell to human B lymphocyte cultured cell line Raji cell. The ordinate and the abscissa show the cytotoxic activity and the antibody concentration, respectively.
Fig. 6 shows changes of viable cell density and survival rate when a cell naturalized in a protein-free medium is cultured in the protein-free medium.
Fig. 7 shows changes of viable cell density and survival rate in fed-batch culturing of a cell naturalized in a protein-free medium.
Fig. 8 shows changes of viable cell density (A), cell survival rate (B), cumulative viable cell density (C) and antibody density (D) in suspension agitation reactor serum-free fed-batch using clone Ms704/CD20 naturalized in the serum-free medium. The abscissa of each graph shows cultured days after commencement of the culturing.
Fig. 9 shows ADCC activities of two kinds of purified anti-CD20 human chimeric antibodies upon B cell in human peripheral blood. The ordinate shows the ratio of CD2-negative and CD19-positive human B cell in the lymphocyte fraction, and the abscissa shows the antibody concentration. Open circles show activity of an anti-CD20 human chimeric antibody (Ms704/CD20 antibody) produced by a suspension agitation reactor serum-free fed-batch culturing method using clone Ms704/CD20 naturalized in the serum-free medium, and closed circles show activity of an anti-CD20 human chimeric antibody (DG44/CD20 antibody) produced using the parent cell line CHO/DG44 cell.
Fig. 10 shows *in vitro* ADCC activities of two kinds of purified anti-CD20 human chimeric antibodies upon WIL2-S cell. The ordinate shows the cytotoxic activity, and the abscissa shows the antibody concentration. Open circles show activity of Ms704/CD20 antibody, and closed circles show that of DG44/CD20 antibody, respectively.
Fig. 11 shows *in vitro* ADCC activity of an anti-CD20 human chimeric antibody composition prepared by adding 0 to 300 ng/ml of DG44/CD20 antibody to 3.7 ng/ml of Ms704/CD20 antibody, upon WIL2-S cell. The ordinate shows the cytotoxic activity, and the abscissa shows the antibody concentration of added DG44/CD20 antibody. The symbol * in the drawing shows an antibody composition having 20% or more of the ratio of antibody having a sugar chain to which fucose is not bound.
Fig. 12 shows *in vitro* ADCC activities of an antibody composition consisting of Ms704/CD20 alone and an antibody composition prepared by mixing Ms704/CD20 antibody with 9-fold amount of DG44/CD20 antibody, upon WIL2-S cell. The ordinate shows the cytotoxic activity. The numerical values shown on the abscissa represent concentration of the Ms704/CD20 antibody, concentration of the added DG44/CD20 antibody, and total antibody concentration from the upper side. Open squares show the activity of the antibody composition consisting of Ms704/CD20 alone, and closed squares show that of the antibody composition prepared by mixing Ms704/CD20 antibody with 9-fold amount of DG44/CD20 antibody.

## Best Mode for Carrying Out the Invention

The present invention will be described below in detail based on Examples; however, Examples are only simple illustrations, and the scope of the present invention is not limited thereto.

### Example 1

### Construction of CHO/DG44 cell in which both alleles of α-1,6-fucosyltransferase (FUT8) gene on the genome have been disrupted

The cell line CHO/DG44 comprising the deletion of a genome region for both alleles of α-1,6-fucosyltransferase (hereinafter referred to as "FUT8") including the translation initiation codons was constructed according to the following steps.

1. Construction of Chinese hamster FUT8 gene targeting vector plasmid pKOFUT8Neo comprising exon 2

A plasmid pKOFUT8Neo was constructed in the following manner using targeting vector plasmid pKOFUT8Puro of exon 2 of Chinese hamster FUT8 gene constructed by the method described in Example 13-1 of WO02/31140, and a plasmid pKOSelectNeo (manufactured by Lexicon).

Using 16 units of a restriction enzyme *Asc*I (New England Biolabs), 1.0 μg of plasmid pKOSelectDT (manufactured by Lexicon) was allowed to react at 37°C for 2 hours. The reacting solution was subjected to agarose gel electrophoresis, and approximately 1.6 Kb *Asc*I fragment comprising the neomycin resistance gene expression unit was recovered using QIAquick Gel Extraction Kit (manufactured by QIAGEN).

Next, 1.0 μg of plasmid pKOFUT8Puro was allowed to react at 37°C for 2 hours by using 16 units of a restriction enzyme *Asc*I (manufactured by New England Biolabs). After the digestion reaction, the end of the DNA fragment was dephosphorylated with alkaline phosphatase derived from *Escherichia coli* C15 (manufactured by Takara Shuzo Co., Ltd.) according to the attached instructions. After the reaction, the DNA fragment was recovered by phenol/chloroform extraction and ethanol precipitation.

To 0.1 μg of the plasmid pKOSelectNeo-derived *Asc*I fragment (approximately 1.6 Kb) and 0.1 μg of the plasmid pKOFUT8Puro-derived *Asc*I fragment (approximately 10.1 Kb) obtained above, sterlized water was added to give a total volume of 5 μL, and 5 μL, of Ligation High (manufactured by Toyobo Co., Ltd.) was further added to react at 16°C for 30 minutes. *Escherichia coli* DH5α (manufactured by Toyobo Co., Ltd.) was transformed by using the resulting recombinant plasmid DNA. Each plasmid DNA was prepared from each transformant and each nucleotide sequence was analyzed by using BigDye Terminator Cycle Sequencing Ready Reaction Kit v2.0 (manufactured by Applied Biosystems) according to the attached instructions and DNA Sequencer ABI PRISM 377. The plasmid pKOFUT8Neo having the objective nucleotide sequence shown in Fig. 1 was obtained, and was used as a targeting vector for the preparation of FUT8 gene-knockout cell of CHO cell.

2. Preparation of CHO cell in which one copy of the FUT8 gene on the genome has been disrupted

(1) Obtaining of clone in which the targeting vector pKOFUT8Neo has been introduced

The Chinese hamster FUT8 genome region targeting vector pKOFUT8Neo constructed in Example 1-1 was introduced into Chinese hamster ovary-derived CHO/DG44 cells deficient in the dihydrofolate reductase gene (*dhfr*) [*Somataic Cell and Molecular Genetics,* 12, 555 (1986)] in the following manner.

After 280 μg of plasmid pKOFUT8Neo was allowed to react at 37°C for 5 hours by adding 400 units of a restriction enzyme *Sal*I (manufactured by New England Biolabs) for linearization, 4 μg of the linearized pKOFUT8Neo was introduced into $1.6 \times 10^6$ cells by electroporation *[Cytotechnology,* 3, 133 (1990)]. The resulting cells were suspended in IMDM-dFBS (10)-HT(1) [IMDM medium (manufactured by Invitrogen) containing 10% dialysis FBS (Invitrogen) and 1-fold concentration HT supplement (manufactured by Invitrogen)] and then inoculated into a 10-cm dish for adherent cell culture (manufactured by Falcon). After culturing in a 5% $CO_2$ incubator at 37°C for 24 hours, the medium was replaced with 10 ml of IMDM-dFBS(10) (IMDM medium containing 10% dialysis FBS) containing 600 μg/ml G418 (manufactured by Nacalai Tesque, Inc.). Culturing was carried out for 15 days while the above medium replacement was repeated every 3 to 4 days to obtain G418-resistant clones.

(2) Confirmation of homologous recombination by genomic PCR

Confirmation of the homologous recombination in the G418-resistant clones obtained in the above (1) was carried out by genomic PCR in the following manner.

The G418-resistant clones on a 96-well plate were subjected to trypsinization, and a 2-fold volume of a frozen medium (20% DMSO, 40% fetal calf serum and 40% IMDM) was added in each well. One half of the cell suspension in each well was inoculated into a flat-bottomed 96-well plate for adherent cells (manufactured by Asahi Techno Glass) to prepare a replica plate, while the other half was stored by cryopreservation as a master plate.

The neomycin-resistant clones on the replica plate were cultured using IMDM-dFBS(10) containing 600 μg/ml G418 for one week, followed by recovery of cells. The genomic DNA of each clone was prepared from the recovered cells according to a known method [*Analytical Biochemistry*, 201, 331 (1992)] and then dissolved overnight in 30 μl of TE-RNase buffer (pH 8.0) [10 mmol/l Tris-HCl, 1 mmol/l EDTA, 200 μg/ml RNase A].

Primers used in the genomic PCR were designed as follows. Primers which bind to the sequence of a part exceeding a targeting vector homologous region (SEQ ID NO:10 or 11) and primers which bind to the sequence within the vector (SEQ ID NO:12 or 13) in the FUT8 genome region obtained by the method described in Example 12 of WO03/31140 (SEQ ID NO:9) were prepared. The following polymerase chain reaction (PCR) was carried out by using them. Specifically, a reaction mixture [25 μl; DNA polymerase ExTaq (manufactured by Takara Shuzo Co., Ltd.), ExTaq

buffer (manufactured by Takara Shuzo Co., Ltd.), 0.2 mmol/l dNTPs, 0.5μ mol/l of the above gene-specific primers (forward primer: SEQ ID NO:10 or 11; reverse primer: SEQ ID NO:12 or 13)] containing 10 μl of each genomic DNA solution prepared above was prepared, and PCR was carried out, after heating at 94°C for 3 minutes, by cycles, one cycle consisting of reaction at 94°C for one minute, reaction at 60°C for one minute and reaction at 72°C for 2 minutes.

After the PCR, the reaction mixture was subjected to 0.8% (w/v) agarose gel electrophoresis, and clones with which a specific amplification (approximately 1.7 Kb) containing a boundary part of the CHO cell genomic region and the target vector homologous region was observed were determined to be positive clones.

(3) Confirmation of homologous recombination by genomic Southern blotting

Confirmation of the homologous recombination in the positive clones obtained in the above (2) was carried out by genomic Southern blotting in the following manner.

Among the master plates stored by cryopreservation in the above (2), a 96-well plate containing the positive clones found in (2) was selected. After the plate was allowed to stand at 5% $CO_2$ and 37°C for 10 minutes, the cells in the wells corresponding to the positive clones were inoculated into a flat-bottomed 24-well plate for adherent cells (manufactured by Greiner). After culturing using IMDM-dFBS(10) containing at a concentration of 600 μg/ml for one week, the cells were inoculated into a flat-bottomed 6-well plate for adherent cells (Greiner). The genomic DNA of each clone was prepared from the recovered cells in the plate according to a known method [*Nucleic Acids Research,* 3, 2303 (1976)] and then dissolved overnight in 150 μl of TE-RNase buffer (pH 8.0) [10 mmol/l Tris-HCl, 1 mmol/l EDTA, 200 μg/ml RNase A].

The genomic DNA prepared above (12 μg) was digested with 25 units of a restriction enzyme *Bam*HI (manufactured by New England Biolabs) at 37°C overnight. A DNA fragment was recovered by ethanol precipitation from the reaction mixture. The recovered fragment was dissolved in 20 μl of TE buffer (pH 8.0) (10 mmol/l Tris-HCl, 1 mmol/l EDTA) and then subjected to 0.6% (w/v) agarose gel electrophoresis. After the electrophoresis, the genomic DNA was transferred to a nylon membrane according to a known method [*Proc. Natl. Acad. Sci. USA,* 76, 3683 (1979)], followed by heat treatment of the nylon membrane at 80°C for 2 hours.

Separately, a probe used in the Southern blotting was prepared in the following manner. Firstly, PCR was carried out as follows by using primers which bind to the sequence of a part exceeding the targeting vector homologous region (SEQ ID NOs:14 and 15) in the FUT8 genome region (SEQ ID NO:9) obtained by the method described in Example 12 of WO02/31140. That is, 20 μl of a reaction mixture [DNA polymerase ExTaq (manufactured by Takara Shuzo Co., Ltd.), ExTaq buffer (manufactured by Takara Shuzo Co., Ltd.), 0.2 mmol/l dNTPs, 0.5 μmol/l each of the above gene-specific primers (SEQ ID NOs:14 and 15)] containing 4.0 ng of plasmid pFUT8fgE2-2 described in Example 12 of WO02/31140 was prepared, and PCR was carried out, after heating at 94°C for one minute, by 25 cycles, one cycle consisting of reaction at 94°C for 30 seconds, reaction at 55°C for 30 seconds and reaction at 74°C for one minute. After the PCR, the reaction mixture was subjected to 1.75% (w/v) agarose gel electrophoresis, and approximately 230 bp probe DNA fragment was purified. Then, 5 μl of the obtained probe DNA solution was subjected to radiolabeling using [$\alpha$-$^{32}$P] dCTP 1.75 MBq and Megaprime DNA Labelling system, dCTP (manufactured by Amersham Pharmacia Biotech).

Hybridization was carried out in the following manner. The above nylon membrane was put into a roller bottle and 15 ml of a hybridization solution [5 × SSPE, 50 × Denhaldt's solution, 0.5% (w/v) SDS, 100 μg/ml salmon sperm DNA] was added thereto. Prehybridization was carried out at 65°C for 3 hours. Then, the $^{32}$P-labeled probe DNA was heat-denatured and put into the bottle, followed by heating at 65°C overnight.

After the hybridization, the nylon membrane was immersed in 50 ml of 2 × SSC-0.1% (w/v) SDS and heated at 65°C for 15 minutes. After a washing step was repeated twice, the nylon membrane was immersed in 50 ml of 0.2 × SSC-0.1% (w/v) SDS and heated at 65°C for 15 minutes. After washing, the nylon membrane was exposed to an X-ray film at -80°C for development.

The genomic DNAs of the parent cell line CHO/DG44 and the 50-10-104 clone, which is the positive clone obtained in the above (2) were analyzed according to the present method. In the cell line CHO/DG44, only approximately 25.5 Kb fragment derived from the wild-type FUT8 allele was detected. On the other hand, in the positive clone, i.e., clone 50-10-104, approximately 20.0 Kb fragment specific to the allele which underwent homologous recombination was detected in addition to approximately 25.5 Kb fragment derived from the wild-type FUT8 allele. The quantitative ratio of these two kinds of fragments was 1:1, whereby it was confirmed that the clone 50-10-104 was a hemi-knockout clone wherein one copy of the FUT8 allele was disrupted.

3. Preparation of CHO/DG44 cell in which the FUT8 gene on the genome has been double-knocked out

(1) Obtaining of clone in which targeting vector pKOFUT8Puro has been introduced

To the FUT8 hemi-knockout clone 50-10-104 obtained in Example 1-2(2), the Chinese hamster FUT8 gene

exon 2 targeting vector plasmid pKOFUT8Puro constructed by the method described in Example 13-1 of WO02/31140 was introduced in the following manner.

After 440 μg of plasmid pKOFUT8Puro was allowed to react at 37°C for 5 hours by adding 800 units of a restriction enzyme *Sal*I (manufactured by New England Biolabs) for linearization, 4 μg of the linearized pKOFUT8Puro was introduced into 1.6 × $10^6$ cells of the FUT8 gene-hemi-knockout clone by electroporation [*Cytotechnology*, 3, 133 (1990)]. The resulting cells were suspended in IMDM-dFBS(10)-HT(1) and then inoculated into a 10-cm dish for adherent cell culture (manufactured by Falcon). After culturing in a 5% $CO_2$ incubator at 37°C for 24 hours, the medium was replaced with 10 ml of IMDM-dFBS(10)-HT(1) containing 15 μg/ml puromycin (manufactured by SIGMA).

Culturing was carried out for 15 days while the above medium replacement was repeated every 7 days to obtain drug-resistant clones.

(2) Confirmation of homologous recombination by genomic Southern blotting

Confirmation of the homologous recombination in the drug-resistant clones obtained in the above (1) was carried out by genomic Southern blotting in the following manner.

A culture supernatant was removed from a 10-cm dish in which the puromycin-resistant clones were expressed, 7 ml of a phosphate buffer was poured, and the dish was moved under a stereoscopic microscope. Next, colonies were ripped off and sucked by using PIPETMAN (manufactured by GILSON) and were collected in a round-bottomed 96-well plate (manufactured by Falcon). After trypsinizaton, each clone was inoculated into a flat-bottomed 96-well plate for adherent cells (manufactured by Asahi Techno Glass), followed by culturing using IMDM-dFBS(10)-HT(1) containing 15 μg/ml puromycin (manufactured by SIGMA) for one week.

After the culturing, each clone on the above plate was subjected to trypsinization and the resulting cells were inoculated into a flat-bottomed 24-well plate for adherent cells (manufactured by Greiner). After culturing using IMDM-dFBS(10)-HT(1) containing 15 μg/ml puromycin (manufactured by SIGMA) for one week, the cells were inoculated into a flat-bottomed 6-well plate for adherent cells (manufactured by Greiner). The genomic DNA of each clone was prepared from the plate according to a known method [*Nucleic Acids Research,* 3, 2303 (1976)] and then dissolved overnight in 150 μl of TE-RNase buffer (pH 8.0).

The genomic DNA prepared above (12 μg) was digested with 25 units of a restriction enzyme *Bam*HI (manufactured by New England Biolabs) at 37°C overnight for digestion reaction, and a DNA fragment recovered by ethanol precipitation was dissolved in 20 μl of TE buffer (pH 8.0) and then subjected to 0.6% (w/v) agarose gel electrophoresis. After the electrophoresis, the genomic DNA was transferred to a nylon membrane according to a known method [*Proc. Natl. Acad. Sci. USA,* 76, 3683 (1979)], followed by heat treatment of the nylon membrane at 80°C for 2 hours.

Separately, a probe used in the Southern blotting was prepared in the following manner. First, the following PCR was carried out by using primers which bind to the sequence of a part exceeding the targeting vector homologous region in the FUT8 genomic region (SEQ ID NOs:16 and 17). That is, 20 μl of a reaction mixture [DNA polymerase ExTaq (manufactured by Takara Shuzo Co., Ltd.), ExTaq buffer (manufactured by Takara Shuzo Co., Ltd.), 0.2 mmol/l dNTPs, 0.5 μmol/l each of the above gene-specific primers (SEQ ID NOs:16 and 17)] containing 4.0 ng of the plasmid pFUT8fgE2-2 constructed by the method described in Example 12 of WO02/31140 was prepared, and PCR was carried out, after heating at 94°C for one minute, by 25 cycles, one cycle consisting of reaction at 94°C for 30 seconds, reaction at 55°C for 30 seconds and reaction at 74°C for one minute. After the PCR, the reaction mixture was subjected to 1.75% (w/v) agarose gel electrophoresis, and approximately 230 bp probe DNA fragment was purified. Then, 5 μl of the obtained probe DNA solution was subjected to radiolabeling using [$\alpha$-$^{32}$P] dCTP 1.75 MBq and Megaprime DNA Labelling system, dCTP (manufactured by Amersham Pharmacia Biotech).

Hybridization was carried out in the following manner. The above nylon membrane was put into a roller bottle and 15 ml of a hybridization solution [5 × SSPE, 50 × Denhaldt's solution, 0.5% (w/v) SDS, 100 μg/ml salmon sperm DNA] was added thereto. Prehybridization was carried out at 65°C for 3 hours. Then, the $^{32}$P-labeled probe DNA was heat-denatured and put into the bottle, followed by heating at 65°C overnight.

After the hybridization, the nylon membrane was immersed in 50 ml of 2 × SSC-0.1% (w/v) SDS and heated at 65°C for 15 minutes. After this washing step was repeated twice, the nylon membrane was immersed in 50 ml of 0.2 × SSC-0.1% (w/v) SDS and heated at 65°C for 15 minutes. After washing, the nylon membrane was exposed to an X-ray film at -80°C for development.

The genomic DNA of the clone WK704, which is one of the puromycin-resistant clones obtained from the clone 50-10-104 according to the method described in the above (1), was analyzed according to the present method. In the clone WK704, approximately 25.5 Kb fragment derived from the wild-type FUT8 allele was eliminated and only approximately 20.0 Kb fragment specific to the allele which underwent homologous recombination was detected. From this result, it was confirmed that the clone WK704 was a clone wherein both FUT8 alleles were disrupted.

4. Removal of the drug resistance genes from FUT8 gene-double-knockout cells

(1) Introduction of Cre recombinase expression vector

Into WK704 of FUT8-double-knockout clone obtained in the item 3 of Example 1, the Cre recombinase expression vector pBs185 (manufactured by Life Technologies) was introduced in the following manner.

After 4 μg of plasmid pBs185 was introduced into $1.6 \times 10^6$ cells by electroporation [*Cytotechnology,* 3, 133 (1990)], the resulting cells were suspended in 10 ml of IMDM-dFBS(10)-HT(1) and the suspension was diluted 20000-fold with the same medium. The diluted suspension was inoculated into seven 10-cm dishes for adherent cell culture (manufactured by Falcon), followed by culturing in 5% $CO_2$ at 37°C for 10 days to form colonies.

(2) Obtaining of clone in which the Cre recombinase expression vector has been introduced

Arbitrary clones were collected from colonies obtained by gene introduction into WK704 in the following manner. First, a culture supernatant was removed from a 10-cm dish, 7 ml of a phosphate buffer was poured, and the dish was moved under a stereoscopic microscope. Next, colonies were ripped off and sucked using PIPETMAN (manufactured by GILSON) and were collected in a round-bottomed 96-well plate (manufactured by Falcon). After trypsinization, each clone was inoculated into a flat-bottomed 96-well plate for adherent cells (manufactured by Iwaki Glass), followed by culturing using IMDM-dFBS(10)-HT(1) for one week.

After the culturing, each clone on the above plate was subjected to trypsinization, and a 2-fold volume of a frozen medium (20% DMSO, 40% fetal calf serum and 40% IMDM) was mixed therewith. One half thereof was inoculated into a flat-bottomed 96-well plate for adherent cells (manufactured by Iwaki Glass) to prepare a replica plate, while the other half was stored by cryopreservation as a master plate.

The replica plate was cultured using IMDM-dFBS(10)-HT(1) containing 600 μg/ml G418 and 15 μg/ml puromycin for 7 days. Positive clones in which the drug resistance genes on both alleles between loxP sequences has been removed by the expression of Cre recombinase die in the presence of G418 and puromycin. The positive clones were selected according to this negative selection method.

(3) Confirmation of removal of the drug resistance genes by genomic Southern blotting

Confirmation of the removal of the drug resistance genes in the positive clones collected in the above (2) was carried out by genomic Southern blotting in the following manner.

Among the master plates stored by cryopreservation in the item (2), a 96-well plate containing the above positive clones was selected. After the plate was allowed to stand at 5% $CO_2$ and 37°C for 10 minutes, the cells in the wells corresponding to the above clones were inoculated into a flat-bottomed 24-well plate for adherent cells (manufactured by Greiner). After culturing using IMDM medium (manufactured by Invitrogen) to which 10% fetal bovine serum (manufactured by Invitrogen) and 1× concentration HT supplement (manufactured by Invitrogen) had been added for one week, the cells were inoculated into a flat-bottomed 6-well plate for adherent cells (manufactured by Greiner). The genomic DNA of each clone was prepared from the plate according to a known method [*Nucleic Acids Research,* 3, 2303 (1976)] and then dissolved overnight in 150 μl of TE-RNase buffer (pH 8.0).

The genomic DNA prepared above (12 μg) was digested with 20 units of a restriction enzyme *Nhe*I (manufactured by New England Biolabs) at 37°C overnight. A DNA fragment recovered from the reaction mixture by ethanol precipitation was dissolved in 20 μl of TE buffer (pH 8.0) and then subjected to 0.6% (w/v) agarose gel electrophoresis. After the electrophoresis, the genomic DNA was transferred to a nylon membrane according to a known method [*Proc. Natl. Acad Sci. USA,* 76, 3683 (1979)], followed by heat treatment of the nylon membrane at 80°C for 2 hours for immobilization.

Separately, a probe used in the Southern blotting was prepared in the following manner. First, the following PCR was carried out by using primers which bind to the sequence of a part exceeding the targeting vector homologous region in the FUT8 genomic region (SEQ ID NOs:16 and 17). That is, a reaction mixture [20 μl; DNA polymerase ExTaq (manufactured by Takara Shuzo Co., Ltd.), ExTaq buffer (manufactured by Takara Shuzo Co., Ltd.), 0.2 mmol/l dNTPs, 0.5 μmol/l each of the above gene-specific primers (SEQ ID NOs:16 and 17)] containing 4.0 ng of the plasmid pFUT8fgE2-2 obtaiend in item 1(2) of Example 1 was prepared, and PCR was carried out, after heating at 94°C for one minute, by 25 cycles, one cycle consisting of reaction at 94°C for 30 seconds, reaction at 55°C for 30 seconds and reaction at 74°C for one minute. After the PCR, the reaction mixture was subjected to 1.75% (w/v) agarose gel electrophoresis, and approximately 230 bp probe DNA fragment was purified. Then, 5 μl of the obtained probe DNA solution was subjected to radiolabeling using [$\alpha$-$^{32}$P] dCTP 1.75 MBq and Megaprime DNA Labelling system, dCTP (manufactured by Amersham Pharmacia Biotech).

Hybridization was carried out in the following manner. The above nylon membrane was put into a roller bottle

and 15 ml of a hybridization solution [5 × SSPE, 50 × Denhaldt's solution, 0.5% (w/v) SDS, 100 μg/ml salmon sperm DNA] was added thereto. Prehybridization was carried out at 65°C for 3 hours. Then, the $^{32}$P-labeled probe DNA was heat-denatured and put into the bottle, followed by heating at 65°C overnight.

After the hybridization, the nylon membrane was immersed in 50 ml of 2 × SSC-0.1% (w/v) SDS and heated at 65°C for 15 minutes. After this washing step was repeated twice, the nylon membrane was immersed in 50 ml of 0.2 × SSC-0.1% (w/v) SDS and heated at 65°C for 15 minutes. After washing, the nylon membrane was exposed to an X-ray film at -80°C for development.

By the above-described treatment with the restriction enzyme *Nhe*I, approximately 8.0 Kb DNA fragment was derived from the wild-type FUT8 allele. Also, by the similar treatment with the restriction enzyme, approximately 9.5 Kb DNA fragment was obtained from the allele which underwent homologous recombination with the targeting vector. Furthermore, by the similar treatment; approximately 8.0 Kb DNA fragment was derived when the neomycin resistance gene (approximately 1.6 Kb) and the puromycin resistance gene (approximately 1.5 Kb) were removed from the allele which underwent homologous recombination.

The genomic DNAs of the parent cell line CHO/DG44, the clone 50-10-104 described in the above item 2, the clone WK704 described in the above item 3, and the clone 4-5-C3, which is one of the drug-sensitive clones obtained from the clone WK704 by the method described in the above (2), were analyzed according to the present method. In the cell line CHO/DG44, only approximately 8.0 Kb DNA fragment derived from the wild-type FUT8 allele was detected. In the clone 50-10-104 and the clone WK704, approximately 9.5 Kb DNA fragment derived from the allele which underwent homologous recombination was observed. On the other hand, in the 4-5-C3 clone, only approximately 8.0 Kb DNA fragment resulting from the removal of the neomycin resistance gene (approximately 1.6 Kb) and the puromycin resistance gene (approximately 1.5 Kb) from the allele which underwent homologous recombination was detected. From the above results, it was confirmed that the drug resistance genes had been removed by Cre recombinase in the clone 4-5-C3.

Besides the clone 4-5-C3, plural FUT8 gene-double-knockout clones in which the drug-resistance gene had been removed (hereinafter referred to as FUT8 gene-double-knockout cells) were obtained.

Example 2

Expression of antibody molecule in CHO/DG44 cell in which FUT8 allele was double knocked out:

1. Preparation of anti-CD20 chimeric antibody expression vector

(1) Construction of cDNA encoding VL of anti-CD20 mouse monoclonal antibody

The cDNA (SEQ ID NO:18) encoding the amino acid sequence of VL in anti-CD20 mouse monoclonal antibody 2B8 described in WO94/11026 was constructed using PCR as follows.

First, the binding nucleotide sequence (including a restriction enzyme recognizing sequence for cloning to a vector for expression of humanized antibody) of the amplification primer for PCR was added to the 5'-terminal and 3'-terminal of the nucleotide sequence of VL described in WO94/11026. The designed nucleotide sequence was divided into total of 6 nucleotide sequences from the 5'-terminal side with about 100 bases each (the adjacent nucleotide sequences were adjusted in such a manner that their ends have a common sequence of about 20 bases at their termini) and 6 synthetic DNA of SEQ ID NOs:20, 21, 22, 23, 24 and 25 were prepared (consignment to GENSET company) in alternate order of a sense chain and an antisense chain.

Each oligonucleotide was added to 50 μL of a reaction solution [KOD DNA Polymerase affixture PCR Buffer #1 (manufactured by Toyobo), 0.2 mM dNTPs, 1 mM magnesium chloride, 0.5 μM M13 primer M4 (manufactured by Takara Shuzo Co., Ltd.), 0.5μM M13 primer RV (manufactured by Takara Shuzo Co., Ltd.)] to give a final concentration of 0.1 μM. Using a DNA thermal cycler GeneAmp PCR System 9600 (manufactured by Perkin Elmer), the reaction solution was heated at 94°C for 3 minutes, and the reaction was carried out by 25 cycles, one cycle consisting of reaction at 94°C for 30 seconds, reaction at 55°C for 30 seconds and reaction at 74°C for 1 minute after 2.5 units of KOD DNA Polymerase (manufactured by Toyobo) were added, and further heated at 72°C for 10 minutes. Then, 25 μL of the reaction solution was subjected to agarose gel electrophoresis and a PCR product of about 0.44 kb of VL was collected by using a QIAquick Gel Extraction Kit (manufactured by QIAGEN).

Then, 0.1 μg of DNA obtained from plasmid pBluescriptII SK(-) (manufactured by Stratagene) by a restriction enzyme *Sma*I (manufactured by Takara Shuzo Co., Ltd.) and about 0.1 μg of the PCR product obtained above were added to sterilized water to give a total volume of 7.5 μL, and 7.5 μL of solution I of a TAKARA ligation kit ver. 2 (manufactured by Takara Shuzo Co., Ltd.) and 0.3 μL of a restriction enzyme *Sma*I (manufactured by Takara Shuzo Co., Ltd.) were added thereto, followed by reaction at 22°C for 2 hours. Using the thus obtained recombinant plasmid DNA solution, *E. coli* DH5α strain (manufactured by Toyobo) was transformed. From the clones of the transformant, each plasmid DNA was prepared and was allowed to react by using a BigDye Terminator Cycle Sequencing Ready

Reaction Kit v2.0 (manufactured by Applied Biosystems) according to the attached manufacture's instruction, and then the nucleotide sequence was analyzed by a DNA sequencer ABI PRISM 377 of the same company. Thus, a plasmid pBs-2B8L having the nucleotide sequence of interest shown in Fig. 2 was obtained.

(2) Construction of cDNA encoding VH of anti-CD20 mouse monoclonal antibody

cDNA (SEQ ID NO:19) encoding the amino acid sequence of VH of anti-CD20 mouse monoclonal antibody 2B8 described in WO94/11026 was constructed using PCR as follows.

First, the binding nucleotide sequence (including a restriction enzyme recognition sequence for cloning to a vector for expression of humanized antibody) of the amplification primer for PCR was added to the 5'-terminal and 3'-terminal of the nucleotide sequence of VH described in WO94/11026. The designed nucleotide sequence was divided into total of 6 nucleotide sequences from the 5'-terminal side with about 100 bases each (the adjacent nucleotide sequences were adjusted in such a manner that their ends have a common sequence of about 20 bases at their termini) and 6 synthetic DNA of SEQ ID NOs: 26, 27, 28, 29, 30 and 31 were prepared (consignment to GENSET company) in alternate order of a sense chain and an antisense chain.

Each oligonucleotide was added to 50 μL of the reaction solution [KOD DNA Polymerase affixture PCR Buffer #1 (manufactured by Toyobo), 0.2 mM dNTPs, 1 mM magnesium chloride, 0.5 μM M13 primer M4 (manufactured by Takara Shuzo Co., Ltd.), 0.5 μM M13 primer RV (manufactured by Takara Shuzo Co., Ltd.)] to give a final concentration of 0.1 μM, and, using a DNA thermal cycler GeneAmp PCR System 9600 (manufactured by Perkin Elmer), the reaction solution was heated at 94°C for 3 minutes, and the reaction was carried out by 25 cycles, one cycle consisting of reaction at 94°C for 30 seconds, reaction at 55°C for 30 seconds and reaction at 74°C for 1 minute after 2.5 units of KOD DNA Polymerase (manufactured by Toyobo) was added thereto, and further heated at 72°C for 10 minutes. After 25 μL of the reaction solution was subjected to agarose gel electrophoresis, and a PCR product of about 0.49 kb of VH was collected by using a QIAquick Gel Extraction Kit (manufactured by QIAGEN).

Then, 0.1 μg of the DNA obtained from the plasmid pBluescriptII SK(-) (manufactured by Stratagene) by a restriction enzyme *Sma*I (manufactured by Takara Shuzo Co., Ltd.) and about 0.1 μg of the PCR product obtained as described above were added to sterilized water to give a total volume of 7.5 μL, and 7.5 μL solution I of a TAKARA ligation kit ver. 2 (manufactured by Takara Shuzo Co) and 0.3 μL of a restriction enzyme *Sma*I (manufactured by Takara Shuzo Co., Ltd.) were added thereto, followed by reaction overnight at 22°C.

Using the thus obtained recombinant plasmid DNA solution, *E. coli* DH5α strain (manufactured by Toyobo) was transformed. Each plasmid DNA from the clones of the transformant was prepared and was allowed to react by using a BigDye Terminator Cycle Sequencing Ready Reaction Kit v2.0 (manufactured by Applied Biosystems) according to the attached manufacture's instruction, and then the nucleotide sequence was analyzed by a DNA sequencer ABI PRISM 377 of the same company. Thus, a plasmid pBs-2B8H having the nucleotide sequence of interest shown in Fig. 3 was obtained.

Then, the synthetic DNA represented by SEQ ID NO:32 was designed in order to substitute the amino acid residue at position 14 from Ala to Pro, and the sulistitution was carried out as follows by PCR using a LA PCR *in vitro* Mutagenesis Primer Set for pBluescriptII (manufactured by Takara Shuzo Co., Ltd.). After 50 μL of a reaction solution [LA PCR Buffer II (manufactured by Takara Shuzo Co., Ltd.), 2.5 units of TAKARA LA Taq, 0.4 mM dNTPs, 2.5 mM magnesium chloride, 50 nM T3 BcaBEST Sequencing primer (manufactured by Takara Shuzo Co., Ltd.), 50 nM of the above-described primer for introducing mutation (SEQ ID NO:32, produced by GENSET)] containing 1 ng of the above-described plasmid pBs-2B8H was prepared and, using a DNA thermal cycler GeneAmp PCR System 9600 (manufactured by Perkin Elmer), the reaction solution was allowed to react by 25 cycles, one cycle consisting of reaction at 94°C for 30 seconds, reaction at 55°C for 2 minutes and reaction at 72°C for one and a half minute. Then, 30 μL of the reaction solution was subjected to agarose gel electrophoresis, and a PCR product of about 0.44 kb was collected using a QIAquick Gel Extraction Kit (manufactured by QIAGEN) and made to a 30 μL aqueous solution. Furthermore, PCR was carried out in the same manner using 50 μL of a reaction solution [LA PCR Buffer II (manufactured by Takara Shuzo Co., Ltd.), 2.5 units of TAKARA LA Taq, 0.4 mM dNTPs, 2.5 mM magnesium chloride, 50 nM T7 BcaBEST Sequencing primer (manufactured by Takara Shuzo Co., Ltd.), 50 nM MUT B 1 primer (manufactured by Takara Shuzo Co., Ltd.)] containing 1 ng of the above-described plasmid pBs-2B8H. Then, 30 μL of the reaction solution was subjected to agarose gel electrophoresis, and a PCR product of about 0.63 kb was collected using a QIAquick Gel Extraction Kit (manufactured by QIAGEN) and made to a 30 μL aqueous solution. Subsequently, 0.5 μL of the above obtained PCR product of about 0.44 kb and the PCR product of about 0.63 kb, respectively, were added to 47.5 μL of a reaction solution [LA PCR Buffer II (manufactured by Takara Shuzo Co., Ltd.), 0.4 mM dNTPs, 2.5 mM magnesium chloride] and, using a DNA thermal cycler GeneAmp PCR System 9600 (manufactured by Perkin Elmer), the reaction solution was allowed to react by heating at 90°C for 10 minutes, subsequent cooling to 37°C over 60 minutes, and then maintaining the temperature at 37°C for 15 minutes to thereby carry out annealing of DNA. After 2.5 units of TAKARA LA Taq (manufactured by Takara Shuzo Co., Ltd.) were added and allowed to react at 72°C for 3 minutes, 10 pmol of T3 BcaBEST Sequencing primer

(manufactured by Takara Shuzo Co., Ltd.) and T7 BcaBEST Sequencing primer (manufactured by Takara Shuzo Co., Ltd.), respectively, were added, and the reaction solution was made to 50 μL and allowed to react by 10 cycles, one cycle consisting of reaction at 94°C for 30 seconds, reaction at 55°C for 2 minutes and reaction at 72°C for one and a half minute as one cycle. Then, 25 μL of the reaction solution was purified with QIA quick PCR purification kit (manufactured by QIAGEN) and half the amount was allowed to react at 37°C for I hour using 10 units of a restriction enzyme *Kpn*I (manufactured by Takara Shuzo Co., Ltd.) and 10 units of a restriction enzyme *Sac*I (manufactured by Takara Shuzo Co., Ltd.). The reaction solution was fractionated by agarose gel electrophoresis and a *Kpn*I-*Sac*I fragment of about 0.59 kb was collected.

Next, 1 μg of pBluescriptII SK(-) (manufactured by Stratagene) was reacted at 37°C for 1 hour using 10 units of a restriction enzyme *Kpn*I (manufactured by Takara Shuzo Co., Ltd.) and 10 units of *Sac*I (manufactured by Takara Shuzo Co., Ltd.), and then, the reaction solution was fractionated by agarose gel electrophoresis to collect a *Kpn*I-*Sac*I fragment of about 2.9 kb.

The *Kpn*I-*Sac*I fragment derived from the PCR product obtained as described above and the *Kpn*I-*Sac*I fragment derived from the plasmid pBluescriptII SK(-) were ligated by using solution I of DNA Ligation Kit Ver.2 (manufactured by Takara Shuzo Co., Ltd.) according to the attached manufacture's instruction. Using the thus obtained recombinant plasmid DNA solution, *E. coli* DH5α strain (manufactured by Toyobo) were transformed. Each plasmid DNA was prepared from the clones of the transformant and allowed to react by using a BigDye Terminator Cycle Sequencing Ready Reaction Kit v2.0 (manufactured by Applied Biosystems) according to the attached manufacture's instruction, and then, the nucleotide sequence was analyzed with a DNA sequencer ABI PRISM 377 of the same company.

Thus, a plasmid pBs-2B8Hm having the nucleotide sequence of interest shown in Fig. 3 was obtained.

(3) Construction of anti-CD20 human chimeric antibody expression vector

Using vector pKANTEX93 for humanized antibody expression [*Mol. Immunol*., 37, 1035 (2000)] and plasmid pBs-2B8L and pBs-2B8Hm obtained in the items (1) and (2), the expression vector pKANTEX2B8P of the anti-CD20 human chimeric antibody (hereinafter referred to as "anti-CD20 chimeric antibody") was constructed as follows.

After 2 μg of the plasmid pBs-2B8L obtained in the item (1) was allowed to react at 55°C for 1 hour using 10 units of a restriction enzyme *Bsi*WI (manufactured by New England Biolabs), further reaction was carried out at 37°C for 1 hour using 10 units of a restriction enzyme *EcoRI* (manufactured by Takara Shuzo Co., Ltd.). The reaction solution was fractionated by agarose gel electrophoresis to collect a *Bsi*WI-*EcoRI* fragment of about 0.41 kb.

Then, 2 μg of the vector pKANTEX93 for humanized antibody expression was allowed to react at 55°C for 1 hour using 10 units of a restriction enzyme *Bsi*WI (manufactured by New England Biolabs), and then further reaction was carried out at 37°C for 1 hour using 10 units of a restriction enzyme *EcoRI* (manufactured by Takara Shuzo Co., Ltd.). The reaction solution was fractionated by agarose gel electrophoresis to collect a *Bsi*WI-*Eco*RI fragment of about 12.75 kb.

Next, the above obtained *Bsi*WI-*Eco*RI fragment derived from plasmid pBs-2B8L and the *Bsi*WI-*Eco*RI fragment derived from the plasmid pKANTEX93 were ligated by using solution I of DNA Ligation Kit Ver.2 (manufactured by Takara Shuzo Co., Ltd.) according to the attached manufacture's instruction. Using the thus obtained recombinant plasmid DNA solution, *E. coli* DH5α strain (manufactured by Toyobo) was transformed to obtain a plasmid pKANTEX2B8-L shown in Fig. 4.

Then, 2 μg of the plasmid pBs-2B8Hm obtained in the item (2) was allowed to react at 37°C for 1 hour by using 10 units of a restriction enzyme *Apa*I (manufactured by Takara Shuzo Co., Ltd.), and then, further reaction was carried out at 37°C for 1 hour by using 10 units of a restriction enzyme *Not*I (manufactured by Takara Shuzo Co., Ltd.). The reaction solution was fractionated by agarose gel electrophoresis to collect an *Apa*I-*Not*I fragment of about 0.45 kb.

Next, 3 μg of the plasmid pKANTEX2B8-L obtained above was allowed to react at 37°C for 1 hour by using 10 units of a restriction enzyme *Apa*I (manufactured by Takara Shuzo Co., Ltd.), and then further reaction was carried out at 37°C for 1 hour by using 10 units of a restriction enzyme *Not*I (manufactured by Takara Shuzo Co., Ltd.). The reaction solution was fractionated by agarose gel electrophoresis to collect an *Apa*I-*Not*I fragment of about 13.16 kb.

Then, the above obtained *Apa*I-*Not*I fragment derived from plasmid pBs-2B8Hm and the *Apa*I-*Not*I fragment derived from plasmid pKANTEX2B8-L were ligated by using solution I of DNA Ligation Kit Ver.2 (manufactured by Takara Shuzo Co., Ltd.) according to the attached manufacture's instruction. Using the recombinant plasmid DNA solution obtained in this manner, *E. coli* DH5α strain (manufactured by Toyobo) was transformed and each plasmid DNA was prepared from the clone of the transformant.

Using the obtained plasmid, the nucleotide sequence was analyzed by using a BigDye Terminator Cycle Sequencing Ready Reaction Kit v2.0 (manufactured by Applied Biosystems) with DNA sequencer 377 of the same company. As a result, it was confirmed that a plasmid pKANTEX2B8P cloned with the DNA of interest shown in Fig. 4 was obtained.

2. Expression of anti-CD20 chimeric antibody

The expression vector pKANTEX2B8P of the anti-CD20 antibody obtained in the item 1 of this Example was introduced into the FUT8 gene double knockout clone WK704 prepared in the item 5(2) of Example 1.

The gene introduction into WK704 of the plasmid pKANTEX2B8P was carried out by electroporation *[Cytotechnology,* 3, 133 (1990)] by the procedure as follows. First, 10 μg of the plasmid pKANTEX2B8P was dissolved in 100 μl of NEBuffer 4 (manufactured by New England Biolabs), and 40 units of a restriction enzyme *Aat*II (manufactured by New England Biolabs) were added thereto, then the linearization was carried out by digestion at 37°C for 2 hours. The reaction solution was extracted with phenol/chloroform extraction, followed by ethanol precipitation, and the recovered linear plasmid was made into a 1 μg/μl aqueous solution. Separately, WK704 was suspended into K-PBS buffer (137 mmol/l KCl, 2.7 mmol/l NaCl, 8.1 mmol/l $Na_2HPO_4$, 1.5 mmol/l $KH_2PO_4$, 4.0 mmol/l $MgCl_2$) to give a density of $8\times10^7$ cells/ml. After 200 μl of the cell suspension ($1.6\times10^6$ cells) were combined with 4 μl (4 μg) of the above-described linearized plasmid, the total cell-DNA mixture was transferred to Gene Pulser Cuvette (electrode distance: 2 mm) (manufactured by BIO-RAD) and the gene introduction was carried out by using Gene Pulser cell fusion device (manufactured by BIO-RAD) at a pulse voltage of 350 V and a capacity of 250 μF. After the gene introduction, the cell suspension was suspended in IMDM medium (manufactured by Invitrogen) supplemented with 10% fetal bovine serum (manufactured by Invitrogen) and 1 × concentration HT supplement (manufactured by Invitrogen), and inoculated onto T75 flasks for adhesion cell culture (manufactured by Greiner). After culturing at 5 % $CO_2$ and 37°C for 24 hours, the culture supernatant was removed and 10 ml IMDM medium (manufactured by Invitrogen) supplemented with 10% fetal bovine dialysis serum (manufactured by Invitrogen) were filled therein. The culturing was carried out for 15 days while the medium exchange process was repeated every 3 to 4 days, and a transformant WK704-2B8P was obtained. Furthermore, the clone WK704-2B8P, as a name of WK704-2B8P, has been deposited on March 20, 2003, as FERM BP-8337 in International Patent Organism Depositary, National Institute of Advanced Industrial Science and Technology (Tsukuba Central 6, 1-1, Higashi 1-Chome Tsukuba-shi, Ibaraki-ken, Japan).

3. Expression of anti-ganglioside GD3 chimeric antibody

The vector plasmid pKANTEX641 for expression of the anti-ganglioside GD3 chimeric antibody was introduced into the FUT8 gene double knockout clone WK704 prepared in the item 5(2) of Example 1 and a stable expression clone of anti-GD3 chimeric antibody was prepared. The pKANTEX641 is a derivative comprising the vector plasmid pChi641LHGM4 for expression of the anti-GD3 chimeric antibody described in W000/61739 and the vector pKANTEX93 for humanized antibody expression [*Mol. Immunol.,* 37, 1035 (2000)] in which an *Eco*RI-*Hin*dIII fragment containing a tandem type antibody expression unit obtained from pChi641LHGM4 is ligated with an *Eco*RI-*Hin*dIII fragment containing the origin of replication obtained from pKANTEX93.

The gene introduction into WK704 of the plasmid pKANTEX641 was carried out by electroporation *[Cytotechnology,* 3, 133 (1990)] by the procedure as follows. First, 10 μg of the plasmid pKANTEX641 was dissolved in 100 μl of NEBuffer 4 (manufactured by New England Biolabs), and 40 units of a restriction enzyme *Aat*II (manufactured by New England Biolabs) were added thereto, and then the linearization was carried out by digestion at 37°C for 2 hours. The reaction solution was extracted with phenol/chloroform extraction, followed by ethanol precipitation, and the recovered linear plasmid was made into a 1 μg/μl aqueous solution. Separately, WK704 was suspended into K-PBS buffer (137 mmol/l KCl, 2.7 mmol/l NaCl, 8.1 mmol/l $Na_2HPO_4$, 1.5 mmol/l $KH_2PO_4$, 4.0 mmol/l $MgCl_2$) to give a density of $8\times10^7$ cells/ml. After 200 μl of the cell suspension ($1.6\times10^6$ cells) was mixed with 4 μl (4 μg) of the above-described linearized plasmid, the whole cell-DNA mixture was transferred to Gene Pulser Cuvette (electrode distance: 2 mm) (manufactured by BIO-RAD) and the gene introduction was carried out by using Gene Pulser cell fusion device (manufactured by BIO-RAD) at a pulse voltage of 350 V and a capacity of 250 μF. After the gene introduction, the cell suspension was suspended in IMDM medium (manufactured by Invitrogen) supplemented with 10 % fetal bovine serum (manufactured by Invitrogen) and 1 × concentration HT supplement (manufactured by Invitrogen), and inoculated onto T75 flasks for adhesion cell culture (manufactured by Greiner). After culturing at 5 % $CO_2$ and 37°C for 24 hours, the culture supernatant was removed and 10 ml of IMDM medium (manufactured by Invitrogen) supplemented with 10% fetal bovine dialysis serum (manufactured by Invitrogen) were filled therein. The culturing was carried out for 15 days while the medium exchange process was repeated every 3 to 4 days, and a transformant WK704-2871 was obtained. Furthermore, the clone WK704-2871, as a name of WK704-2871, has been deposited on March 20, 2003, as FERM BP-8336 in International Patent Organism Depositary, National Institute of Advanced Industrial Science and Technology (Tsukuba Central 6, 1-1, Higashi 1-Chome Tsukuba-shi, Ibaraki-ken 305-8566 Japan).

4. Expression of the anti-CCR4 chimeric antibody

The vector pKANTEX2160 for expression of anti-CCR4 chimeric antibody described in W001/64754 was introduced into the FUT8 gene double knockout clone WK704 prepared in the item 5(2) of Example 1 and a stable expression clone of the anti CCR4 chimeric antibody was prepared.

The gene introduction into the WK704 of the plasmid pKANTEX2B8P was carried out in a similar manner to the electroporation technique *[Cytotechnology,* 3, 133 (1990)] according to the procedure as follows. First, 15 μg of the plasmid pKANTEX2160 were dissolved in 100 μl of NEBuffer 4 (manufactured by New England Biolabs), 40 units of a restriction enzyme *Aat*II (manufactured by New England Biolabs) were added thereto, and then the linearization was carried out by digestion at 37°C for 2 hours. The reaction solution was extracted with phenol/chloroform extraction, followed by ethanol precipitation, and the recovered linear plasmid was made into a 1 μg/μl aqueous solution. Separately, WK704 was suspended in K-PBS buffer (137 mmol/l KCl, 2.7 mmol/l NaCl, 8.1 mmol/l $Na_2HPO_4$, 1.5 mmol/l $KH_2PO_4$, 4.0 mmol/l $MgCl_2$) to give a density of $8\times10^7$ cells/ml. After 200 μl of the cell suspension ($1.6\times10^6$ cells) was combined with 4 μl (4 μg) of the above-described linearized plasmid, the whole cell-DNA mixture was transferred to Gene Pulser Cuvette (electrode distance: 2 mm) (manufactured by BIO-RAD) and the gene introduction was carried out by using Gene Pulser cell fusion device (manufactured by BIO-RAD) at a pulse voltage of 350 V and a capacity of 250 μF. After the gene introduction, the cell suspension was suspended in IMDM medium (manufactured by Invitrogen) supplemented with 10% fetal bovine serum (manufactured by Invitrogen) and 1 × concentration HT supplement (manufactured by Invitrogen), and inoculated onto T75 flasks for adhesion cell culture (manufactured by Greiner). After culturing at 5 % $CO_2$ and 37°C for 24 hours, the culture supernatant was removed and 10 ml IMDM medium (manufactured by Invitrogen) supplemented with 10 % fetal bovine dialysis serum (manufactured by Invitrogen) were filled therein. The culturing was carried out for 15 days while this medium exchange process was repeated every 3 to 4 days, and a transformant WK704-2760 was obtained. Furthermore, the clone WK704-2760, as a name of WK704-2760, has been deposited on March 20, 2003, as FERM BP-8335 in International Patent Organism Depositary, National Institute of Advanced Industrial Science and Technology (Tsukuba Central 6, 1-1, Higashi 1-Chome Tsukuba-shi, Ibaraki-ken, Japan).

5. Measurement of human IgG antibody concentration in culture supernatant (ELISA)

A goat anti-human IgG (H & L) antibody (manufactured by American Qualex) was diluted with phosphate buffered saline (hereinafter referred to as PBS) to give a concentration of 1 μg/ml, dispensed at 50 μl/well into a 96-well ELISA plate (manufactured by Greiner) and then allowed to stand at 4°C overnight for adsorption. After washing with PBS, PBS containing BSA at a concentration of 1% (hereinafter referred to as 1% BSA-PBS) (manufactured by Wako Pure Chemical Industries) was added at 100 μl/well and allowed to react at room temperature for 1 hour to thereby block the remaining active groups. The 1% BSA-PBS was discarded, and culture supernatant of a transformant or variously diluted solution of antibody purified from the culture supernatant was added at 50 μl/well and allowed to react at room temperature for 1 hour. After the reaction, each well was washed with PBS containing Tween 20 at a concentration of 0.05% (hereinafter referred to as Tween-PBS) (manufactured by Wako Pure Chemical Industries), and then a peroxidase-labeled goat anti-human IgG (H & L) antibody solution (manufactured by American Qualex), diluted 2000-fold with 1% BSA-PBS, was added as a secondary antibody solution at 50 μl/well and allowed to react at room temperature for 1 hour. After the reaction and subsequent washing with Tween-PBS, an ABTS substrate solution [a solution prepared by dissolving 0.55 g of 2,2'-azino-bis(3-ethylbenzothiazoline-6-sulfonic acid)ammonium (manufactured by Wako Pure Chemical Industries) in 1 liter of 0.1 M citrate buffer (pH 4.2), and adding hydrogen peroxide (manufactured by Wako Pure Chemical Industries) to give a concentration of 1 μl/ml just before use] was added at 50 μl/well to develop color, and the absorbance at 415 nm (hereinafter referred to as OD415) was measured.

6. Purification of antibody molecule

The clone WK704-2B8P for expression of anti-CD20 antibody obtained in the item 2 of this Example was suspended in IMDM medium (manufactured by Invitrogen) supplemented with 10% fetal bovine dialysis serum (manufactured by Invitrogen) to give a density of $3\times10^5$ cells/ml and a total volume of 300 ml was inoculated into 10 bottles of T182 flasks for adhesion cell culture (manufactured by Greiner). The clone WK704-2871 for expression of anti-GD3 antibody obtained in the item 3 of this Example and the clone WK704-2760 for expression of anti-CCR4 antibody obtained in the item 4 of this Example were inoculated in the same manner. After culturing for 3 days, all culture supernatants of each clone were removed and exchanged to EXCELL301 medium (manufactured by JRH Biosciences). They were cultured for 7 days at 37°C in a 5% $CO_2$ incubator, and then each cell suspension was collected. Each of all collected cell suspensions was centrifuged for 10 minutes at 3000 rpm and 4°C to recover a supernatant, and then the supernatant was filtered with a PES membrane having a pore size of 0.22 μm and a volume of 500 ml (manufactured by Asahi Technoglass).

In a column having a diameter of 0.8 cm, 0.5 ml of Mab Select (manufactured by Amersham Pharmacia Biotech) was packed and then 3.0 ml purified water and 3.0 ml of 0.2 mol/l boric acid - 0.15 mol/l NaCl buffer (pH 7.5) were filled into the tube successively. Furthermore, the carrier was equilibrated by successive cleaning with 2.0 ml of 0.1 mol/l

citrate buffer (pH 3.5) and 1.5 ml of 0.2 mol/l boric acid - 0.15 mol/l NaCl buffer (pH 7.5). Then, after 300 ml of the above-described culture supernatant was packed into the column, it was washed with 3.0 ml of 0.2 mol/l boric acid - 0.15 mol/l NaCl buffer (pH 7.5). After the washing, the antibody absorbed on the carrier was eluted by using 1.25 ml of 0.1 mol/l citrate buffer (pH 3.5). After 250 μl of the first eluted fraction was disposed, 1 ml of the next eluted fraction was recovered and naturalized by mixing with 200 μl of 2 mol/l Tris-HCl (pH 8.5). The obtained eluted solution was dialyzed overnight at 4°C by using 10 mol/l citric acid - 0.15 mol/l NaCl buffer (pH 6.0). After the dialysis, the antibody solution was recovered and was sterilized and filtered by using Millex GV having a pore size of 0.22 μm (manufactured by MILLIPORE).

Example 3

*In vitro* cytotoxic activity (ADCC activity) of antibody composition produced by CHO/DG44 cell in which FUT8 gene was double knocked out:

In order to evaluate the *in vitro* cytotoxic activity of the anti-CD20 antibody purified in Example 2-6, the ADCC activity was measured as follows.

(1) Preparation of target cell suspension

A human B lymphocyte cultured cell line Raji cell (JCRB9012) cultured in RPMI1640-FCS(10) medium [PRMI1640 medium (manufactured by GIBCO BRL) supplemented with 10% FCS] was washed with RPMI1640-FCS (5) medium [PRMI1640 medium (manufactured by GIBCO BRL) supplemented with 5 % FCS] by centrifuge separation and suspension. Then, the suspension was prepared with RPMI1640-FCS(5) medium to give a density of $2\times10^5$ cells/ml as the target cell suspension.

(2) Preparation of effector cell suspension

After 50 ml of venous blood of a healthy person was collected, 0.5 ml of heparin sodium (manufactured by Shimizu Seiyaku) was added thereto, followed by mixing gently. The mixture was centrifuged (800 g, 20 minutes) with Lymphoprep (manufactured by AXIS SHIELD) according to the manufacture's instruction to separate a mononuclear cell phase. The cells were washed 3 times with RPMI1640-FCS(5) medium by centrifugal separation and re-suspended to give a density of $4\times10^6$ cells/ml by using the same medium, and the resulting suspension was used as effector cell suspension.

(3) Measurement of the ADCC activity

To each well of a 96-well U-shape bottom plate (manufactured by Falcon), 50 μl ($1\times10^4$ cells/well) of the target cell suspension prepared in the above (1) was dispensed. Then, 50 μl ($2\times10^5$ cells/well, the ratio of the effector cells and target cells becomes 20 : 1) of the effector cell suspension prepared in the above (2) was added. Moreover, various anti CD20 chimeric antibodies were added to give a final concentration of 0.3 to 3000 ng/ml and a total volume of 150 μl, and the reaction was carried out at 37°C for 4 hours. After the reaction, the plates were centrifuged and the lactate dehydrogenase (LDH) activity in the supernatant was measured by CytoTox96 Non-Radioactive Cytotoxicity Assay (manufactured by Promega). The spontaneously released LDH amount of the target cells was calculated by carrying out the same procedure as described above, except for using only medium instead of the effector cell suspension and measuring the LDH activity in the supernatant. The absorbance data of the spontaneous release of the effector cells was obtained by carrying out the same procedure as described above, except for using the medium instead of the effector cell suspension and the antibody solution. The total free LDH amount involved in all targeted cytoclasis was calculated by the measurement of the LDH activity in the supernatant, conducting the same procedure as described above, except for using only medium instead of the effector cell suspension and the antibody solution, and adding 15 μl of 9% Triton X-100 solution 45 minutes before the end of the reaction. The ADCC activity was calculated according to the following formula (II) by using these values.

$$\text{ADCC activity (\%)} = \frac{\left(\begin{array}{c}\text{LDH amount in}\\ \text{sample supernatant}\end{array}\right) - \left(\begin{array}{c}\text{spontaneously released}\\ \text{LDH amount}\end{array}\right)}{\left(\begin{array}{c}\text{total released}\\ \text{LDH amount}\end{array}\right) - \left(\begin{array}{c}\text{spontaneously released}\\ \text{LDH amount}\end{array}\right)} \times 100 \quad \text{(II)}$$

The ADCC activity of each anti-CD20 antibody is shown in Fig. 5. The antibodies obtained from FUT8 gene double knockout clone WK704-2B8P showed a higher ADCC activity than commercially available Rituxan™ in all antibody concentrations and the maximum cytotoxic activity value was also higher. Rituxan™ is an anti-CD20 chimeric antibody produced by using CHO cell, as the host cell, in which the FUT8 gene was not disrupted. Furthermore, as a result of the measurement of the ADCC activity of each of the antibodies obtained from the FUT8 gene double knockout clone WK704-2871 and the clone WK704-2760, it was shown that a higher cytotoxic activity than an antibody produced by a usual CHO cell line in which FUT8 gene was not disrupted was obtained in the same manner as in the case of the anti-CD20 antibody. Based on the above results, it was found that an antibody having a higher cytotoxic activity can be prepared by using a host cell in which the FUT8 allele was disrupted, in comparison with the case of using host cells in which FUT8 gene was not disrupted.

## Example 4

Analysis of monosaccharide composition of antibody composition produced by CHO/DG44 cell in which FUT8 allele was double knocked out:

Analysis of the neutral sugar and amino sugar composition of the anti-CD20-antibody, the anti-GD3 antibody and the anti-CCR4 antibody produced by the FUT8 gene double knock out clones in the item 6 of Example 2 was carried out as follows.

After the antibody was dried under reduced pressure using a centrifugal concentrator, a 2.0 to 4.0 mol/l trifluoroacetic acid solution was added thereto and acid hydrolysis was carried out at 100°C for 2 to 4 hours to release neutral sugars and amino sugars from the protein. The trifluoroacetic acid solution was removed with a centrifugal concentrator, and the sugars were redissolved in deionized water and subjected to analysis using a carbohydrate analysis system (DX-500 manufactured by Dionex). The analysis was carried out according to the elution program shown in Table 1 using CarboPac PA-1 column and CarboPac PA-1 guard column (manufactured by Dionex), a 10 to 20 mM solution of sodium hydroxide in deionized water as an eluting solution and a 500 mM solution of sodium hydroxide in deionized water as a washing solution.

Table 1

| Elution program for neutral sugar and amino sugar composition analysis | | | | | | |
|---|---|---|---|---|---|---|
| Time (min.) | 0 | 35 | 35.1 | 45 | 45.1 | 58 |
| Eluting solution (%) | 100 | 100 | 0 | 0 | 100 | 100 |
| Washing solution (%) | 0 | 0 | 100 | 100 | 0 | 0 |

From the obtained peak areas of neutral and amino sugar components, the composition ratio of components (fucose, galactose and mannose) was calculated, regarding the value of N-acetylglucosamine as 4.

The ratios of complex type sugar chains to which fucose is not bound among the total complex type sugar chains were calculated based on the monosaccharide composition of each antibody. As a result, it was shown that fucose is not bound to the complex type sugar chains of the anti-CD20 antibody, the anti-GD3 antibody and the anti-CCR4 antibody produced by the FUT8 gene double knock out clones.

## Example 5

Naturalization of FUT8 gene double knock out CHO/DG44 cell in protein-free medium

Naturalization of the clone 4-5-C3 prepared in Example 1 as an FUT8 gene double knock out CHO/DG44 cell to a protein-free medium was carried out.

A serum-added medium comprising IMDM medium (manufactured by Invitrogen) further supplemented with 1% (v/v) HT Supplement (manufactured by Invitrogen) and 10% (v/v) fetal bovine serum (dFBS; manufactured by Invitrogen) (hereinafter referred to as "basal serum medium") was prepared. Using the medium, the clone 4-5-C3 was inoculated into a T flask at a cell density of 2 to $4\times10^5$ cells/ml, and static culturing was carried out at 37°C under a 5% $CO_2$ concentration by setting the sub-culture period to 2 to 4 days. At the time of sub-culture, total volume exchange from the culture liquid to fresh medium was carried out by centrifugation.

Using the cells obtained by the sub-culturing described in the above, static sub-culture of 6 passages and for 29 days was carried out using a protein-free medium comprising EX-CELL325PF (manufactured by JRH) further supplemented with 1% (v/v) HT Supplement (manufactured by Invitrogen) and 6 mM glutamine (manufactured by Invitrogen) (hereinafter referred to as "basal protein-free medium"). The culturing was carried out in the same manner as the case of serum culturing, using a T flask at 37°C and 5% $CO_2$ concentration. Subsequently, suspension rotary culturing was carried out for 6 passages and for 20 days using the basal protein-free medium in a conical flask. The culturing temperature was set to 35°C, and the rotation speed to 90 to 100 rpm, and at the time of sub-culture, the sub-culturing was carried out after substituting air in the conical flask through the blowing of 5% concentration of $CO_2$ to the upper face of the medium, in an amount of 4 volumes or more of the culture container.

By the sub-culturing using the basal protein-free medium described in the above, the cells which did not proliferate due to cell aggregation in the early culturing stage were finally able to be converted into naturalized cells which can be sub-cultured using the basal protein-free medium.

Next, the thus prepared clone 4-5-C3 naturalized in the protein-free medium was cloned by a limiting dilution method in the following manner.

The naturalized cells prepared using the basal protein-free medium were diluted and then inoculated in 0.05 ml per well portions at 0.5 cell/well into a 96-well plate. Subsequently, a culture supernatant (conditioned medium) of the naturalized clone, which was sterilized using a sterilization filter was added thereto in 0.05 ml per well portions. As a result of inoculating into a total of 768 wells and culturing for 1 to 2 weeks, 49 clones in which single colony growth was confirmed were obtained. The thus obtained 49 clones were cultured in an expanded manner into a 24 well plate and then into a 6 well plate, and then, taking the proliferative ability of the thus obtained individual clones into consideration, 17 clones having good proliferative ability were selected. The thus obtained 17 clones were mixed and used as a cell naturalized in a protein-free medium.

Changes of viable cell density and survival rate by the thus obtained cell naturalized in a protein-free medium during the sub-culture in the protein-free medium were shown. The results are shown in Fig. 6.

As shown in Fig. 6, an amount of cells naturalized in a protein-free medium obtained by the method described in the above had grown 3 times during 2 to 3 days after initiation of culturing in the medium at a low cell density. This shows that, different from the parent cell line, namely the cell line before naturalization in the protein-free medium, even when the cells are cultured in the basal protein-free medium, they can be converted in such a manner that sub-culturing can be stably carried out.

Example 6

Serum-free fed-batch culture of FUT8 gene double knock out CHO/DG44 cell naturalized in protein-free medium:

Fed-batch culture was carried out using the cell naturalized in a protein-free medium obtained in Example 5.

Using the basal protein-free medium, the cell naturalized in a protein-free medium was prepared into a cell density of $2\times10^5$ cells/ml. Then, 15 ml of the thus prepared cell naturalized in a protein-free mediums was added to a 125 ml capacity conical flask and cultured at a culturing temperature of 35°C and at a rotation speed of 100 rpm for 3 days. In this connection, at the time of the cell inoculation, air in the conical flask was substituted through the blowing of 500 ml or more of 5% in concentration of $CO_2$ to the upper face of the medium. The cells obtained by culturing for 3 days were used as the seed cells and adjusted to a cell density of $3\times10^5$ cells/ml using the basal protein-free medium, 30 ml of the suspension was inoculated into a 125 ml capacity conical flask, and then the fed-batch culture was started at a culturing temperature of 35°C and at a rotation speed of 100 rpm. At the time the cells were inoculated, air in the flask was substituted through the blowing of 1 liter or more of 5% in concentration of $CO_2$ to the upper face of the medium. After commencement of the fed-batch culturing, a feed medium having the composition shown below was added in 3.3 ml portions on the 3rd day and 6th day for the purpose of supplementing consumed amounts of amino acids and the like. In addition, 20% (w/v) glucose solution was added on the 3rd day of the culturing to make the final glucose concentration to 5000 mg/l. The results are shown in Fig. 7. The cells grew after commencement of the culturing until day 3, and the cell density was maintained for the most part until day 3 to day 6. Viable cell density on the 6th day of the culturing reached $2\times10^6$ cells/ml. The survival rate rapidly decreased when 6 days had passed from the commencement of culturing, and the cell survival rate became less that 50% on the 9th day, and the fed-bath culturing was completed.

In this connection, the feed medium used in the fed-batch culturing was a medium prepared by supplementing a general medium with amino acids (0.177 g/l L-alanine, 0.593 g/l L-arginine monohydrochloride, 0.177 g/l L-asparagine monohydrate, 0.212 g/l L-aspartic acid, 0.646 g/l L-cystine dihydrochloride, 0.530 g/l L-glutamic acid, 5.84 g/l L-glutamine, 0.212 g/l glycine, 0.297 g/l L-histidine monohydrochloride dihydrate 0.742 g/l, L-isoleucine, 0.742 g/l L-leucine, 1.031 g/l L-lysine monohydrochloride, 0.212 g/l L-methionine, 0.466 g/l L-phenylalanine, 0.283 g/l L-proline, 0.297 g/l L-serine, 0.671 g/l L-threonine, 0.113 g/l L-tryptophan, 0.735 g/l L-tyrosine disodium dihydrate, and 0.664 g/l L-valine), vitamins (0.0918 mg/l d-biotin, 0.0283 g/l calcium D-pantothenate, 0.0283 g/l choline chloride, 0.0283 g/l folic acid, 0.0509 g/l myo-inositol, 0.0283 g/l niacinamide, 0.0283 g/l pyridoxal hydrochloride, 0.00283 g/l riboflavin, 0.0283 g/l thiamin hydrochloride, and 0.0918 mg/l cyanocobalamin) and 0.314 g/l insulin.

Example 7

Production of anti-CD20 human chimeric antibody by FUT8 gene double knock out CHO/DG44 cell naturalized in protein-free medium, and biological activity of the same:

Using the FUT8 gene double knock out cell naturalized in the protein-free medium as described in Example 6, a stably producing cell of an anti-CD20 human chimeric antibody was established, and productivity of the anti-CD20 human chimeric antibody and biological activity of the produced antibody were evaluated. In this case, in order to show superiority of the high ADCC activity antibody production by the FUT8 gene double knock out cell naturalized in the protein-free medium, anti-CD20 human chimeric antibody producing clones were also established from CHO/DG44 cell which is the parent cell line of the FUT8 gene double knock out cell, the GDP-mannose-4,6-dehydratase mutant cell line of CHO cell, Lec13 cell, established by Stanley, *et al. (Somat. Cell Mol. Genet.,* 12, 51 (1986)) and the rat-rat hybridoma YB2/0 (American Type Culture Collection CRL-1662), and their comparison was carried out. In the case of the Lec13 and YB2/0 cells, they were used as comparative controls because it has been reported that it is possible to effect expression of a high ADCC activity antibody having high ratio of sugar chains in which fucose is not bound (*J. Biol. Chem.,* 277, 30, 26733 (2002); WO 02/31140).

1. Construction of clone stably producing anti-CD20 human chimeric antibody

In accordance with the method described in the item 2 of Example 2, an anti-CD20 human chimeric antibody expression vector pKANTEX2B8P was introduced into the FUT8 gene double knock out cell naturalized in the protein-free medium as described in Example 6, the CHO/DG44 cell and the Lec13 cell, and the cells were respectively inoculated into a 96-well culture plate (manufactured by Greiner) and cultured at 37°C for 1 to 2 weeks in a 5% $CO_2$ incubator. Regarding the transformants of wells where their growth was observed in the IMDM-dFBS(10) medium, they were further cultured for 1 to 2 weeks by changing the medium to IMDM-dFBS(10) medium containing 50 nmol/l of MTX (manufactured by Sigma), in order to increase the antibody production by using a dhfr gene amplification system. Regarding the transformants showing resistance to 50 nmol/l of MTX, their culturing was continued by further increasing the MTX concentration. By measuring expression of the anti-CD20 human chimeric antibody in the culture supernatants by the ELISA method described in the item 5 of Example 2, transformants capable of growing in the IMDM-dFBS(10) medium containing 200, 500 or 1000 nmol/l in concentration of MTX and of highly producing anti-CD20 human chimeric antibody were finally selected. Regarding the YB2/0 cell, transformants capable of highly producing anti-CD20 human chimeric antibody were selected in accordance with the method described in WO 03/055993.

Next, the transformants capable of highly producing anti-CD20 human chimeric antibody obtained in this manner were naturalized in a serum-free medium in accordance with the method described in Example 5. EX-CELL 302 medium (manufactured by JRH) containing 200, 500 or 1000 nmol/l of MTX and 6 mM L-glutamine (manufactured by Invitrogen) (hereinafter referred to as "serum-free medium") was used in the serum-free medium naturalization of respective transformants established from the FUT8 gene double knock out cell naturalized in the protein-free medium, the CHO/DG44 cell and the Lec13 cell. CD-Hybridoma medium (manufactured by Invitrogen) (hereinafter referred to as "serum-free medium") was used for the naturalization of transformants established from the YB2/0 cell to the serum-free medium. The transformant established from the FUT8 gene double knock out cell naturalized in the protein-free medium in this manner was named as clone Ms704/CD20, the transformant established from the CHO/DG44 cell was named as clone DG44/CD20, the transformant established from the Lec13 cell was named as clone Lec13/CD20, and the transformant established from the YB2/0 cell was named as clone YB/CD20. In this connection, the clone Ms704/CD20 has been deposited on August 13, 2004, as FERM BP-10092 in International Patent Organism Depositary, National Institute of Advanced Industrial Science and Technology (Tsukuba Central 6, 1-1, Higashi 1-Chome Tsukuba-shi, Ibaraki-ken, Japan).

2. Production of anti-CD20 human chimeric antibody by conical flask serum-free fed-batch culture

Using the clone Ms704/CD20, clone DG44/CD20, clone Lec13/CD20 and clone YB/CD20 established in the item 1 of this Example, serum-free fed-batch culture using a conical flask was carried out, and production of anti-CD20 human chimeric antibody by each clone was carried out.

(1) Serum-free fed-batch culture using conical flask

A medium prepared by further adding 20% (w/v) glucose solution to the serum-free medium of the previous item to a final concentration of 5000 mg/l (hereinafter referred to as "serum-free fed-batch culture medium") was used as the basal medium of the fed-batch culture. A medium containing various amino acids (0.177 g/l L-alanine, 0.593 g/l

L-arginine monohydrochloride, 0.177 g/l L-asparagine monohydrate, 0.212 g/l L-aspartic acid, 0.646 g/l L-cystine dihydrochloride, 0.530 g/l L-glutamic acid, 5.84 g/l L-glutamine, 0.212 g/l glycine, 0.297 g/l L-histidine monohydrochloride dihydrate, 0.742 g/l L-isoleucine, 0.742 g/l L-leucine, 1.031 g/l L-lysine monohydrochloride, 0.212 g/l L-methionine, 0.466 g/l L-phenylalanine, 0.283 g/l L-proline, 0.297 g/l L-serine, 0.671 g/l L-threonine, 0.113 g/l L-tryptophan, 0.735 g/l L-tyrosine disodium dihydrate, and 0.664 g/l L-valine), various vitamins (0.0918 mg/l d-biotin, 0.0283 g/l calcium D-pantothenate, 0.0283 g/l choline chloride, 0.0283 g/l folic acid, 0.0509 g/l myo-inositol, 0.0283 g/l niacinamide, 0.0283 g/l pyridoxal hydrochloride, 0.00283 g/l riboflavin, 0.0283 g/l thiamin hydrochloride, and 0.0918 mg/l cyanocobalamin) and 0.314 g/l insulin was used as the feed medium.

Each of the clone Ms704/CD20, clone DG44/CD20, clone Lec13/CD20 and clone YB/CD20 was suspended in the serum-free fed-batch culture medium at a density of $3\times10^5$ cells/ml, and 40 ml of said cell suspension was inoculated into a 250 ml capacity conical flask (manufactured by Coming Glassworks). After substituting air in the flask through the blowing of 5% $CO_2$ gas in an amount of 4 volumes or more of the culture container, the container was sealed to carry out the culturing at 35°C while stirring at a revolution speed of 90 to 100 rpm. On the 3rd day, 6th day, 9th day and 11th day after commencement of the culturing, 3.3 ml of the above-described feed medium was added for the purpose of supplementing consumption of amino acids and the like, and 20% (w/v) glucose solution was added to a final concentration of 5000 mg/l for the purpose of controlling glucose concentration. Also, on the 0th day, 3rd day, 6th day, 9th day, 11th day and 13th day, about 2 ml of the culture liquid was collected, and viable cell density and cell survival rate were measured by the trypan blue staining method, and the concentration of anti-CD20 human chimeric antibody contained in each culture supernatant was measured by the ELISA method described in the item 2 of Example 3, respectively.

The fed-batch culturing was completed when the cell survival rate of each clone became 60% or less. Viable cell density and cell survival ratio of the clone Ms704/CD20 at each period after commencement of the culturing were identical to or larger than those of the clone DG44/CD20. On the other hand, the clone Lec13/CD20 was slow in the growing ability and also low in the maximum cell reach density. Also, the cell survival ratio of the clone YB/CD20 rapidly lowered after reaching the maximum cell reach density, so that it was difficult to carry out the fed-batch culturing for a prolonged period of time. In addition, regarding the antibody production, accumulated amount of the antibody increased in proportion to the cumulative viable cell density in all of the clones, and reached maximum production level at the time of the completion of culturing. Accordingly, it was revealed that, even in the cell clone in which the FUT8 gene was disrupted, difference in the surviving ability due to cell growth property and culturing stress was not found in comparison with the parent clone, and it shows good culturing behavior in comparison with other clones capable of expressing high ADCC activity antibody having high ratio of sugar chains to which fucose is not bound.

(2) Analysis of biological activities of antibody compositions produced by serum-free fed-batch culture

Using the culture liquids periodically collected from the clone Ms704/CD20, clone DG44/CD20, clone Lec13/CD20 and clone YB/CD20 during their serum-free fed-batch culturing of the above-described (1), the ratio of sugar chains, in which fucose is not bound, in the anti-CD20 human chimeric antibody composition contained in each of the cultured mediums, was analyzed. The ratio of sugar chains in which fucose is not bound was measured by the ELISA method using the binding activity for soluble human FcγRIIIa (hereinafter referred to as "shFcγRIIIa") as the index, based on the conventionally known method described in *Biotechnology and Bioengineering,* 87, 618, (2004). As the standard antibodies having already known ratio of sugar chains in which fucose is not bound, the KM2760-1 (ratio of sugar chains in which fucose is not bound: 90%) and KM3060 (ratio of sugar chains in which fucose is not bound: 10%) described in the item 5 of Example 4 of WO 03/085119 were used. The cultured mediums collected from the respective clones in the above-described (1) and the standard antibodies were prepared into antibody solutions of 5 μg/ml by diluting with 1% BSA-PBS and used as the evaluation samples.

As a result of the measurement, the binding activity for shFcγRIIIa was hardly found in samples of the clone DG44/CD20, so that it was revealed that an antibody composition having a sugar structure to which fucose is bound is contained therein. In the case of the samples of clone Lec13/CD20 and YB/CD20, it was revealed that they contain an antibody composition having high ratio of sugar chains in which fucose is not bound, in the early stage of the culturing, but the ratio of antibody composition having a sugar structure in which fucose is bound is increased as the culturing period prolongs. The ratio of sugar chains in which fucose is not bound was 60% or less in the anti-CD20 human chimeric antibody contained in the sample at the time of completion of the fed-batch culturing of clone Lec13/CD20. On the other hand, samples of the clone Ms704/CD20 showed strong binding activity against shFcγRIIIa stably throughout the culturing period. When the ratio of the sugar chain in which fucose is not bound in the anti-CD20 human chimeric antibody contained in the samples of clone Ms704/CD20 was calculated based on the absorbance of ELISA on the standard antibodies, it was estimated that this is kept at 100% throughout the culturing period.

Based on the above results, it was revealed that the FUT8 gene double knock out cell naturalized in the serum-free medium can stably produce the antibody composition having a fucose free sugar chain of N-acetylglucosamine in

the reducing end in the complex type N-glycoside-linked sugar chain, by the serum-free fed-batch culture using a conical flask.

3. Production of anti-CD20 human chimeric antibody having a sugar chain in which fucose is not bound, by suspension agitation reactor serum-free fed-batch culture

An examination was carried out on whether or not stable production of an antibody having a sugar chain in which fucose is not bound can be made even by the suspension agitation reactor serum-free fed-batch culture which is used for the commercial production of antibody medicines.

(1) Expansion culturing of seed cells

EX-CELL 302 medium containing 500 nM of MTX and 1.75 g/l of L-glutamine (hereinafter referred to as "medium for expansion culturing") was used as the medium for carrying out expansion culturing until reaching the reactor culturing. About from 10 to 30% volume of the medium for expansion culturing was put into a 125 ml, 250 ml or 1000 ml capacity conical flask (manufactured by Coming Glassworks), and a cell suspension was inoculated to a density of $3 \times 10^5$ cells/ml and cultured at 37°C for 4 days. Sub-culturing was repeated several times until the number of cells necessary for the inoculation of the reactor culturing was obtained.

(2) Reactor culturing

A medium prepared by further adding 500 nM of MTX and 1.75 g/l of L-glutamine to the medium for expansion culturing of (1) (hereinafter referred to as "medium for reactor culturing") was used as the basal medium of the reactor culturing.

When the number of necessary cells was obtained by the above-described expansion culturing, the cells were inoculated into a 1 liter capacity bioreactor (manufactured by ABLE) filled with 700 ml of the medium for reactor culturing, to a density of 3x105 cells/ml, and cultured for 17 days under a condition of 35°C, pH 7.1 and DO 50%. A medium consisting of amino acids (0.14 g/l L-alanine, 0.47 g/l L-arginine monohydrochloride, 0.16 g/l L-asparagine monohydrate, 0.17 g/l L-aspartic acid, 0.51 g/l L-cystine dihydrochloride, 0.42 g/l L-glutamic acid, 7.3 g/l L-glutamine, 0.17 g/l glycine, 0.24 g/l L-histidine monohydrochloride dihydrate, 0.59 g/l L-isoleucine, 0.59 g/l L-leucine, 0.82 g/l L-lysine monohydrochloride, 0.17 g/l L-methionine, 0.37 g/l L-phenylalanine, 0.22 g/l L-proline, 0.24 g/l L-serine, 0.53 g/l L-threonine, 0.09 g/l L-tryptophan, 0.58 g/l L-tyrosine disodium dihydrate, and 0.3 g/l L-valine), vitamins (0.073 mg/l d-biotin, 0.022 g/l calcium D-pantothenate, 0.022 g/l choline chloride, 0.022 g/l folic acid, 0.040 g/l myo-inositol, 0.022 g/l niacinamide, 0.022 g/l pyridoxal hydrochloride, 0.0022 g/l riboflavin, 0.022 g/l thiamin hydrochloride, and 0.073 mg/l cyanocobalamin), 0.31 g/l recombinant human insulin (manufactured by JRH), 0.025 g/l ethanolamine (manufactured by Sigma-Aldrich), 0.0098 g/l 2-mercaptoethanol (manufactured by Sigma-Aldrich), 8 g/l soybean hydrolysate HY-SOY (manufactured by Quest International), 16.8 μg/l sodium selenite (manufactured by Sigma-Aldrich), 2 ml/l cholesterol lipid concentrated solution (250 × aqueous solution, manufactured by Invitrogen) and 0.05 g/l ferric ethylenediaminetetraacetate sodium salt (manufactured by Sigma-Aldrich) was used as the feed medium, and 8.3% of the initial medium volume was added on the 3rd, 5th, 7th, 9th and 11th days of the culturing. In addition, 500 g/l glucose solution was optionally added in such a manner that glucose concentration on and after the 3rd day of the culturing became about 4 g/l.

By collecting the culture liquid once a day from commencement of the culturing until completion of the culturing, viable cell density (cells/ml) and cell survival rate were measured by a dye-exclusion method using 0.4% trypan blue solution (manufactured by Invitrogen), and antibody concentration (mg/l) measured by HPLC, respectively.

Specific antibody production rate was calculated by the following formula. In this connection, regarding the cumulative viable cell density (cells/ml × day), the product of viable cell density (cells/ml) and unit time (day) was calculated at each measuring time, and their total was used as the value. In this Example, the viable cell density was measured once a day, so that the viable cell density × day at each time was totaled and used as the cumulative viable cell density.

$$\text{Specific antibody production rate (pg/cell/day)} = \text{antibody concentration (mg/l)} \div \text{cumulative viable cell density (cells/ml} \times \text{day)}$$

The results of carrying out serum-free fed-batch culture using a suspension agitation reactor are shown in Fig. 8. The viable cell density reached the maximum on the 13th day of the culturing. The cell survival rate maintained a high value of 90% or more from the commencement of culturing until on the 13th day of the culturing, gradually decreased thereafter and became 12% on the 17th day of the culturing. The cumulative viable cell density was $5.6 \times 10^7$ cells/ml $\times$ day in 17 days, the antibody concentration at the time of the completion of culturing reached 1.7 g/l, and the specific antibody production rate showed 30 pg/cell/day. This result exceeded the standard production titer of antibody medicines, 0.5 to 1.0 g/l. In addition, asparagine binding type sugar chain was released from the antibody samples purified from the cultured mediums collected on the 5th, 7th, 14th and 17th days after commencement of the culturing, using N-glycanase F. After deproteinization and subsequent desalting by an ion exchange resin, sugar chain structures were analyzed by a mass spectrometer. At each point of time of the 5th, 7th, 14th and 17th days after commencement of the culturing, sugar chains in which fucose is bound were less than the detectable limit, and the antibody having the complex type N-glycoside-linked sugar chain in which fucose is not bound was stably produced.

Based on the above results, it was confirmed that the FUT8 gene double knock out cell naturalized in the serum-free medium can stably and highly produce the antibody composition having a sugar chain in which fucose is not bound to the N-acetylglucosamine in the reducing end of the complex type N-glycoside-linked sugar chain, also by the serum-free fed-batch culture using a suspension agitation reactor which is used in the commercial production of antibody medicines.

4. Biological activities of antibody composition which is produced by the serum-free fed-batch culturing and to which fucose is not bound

By measuring biological activities of the anti-CD20 human chimeric antibody produced in the item 3 of this Example (hereinafter also referred to as "Ms704/CD20 antibody"), superiority of the antibody composition produced by the present production method was confirmed. As the comparative control, the anti-CD20 human chimeric antibody described in the item 2 of this Example (hereinafter referred also to as "DG44/CD20 antibody") was used, which was produced using the CHO/DG44 cell used in the production of commercially available antibodies.

(1) Antigen binding activity of anti-CD20 human chimeric antibody against CD20 antigen expressed clone

When the activity was measured in accordance with the fluorescent antibody technique described in the item 1 of Example 2 of WO 03/055993, a difference in the antibody staining strength on Raji cell was not observed by the FACS analysis, and a difference in the antigen binding activity was not observed between the anti-CD20 human chimeric antibody produced in the item 3 of this Example and the anti-CD20 human chimeric antibody produced using the CHO/DG44 cell described in the item 2 of this Example. Accordingly, it was confirmed that when the production is carried out using the FUT8 gene double knock out cell naturalized in the serum-free medium, the product has the antigen binding activity equivalent to that of the antibody composition produced by using the parent cell line CHO/DG44 cell.

(2) *Ex vivo* cytotoxic activity (ADCC activity) of anti-CD20 human chimeric antibodies

ADCC activity, in human peripheral blood, of the anti-CD20 human chimeric antibody produced in the item 3 of this Example and the anti-CD20 human chimeric antibody produced using the CHO/DG44 cell described in the item 2 of this Example was measured in the following manner.

Each of the Ms704/CD20 antibody and DG44/CD20 antibody was diluted with Dulbecco's PBS (manufactured by Invitrogen) and dispensed in 100 μl/well portions into each well of a 24 well flat bottom plate (manufactured by Greiner). Next, a human peripheral blood sample collected from a healthy person was mixed with heparin sodium (manufactured by Shimizu Pharmaceutical) and dispensed in 500 μl/well portions, followed by culturing at 37°C overnight in a 5% $CO_2$ incubator. After the reaction, 150 μl of the reaction liquid was collected from each well and washed with 1% BSA-PBS, and then an FITC-labeled mouse anti-CD19 monoclonal antibody (manufactured by Beckman Coulter) and a PE-labeled mouse anti-CD2 monoclonal antibody (manufactured by Pharmingen) were added thereto and allowed to react at room temperature for 30 minutes in the dark. Removal of erythrocyte and cell fixation treatment were carried out using FACS Lysing Solution (manufactured by Becton Dickinson), and after washing with 1% BSA-PBS, the fixed cells were suspended in 500 μl of 1% BSA-PBS and filtered through a cell strainer (manufactured by Falcon) to prepare as an analyzing sample. A fraction containing about 5,000 lymphocytes per sample was measured using a flow cytometer FACS Caliber (manufactured by Becton Dickinson), and the ratio of CD2-negative and CD19-positive B cell against the total cells was calculated.

The results are shown in Fig. 9. Under the Ms704/CD20 antibody added condition, the ratio of B cell was reduced compared to the DG44/CD20 antibody added condition, so that high ADCC activity of the Ms704/CD20 antibody upon B cells was shown. Based on this result, it was confirmed that the antibody composition produced by the FUT8

gene double knock out cell naturalized in the serum-free medium has higher cytotoxic activity upon human blood plasma than that of the antibody composition produced by its parent cell line CHO/DG44 cell.

*(3) In vitro* cytotoxic activity (ADCC activity) of anti-CD20 human chimeric antibodies

*In vitro* ADCC activity of the anti-CD20 human chimeric antibody produced in the item 3 of this Example and the anti-CD20 human chimeric antibody produced by its parent cell line CHO/DG44 cell described in the item 2 of this Example was measured using a human B lymphocyte cell line WIL2-S cell (ATCC CRL-8885) which expresses human CD20 antigen, as the target cell, in accordance with the method described in Example 3.

The results are shown in Fig. 10. At each of the antibody concentrations, the Ms704/CD20 antibody showed higher ADCC activity upon WIL2-S cell than the DG44/CD20 antibody added condition.

Next, an anti-CD20 human chimeric antibody composition in which ratio of the antibody having a sugar chain in which fucose is not bound was changed by adding DG44/CD20 antibody to a fixed amount of Ms704/CD20 antibody was prepared, and its ADCC activity was measured. Specifically, an anti-CD20 human chimeric antibody composition in which from 0 to 300 ng/ml of DG44/CD20 antibody was added to 3.7 ng/ml of Ms704/CD20 antibody was prepared.

The results are shown in Fig. 11. When Ms704/CD20 antibody was further added to 3.7 ng/ml of Ms704/CD20 antibody, increase of ADCC activity was observed with the increase of the total antibody concentration, but ADCC activity of the prepared antibody composition was conversely decreased when DG44/CD20 antibody was further added to 3.7 ng/ml of Ms704/CD20 antibody, in spite of the increase in the total antibody concentration. This fact shows that an antibody molecule having a sugar chain in which fucose is bound inhibits ADCC activity of an antibody molecule having a sugar chain in which fucose is not bound. In addition, also in the case of antibody compositions in which an antibody molecule having a sugar chain in which fucose is bound was mixed with an antibody molecule having a sugar chain in which fucose is not bound, the antibody composition having 20% or more of the ratio of the antibody having a sugar chain in which fucose is not bound showed considerably high ADCC activity in comparison with that of the antibody composition having less than 20% of the said ratio.

Furthermore, ADCC activity of 1 ng/ml of an Ms704/CD20 antibody sample and an antibody prepared by adding 9 volumes of 9 ng/ml of DG44/CD20 antibody to 1 ng/ml of Ms704/CD20 antibody was measured.

The results are shown in Fig. 12. ADCC activity of Ms704/CD20 antibody was sharply decreased by the addition of DG44/CD20 antibody. In addition, even when antibody concentration of the antibody composition was increased 100-fold or more while keeping the existence ratio of Ms704/CD20 antibody to DG44/CD20 antibody at 1:9, it was not equal to the ADCC activity of the 1 ng/ml Ms704/CD20 antibody sample.

Based on the above, it was revealed that an antibody molecule having a sugar chain in which fucose is bound inhibits ADCC activity of an antibody molecule having a sugar chain in which fucose is not bound, and that the conventional antibody compositions cannot exert the ADCC activity which is equivalent to the antibody composition of the present invention.

In this connection, similar results were obtained from other antibody compositions produced by the production method of the present invention.

Industrial Applicability

The present invention provides a cell in which a genomic gene encoding an enzyme relating to a sugar chain modification in which 1-position of fucose is bound to 6-position of N-acetylglucosamine in the reducing end through $\alpha$-bond in a complex type N-glycoside-linked sugar chain is knocked out, wherein the cell is naturalized in a serum-free medium; a process for producing a glycoprotein composition using the cell; and a glycoprotein composition produced by the process.

Free Texts of Sequence Listing

SEQ ID NO:10 - Explanation of artificial sequence: Synthetic DNA
SEQ ID NO:11 - Explanation of artificial sequence: Synthetic DNA
SEQ ID NO:12 - Explanation of artificial sequence: Synthetic DNA
SEQ ID NO:13 - Explanation of artificial sequence: Synthetic DNA
SEQ ID NO:14 - Explanation of artificial sequence: Synthetic DNA
SEQ ID NO:15 - Explanation of artificial sequence: Synthetic DNA
SEQ ID NO:16 - Explanation of artificial sequence: Synthetic DNA
SEQ ID NO:17 - Explanation of artificial sequence: Synthetic DNA

SEQ ID NO:20 - Explanation of artificial sequence: Synthetic DNA
SEQ ID NO:21 - Explanation of artificial sequence: Synthetic DNA
SEQ ID NO:22 - Explanation of artificial sequence: Synthetic DNA
SEQ ID NO:23 - Explanation of artificial sequence: Synthetic DNA
SEQ ID NO:24 - Explanation of artificial sequence: Synthetic DNA
SEQ ID NO:25 - Explanation of artificial sequence: Synthetic DNA
SEQ ID NO:26 - Explanation of artificial sequence: Synthetic DNA
SEQ ID NO:27 - Explanation of artificial sequence: Synthetic DNA
SEQ ID NO:28 - Explanation of artificial sequence: Synthetic DNA
SEQ ID NO:29 - Explanation of artificial sequence: Synthetic DNA
SEQ ID NO:30 - Explanation of artificial sequence: Synthetic DNA
SEQ ID NO:31 - Explanation of artificial sequence: Synthetic DNA
SEQ ID NO:32 - Explanation of artificial sequence: Synthetic DNA

## Claims

1. A cell in which a genomic gene encoding an enzyme relating to a sugar chain modification in which 1-position of fucose is bound to 6-position of N-acetylglucosamine in the reducing end through α-bond in a complex type N-glycoside-linked sugar chain is knocked out, wherein the cell is naturalized in a serum-free medium.

2. The cell according to claim 1, wherein all of alleles on a genome encoding an enzyme relating to modification of a sugar chain in which 1-position of fucose is bound to 6-position of N-acetylglucosamine in the reducing end through α-bond in a complex type N-glycoside-linked sugar chain are knocked out, and wherein the cell is naturalized in a serum-free medium.

3. The cell according to claim 1 or 2, wherein an exon region containing an initiation codon of the genomic gene encoding an enzyme relating to modification of a sugar chain in which 1-position of fucose is bound to 6-position of N-acetylglucosamine in the reducing end through α-bond in a complex type N-glycoside-linked sugar chain is deleted, and wherein the cell is naturalized in a serum-free medium.

4. The cell according to any one of claims 1 to 3, wherein the enzyme relating to modification of a sugar chain in which 1-position of fucose is bound to 6-position of N-acetylglucosamine in the reducing end through α-bond in a complex type N-glycoside-linked sugar chain is α-1,6-fucosyltransferase.

5. The cell according to claim 4, wherein the α-1,6-fucosyltransferase is a protein encoded by a DNA selected from the following (a) or (b):

   (a) a DNA comprising the nucleotide sequence represented by SEQ ID NO:1;
   (b) a DNA which hybridizes with a DNA consisting of the nucleotide sequence represented by SEQ ID NO:1 under stringent conditions and encodes a protein having α-1,6-fucosyltransferase activity.

6. The cell according to claim 4, wherein the α-1,6-fucosyltransferase is a protein selected from the group consisting of the following (a), (b) and (c):

   (a) a protein comprising the amino acid sequence represented by SEQ ID NO:5;
   (b) a protein consisting of an amino acid sequence in which one or more amino acid(s) is/are deleted, substituted, inserted and/or added in the amino acid sequence represented by SEQ ID NO:5 and having α-1,6-fucosyltransferase activity;
   (c) a protein consisting of an amino acid sequence which has at least 80% amino acid sequence homology to the amino acid sequence represented by SEQ ID NO:5 and having α-1,6-fucosyltransferase activity.

7. The cell according to any one of claims 1 to 6, which is resistant to a lectin which recognizes a sugar chain structure in which 1-position of fucose is bound to 6-position of N-acetylglucosamine in the reducing end through α-bond in a complex type N-glycoside-linked sugar chain.

8. The cell according to claim 7, wherein said resistance is resistance in which the cell survives at a higher ratio than a cell in which the genomic gene has not been knocked out when the cells are cultured in a medium containing the lectin which recognizes a sugar chain structure in which 1-position of fucose is bound to 6-position of N-acetylglucosamine in the reducing end through α-bond in a complex type N-glycoside-linked sugar chain.

9. The cell according to any one of claims 1 to 7, wherein the serum-free medium is a protein-free medium.

10. The cell according to any one of claims 1 to 9, which comprises a gene encoding a glycoprotein.

11. The cell according to claim 10, wherein the glycoprotein is a glycoprotein having no sugar chain structure in which 1-position of fucose is bound to 6-position of N-acetylglucosamine in the reducing end through α-bond in a complex type N-glycoside-linked sugar chain.

12. The cell according to claim 10 or 11, wherein the glycoprotein is an antibody.

13. The cell according to claim 12, wherein the antibody belongs to an IgG class.

**14.** A process for producing a glycoprotein composition, which comprises using the cell according to any one of claims 1 to 13.

**15.** A process for producing a glycoprotein composition, which comprises culturing the cell according to any one of claims 1 to 13 in a medium to form and accumulate the glycoprotein composition in the culture, and recovering and purifying the glycoprotein composition from the culture.

**16.** The process for producing a glycoprotein composition according to claim 14 or 15, wherein the process is carried out by batch culture, fed-batch culture or perfusion culture.

**17.** The process according to any one of claims 14 to 16, wherein at least one selected from a nutrient factor and a physiologically active substance is added to the medium during culturing.

**18.** The process according to claim 17, wherein the nutrient factor is at least one selected from a glucose, an amino acid and a vitamin.

**19.** The process according to claim 17, wherein the physiologically active substance is at least one selected from an insulin, an insulin-like growth factor, transferrin and albumin.

**20.** The process according to any one of claims 14 to 19, wherein the glycoprotein composition is an antibody composition.

**21.** A method for naturalizing a cell in which a genomic gene encoding an enzyme relating to a sugar chain modification in which 1-position of fucose is bound to 6-position of N-acetylglucosamine in the reducing end through α-bond in a complex type N-glycoside-linked sugar chain is knocked out in a serum-free medium, which comprises inoculating the cell into a medium for naturalization to give a cell density of $1\times10^5$ to $1\times10^6$ cells/ml.

**22.** A method for obtaining a clone in which a genomic gene encoding an enzyme relating to a sugar chain modification in which 1-position of fucose is bound to 6-position of N-acetylglucosamine in the reducing end through α-bond in a complex type N-glycoside-linked sugar chain is knocked out, which comprises naturalizing the cell in a serum-free medium by the method according to claim 21, and then cloning the cell.

**23.** A cell in which a genomic gene encoding an enzyme relating to a sugar chain modification in which 1-position of fucose is bound to 6-position of N-acetylglucosamine in the reducing end through α-bond in a complex type N-glycoside-linked sugar chain is knocked out, wherein the cell is naturalized in a serum-free medium, which is obtainable by the method according to claim 21.

**24.** A clone in which a genomic gene encoding an enzyme relating to a sugar chain modification in which 1-position of fucose is bound to 6-position of N-acetylglucosamine in the reducing end through α-bond in a complex type N-glycoside-linked sugar chain is knocked out, wherein the clone is naturalized in a serum-free medium, which is obtainable by the method according to claim 22.

**25.** The method according to claim 21 or 22, wherein the serum-free medium is a protein-free medium,

**26.** The cell according to claim 23, wherein the serum-free medium is a protein-free medium.

**27.** The clone according to claim 24, wherein the serum-free medium is a protein-free medium.

FIG. 1

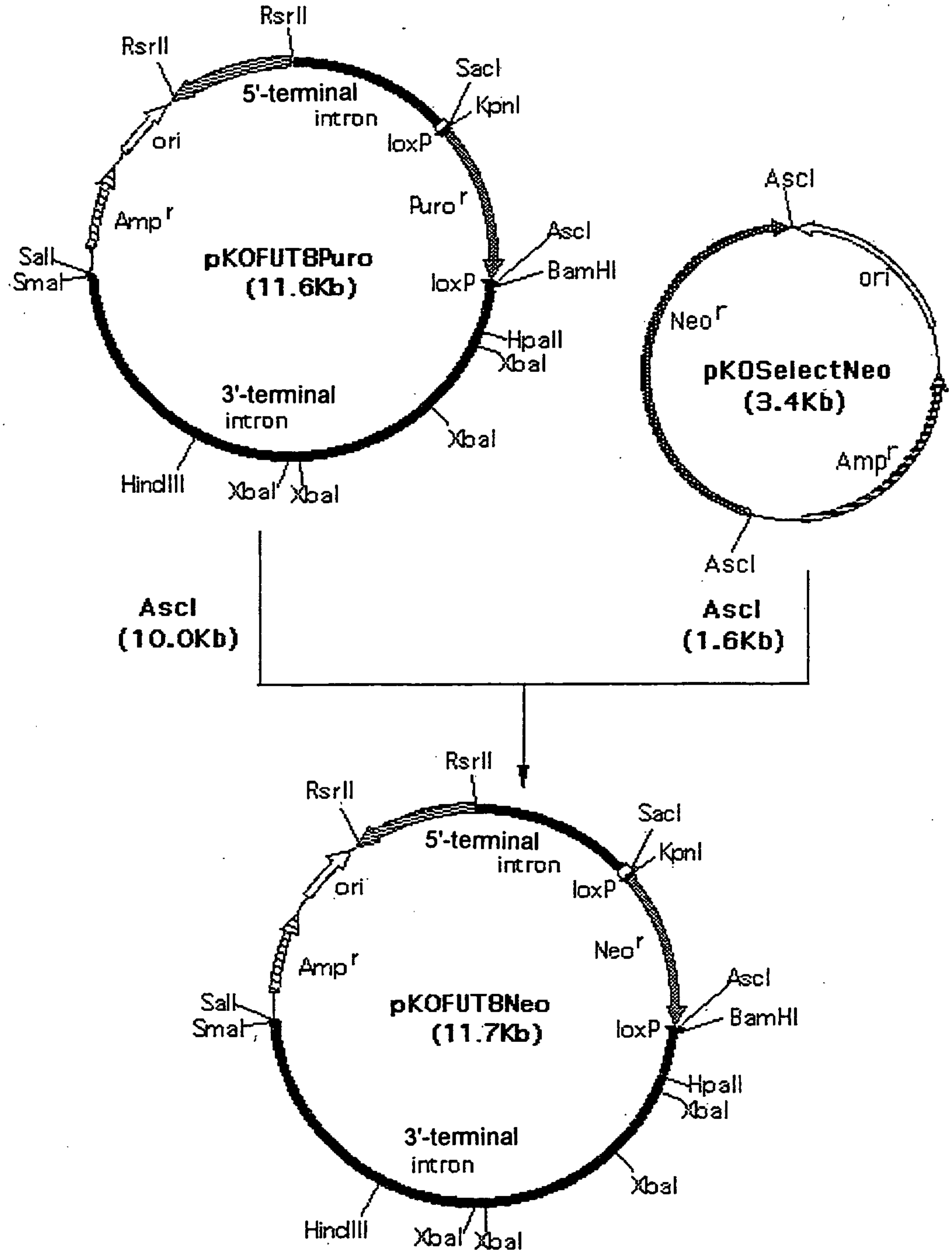
RsrII
RsrII
5'-terminal intron
SacI
KpnI
loxP
ori
Amp^r
Puro^r
AscI
SalI
SmaI
pKOFUT8Puro (11.6Kb)
loxP
BamHI
HpaII
XbaI
3'-terminal intron
XbaI
HindIII
XbaI
XbaI
AscI
ori
Neo^r
pKOSelectNeo (3.4Kb)
Amp^r
AscI
AscI (10.0Kb)
AscI (1.6Kb)
RsrII
RsrII
5'-terminal intron
SacI
KpnI
loxP
ori
Amp^r
Neo^r
AscI
SalI
SmaI
pKOFUT8Neo (11.7Kb)
loxP
BamHI
HpaII
XbaI
3'-terminal intron
XbaI
HindIII
XbaI
XbaI

FIG. 2

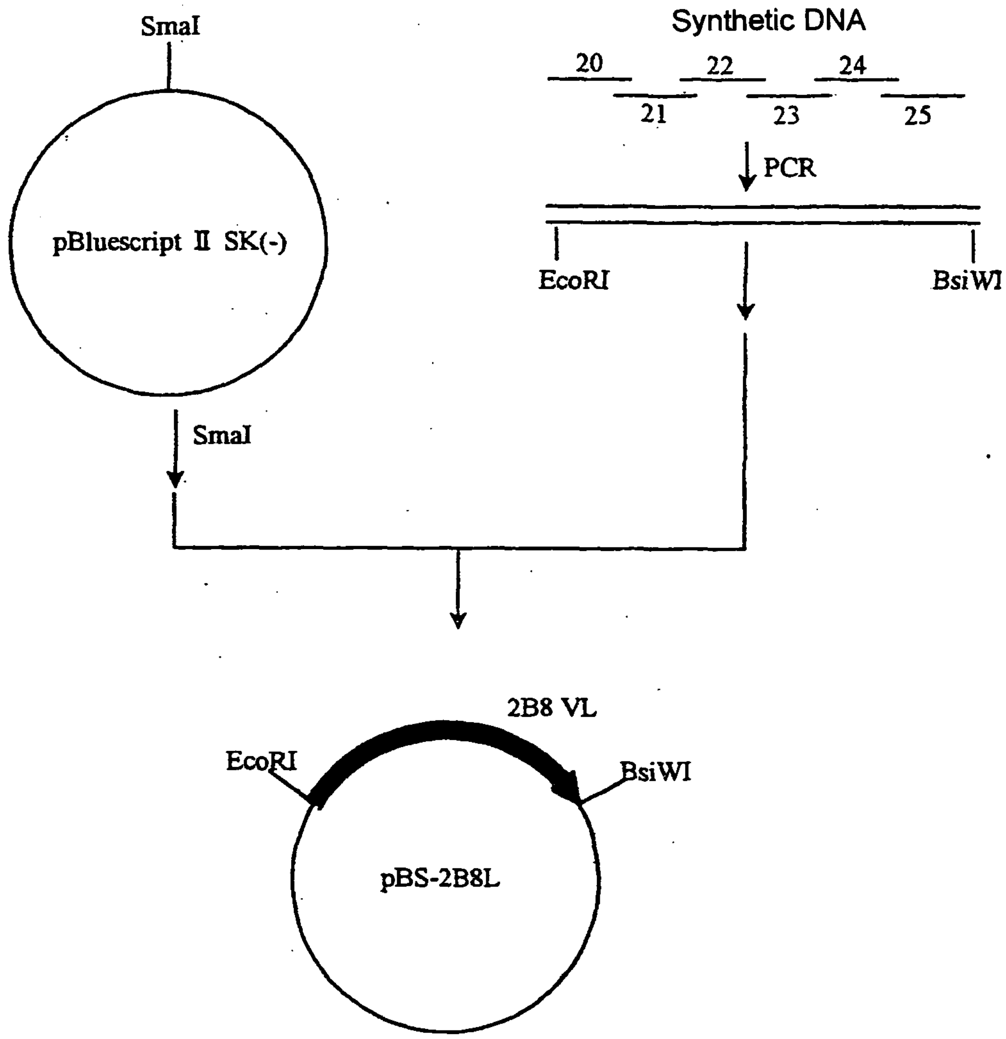
SmaI
pBluescript II SK(-)
Synthetic DNA
20
22
24
21
23
25
PCR
EcoRI
BsiWI
SmaI
2B8 VL
EcoRI
BsiWI
pBS-2B8L

FIG. 3

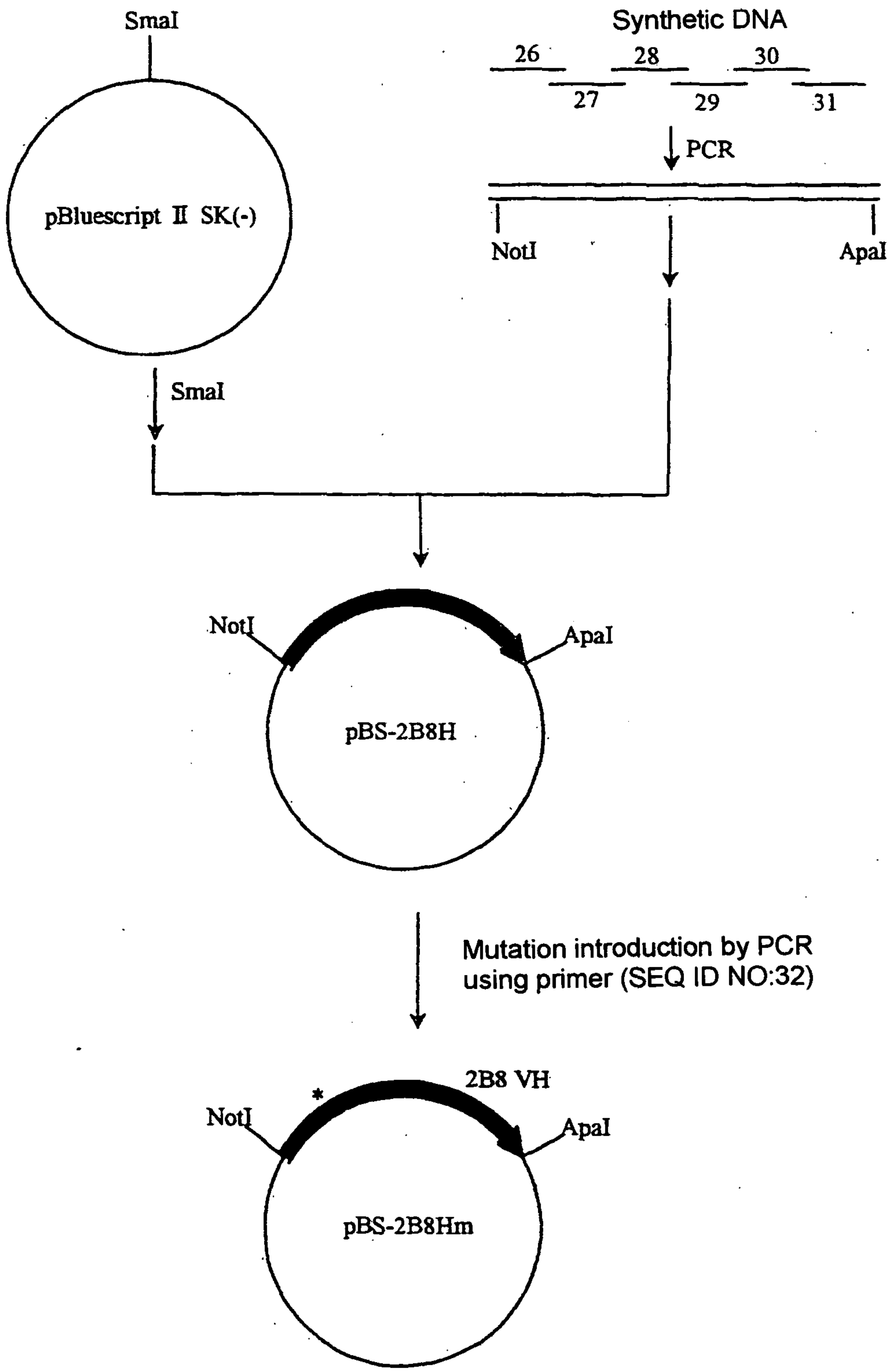
SmaI
Synthetic DNA
26
28
30
27
29
31
PCR
NotI
ApaI
pBluescript II SK(-)
SmaI
NotI
ApaI
pBS-2B8H
Mutation introduction by PCR using primer (SEQ ID NO:32)
2B8 VH
*
NotI
ApaI
pBS-2B8Hm

FIG. 4

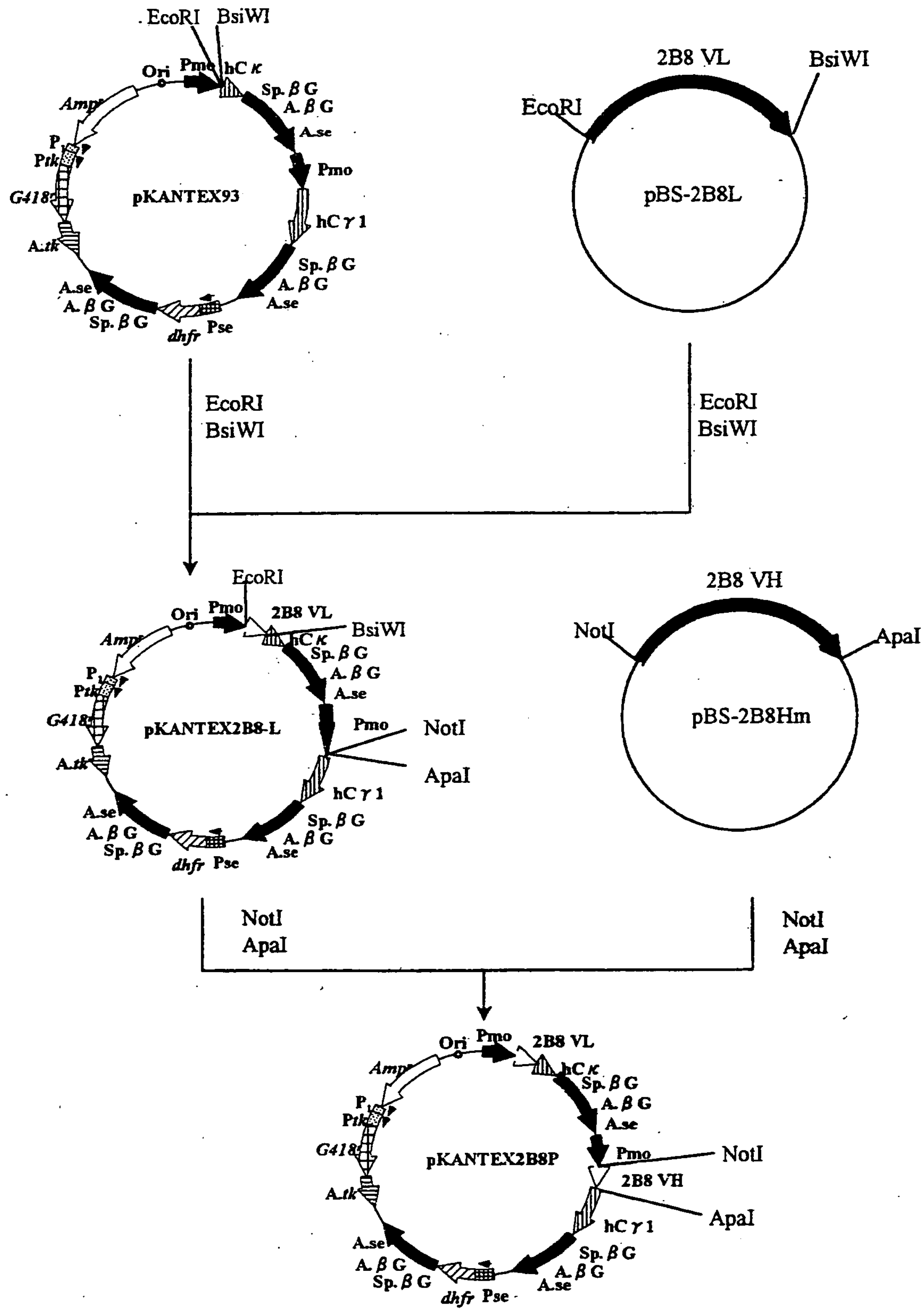
EcoRI
BsiWI
Ori
Pmo
hCκ
Sp.βG
A.βG
A.se
Pmo
hCγ1
Amp
P
Ptk
G418
A.tk
pKANTEX93
dhfr
Pse
2B8 VL
BsiWI
EcoRI
pBS-2B8L
EcoRI
BsiWI
EcoRI
BsiWI
pKANTEX2B8-L
NotI
ApaI
2B8 VH
NotI
ApaI
pBS-2B8Hm
NotI
ApaI
NotI
ApaI
pKANTEX2B8P
2B8 VH

FIG. 5

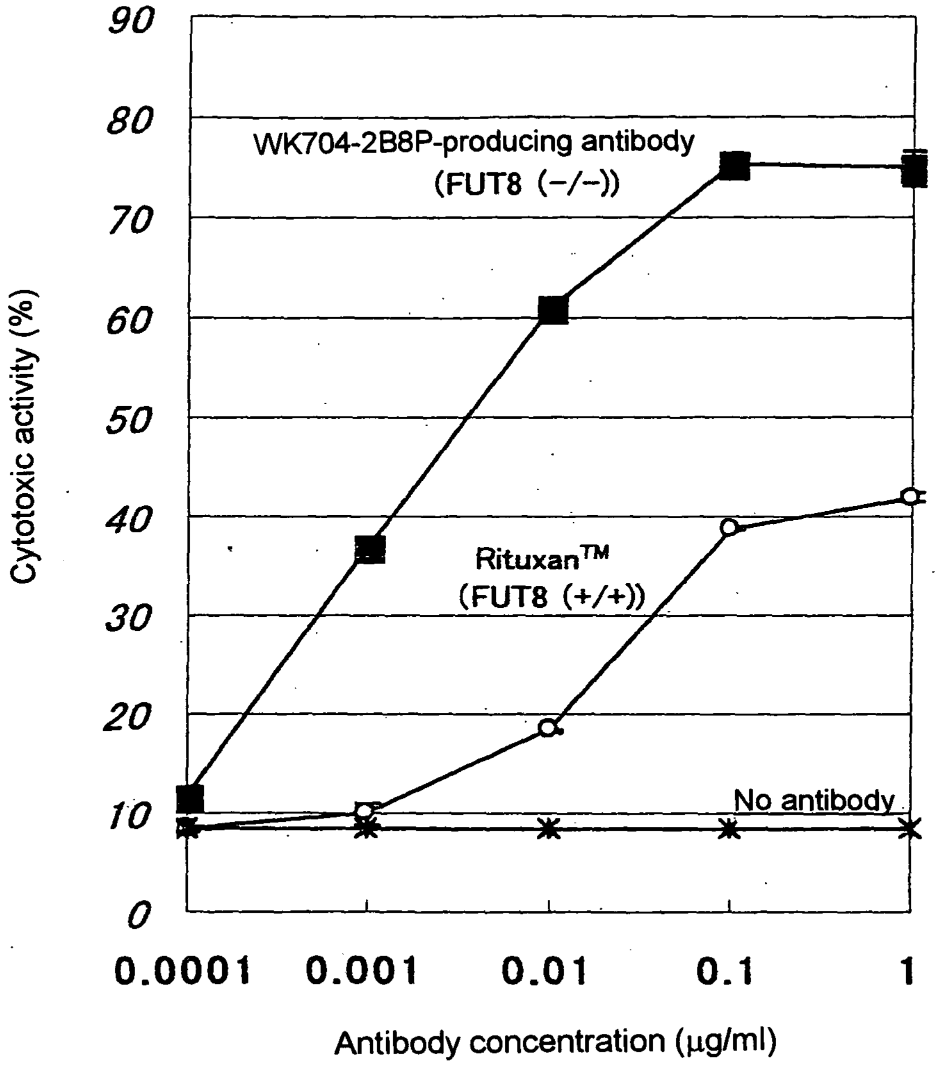
90
80
70
60
50
40
30
20
10
0
Cytotoxic activity (%)
WK704-2B8P-producing antibody
(FUT8 (-/-))
Rituxan™
(FUT8 (+/+))
No antibody
0.0001
0.001
0.01
0.1
1
Antibody concentration (μg/ml)

FIG. 6

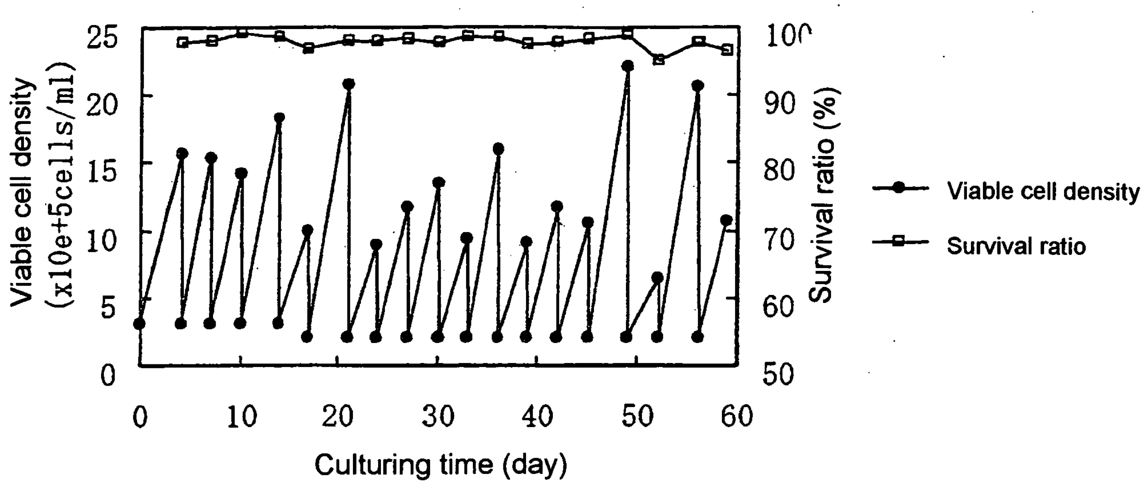
25
20
15
10
5
0
Viable cell density
(x10e+5cells/ml)
100
90
80
70
60
50
Survival ratio (%)
0
10
20
30
40
50
60
Culturing time (day)
Viable cell density
Survival ratio

FIG. 7

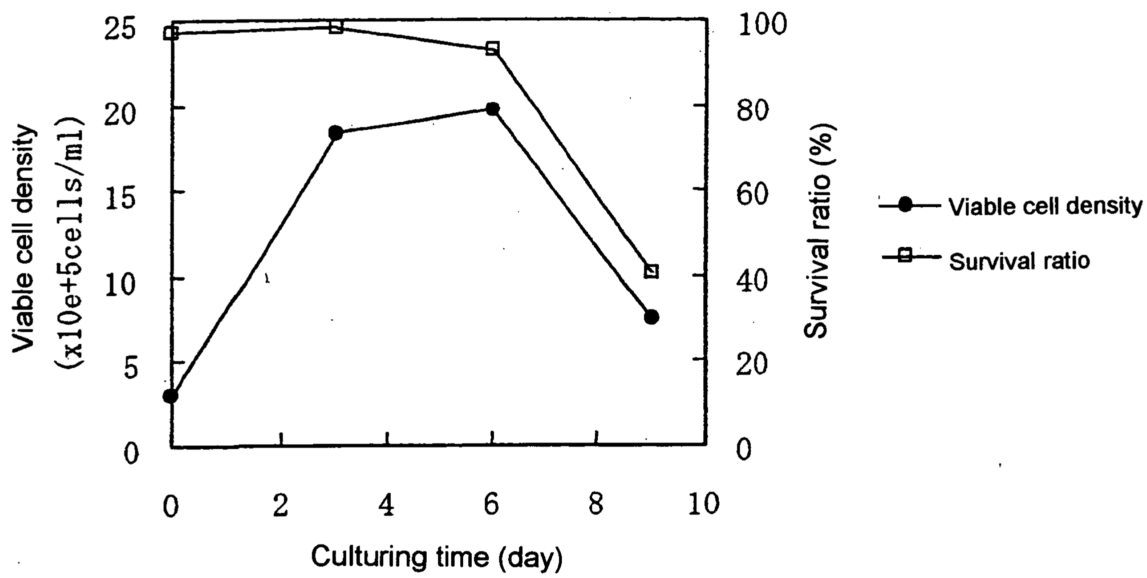
25
20
15
10
5
0
100
80
60
40
20
0
0
2
4
6
8
10
Viable cell density
(x10e+5cells/ml)
Survival ratio (%)
Culturing time (day)
Viable cell density
Survival ratio

FIG. 8

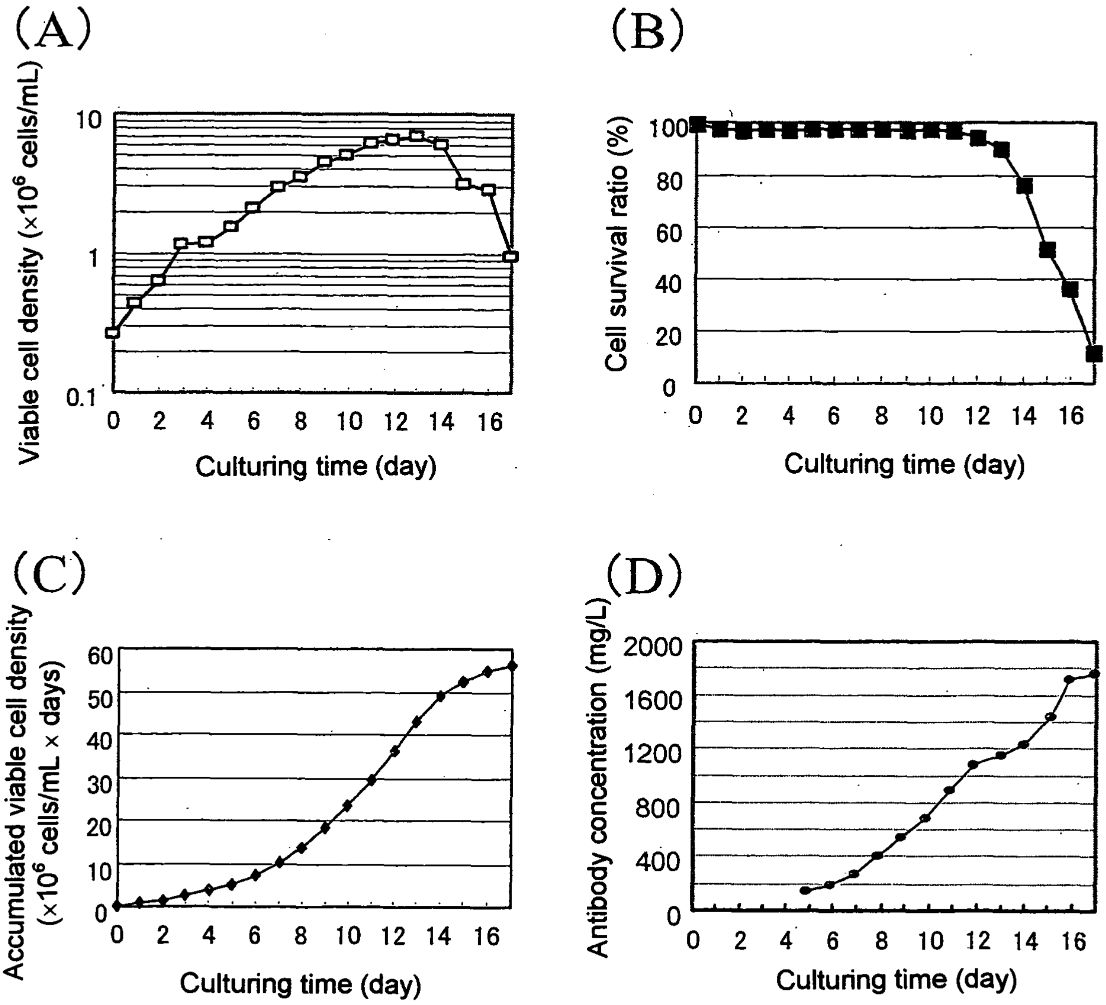
(A)
Viable cell density (×10^6 cells/mL)
10
1
0.1
0 2 4 6 8 10 12 14 16
Culturing time (day)
(B)
Cell survival ratio (%)
100
80
60
40
20
0
0 2 4 6 8 10 12 14 16
Culturing time (day)
(C)
Accumulated viable cell density (×10^6 cells/mL × days)
60
50
40
30
20
10
0
0 2 4 6 8 10 12 14 16
Culturing time (day)
(D)
Antibody concentration (mg/L)
2000
1600
1200
800
400
0
0 2 4 6 8 10 12 14 16
Culturing time (day)

FIG. 9

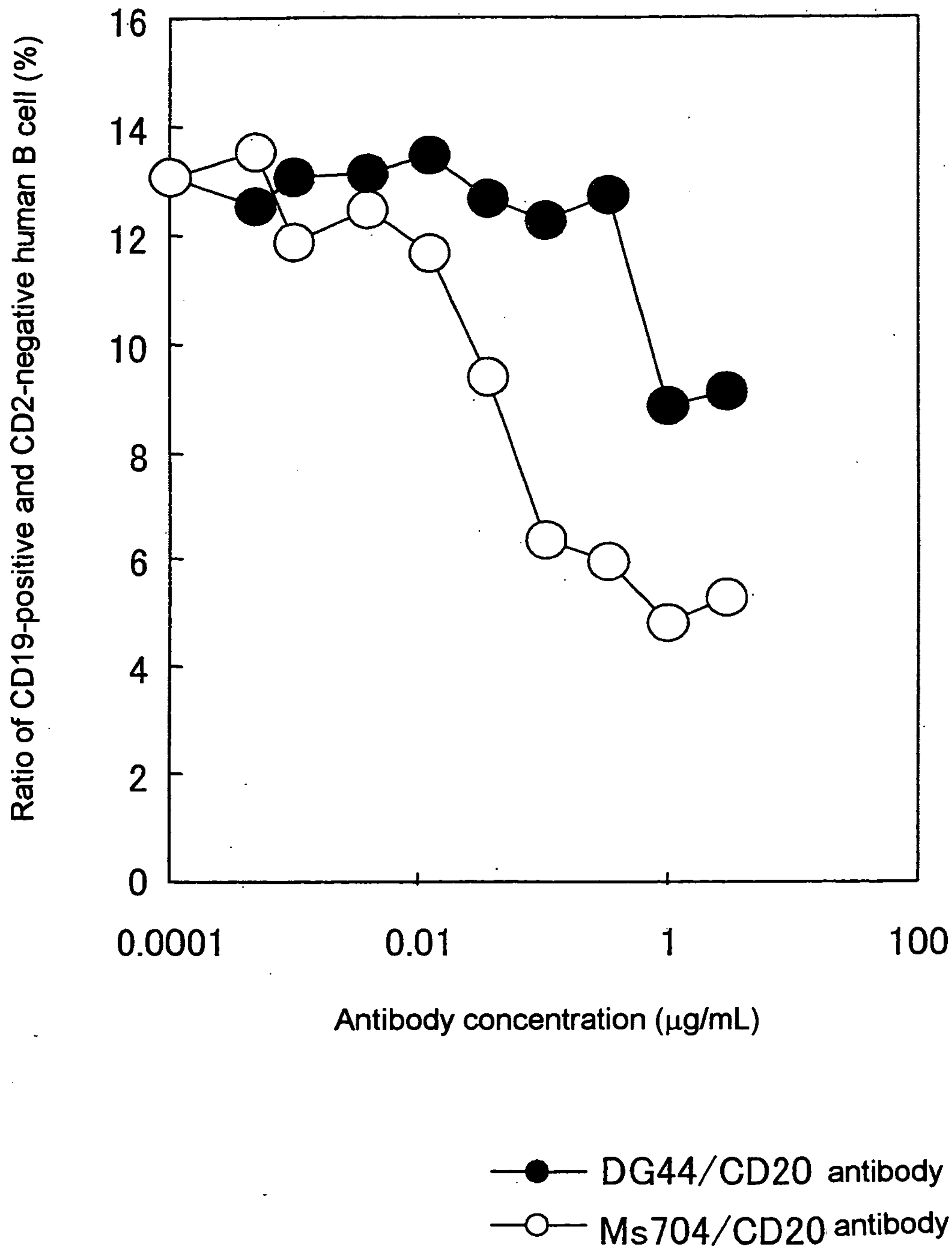
Ratio of CD19-positive and CD2-negative human B cell (%)
16
14
12
10
8
6
4
2
0
0.0001
0.01
1
100
Antibody concentration (μg/mL)
DG44/CD20 antibody
Ms704/CD20 antibody

FIG. 10

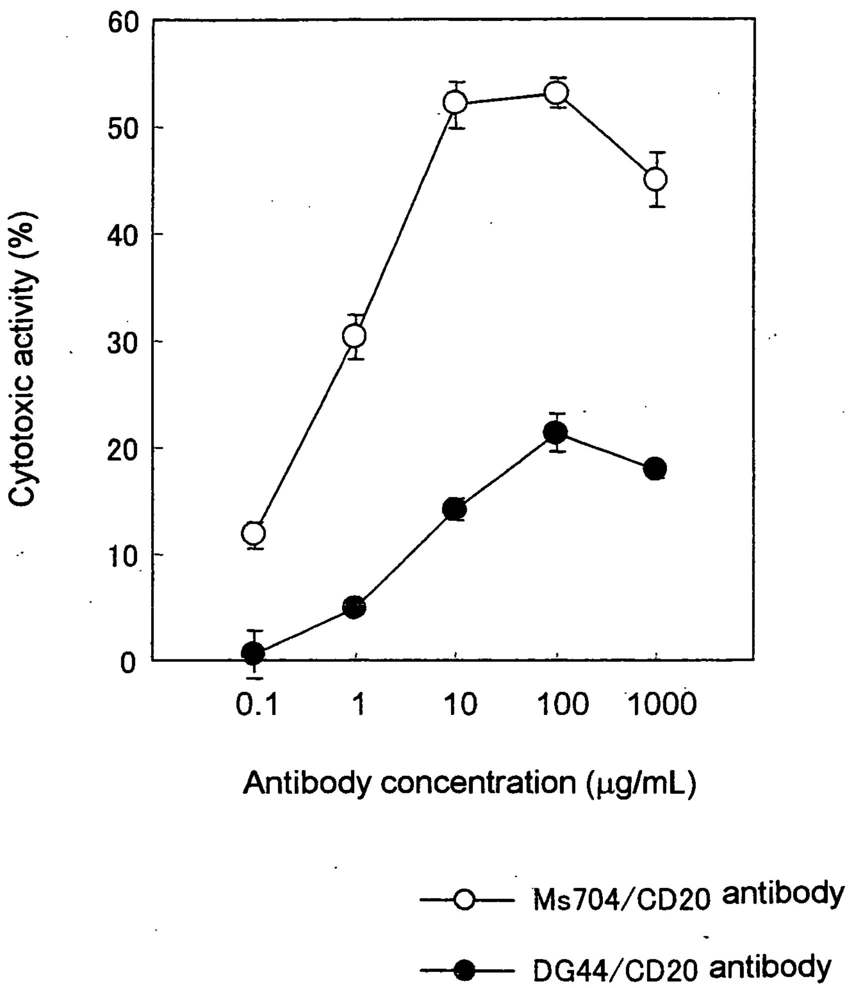

60
50
40
30
20
10
0
Cytotoxic activity (%)
0.1
1
10
100
1000
Antibody concentration (μg/mL)
Ms704/CD20 antibody
DG44/CD20 antibody

FIG. 11

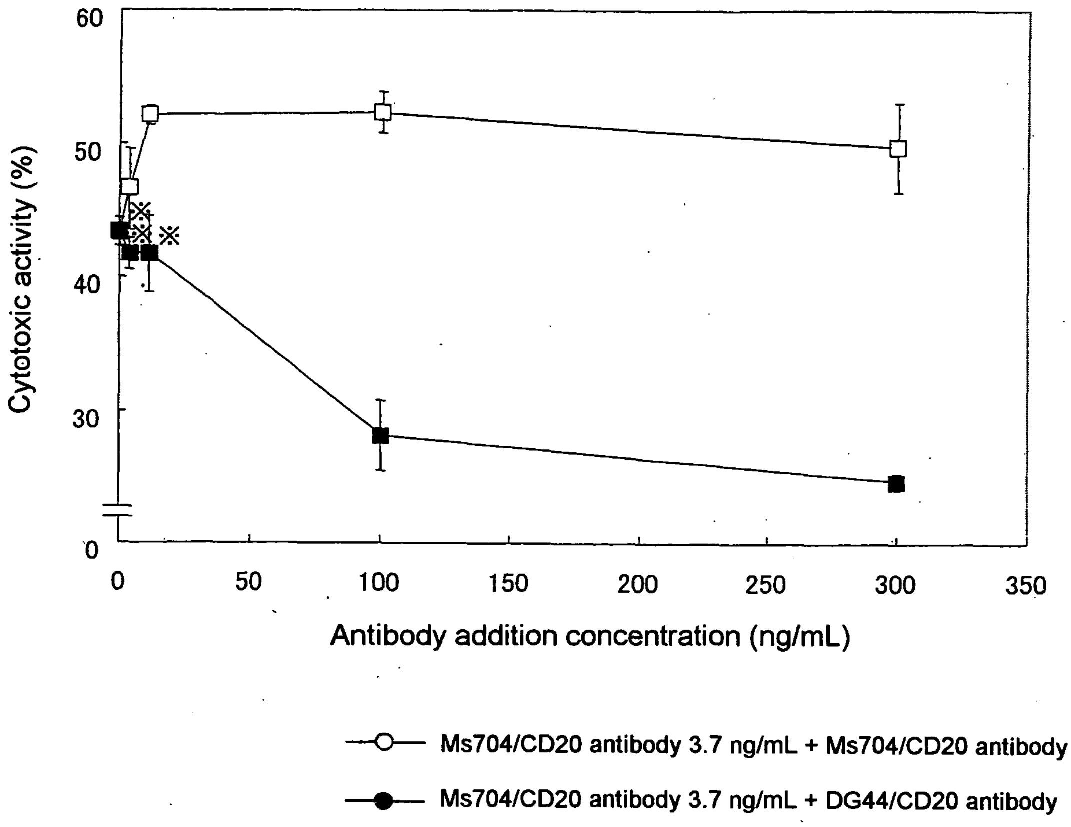
60
50
40
30
0
Cytotoxic activity (%)
※
※
※
0
50
100
150
200
250
300
350
Antibody addition concentration (ng/mL)
Ms704/CD20 antibody 3.7 ng/mL + Ms704/CD20 antibody
Ms704/CD20 antibody 3.7 ng/mL + DG44/CD20 antibody

FIG. 12

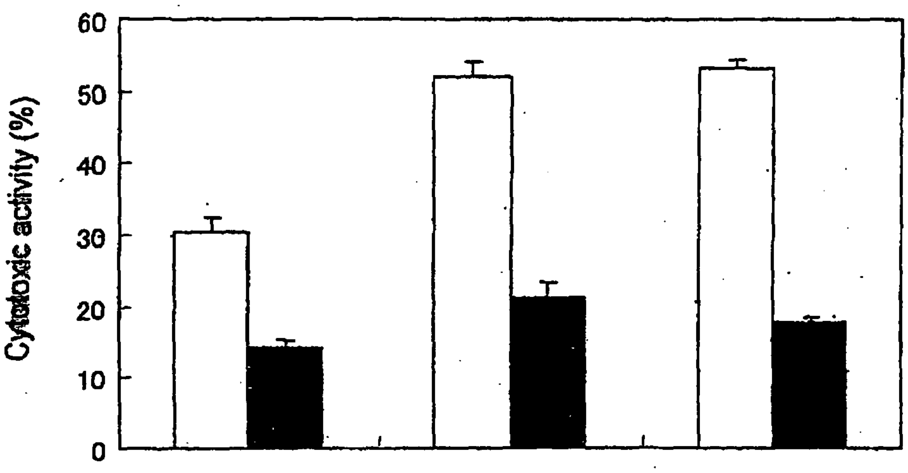


| | | | | | | |
|---|---|---|---|---|---|---|
| Ms704/CD20 antibody (ng/mL) | 1 | 1 | 10 | 10 | 100 | 100 |
| | + | + | + | + | + | + |
| DG44/CD20 antibody (ng/mL) | 0 | 9 | 0 | 90 | 0 | 900 |
| Total antibody concentration (ng/mL) | 1 | 10 | 10 | 100 | 100 | 1000 |

**INTERNATIONAL SEARCH REPORT**

International application No.
PCT/JP2004/015315

A. CLASSIFICATION OF SUBJECT MATTER
Int.Cl[7] C12N5/10, C12N15/13, C12P21/08

According to International Patent Classification (IPC) or to both national classification and IPC

B. FIELDS SEARCHED

Minimum documentation searched (classification system followed by classification symbols)
Int.Cl[7] C12N5/10, C12N15/13, C12P21/08

Documentation searched other than minimum documentation to the extent that such documents are included in the fields searched

Electronic data base consulted during the international search (name of data base and, where practicable, search terms used)
REGISTRY(SNT), CA(STN), WPI(DIALOG), BIOSIS(DIALOG), JSTPlus(JOIS), GenBank/EMBL/DDBJ/GeneSeq, SwissProt/PIR/GeneSeq

C. DOCUMENTS CONSIDERED TO BE RELEVANT

| Category* | Citation of document, with indication, where appropriate, of the relevant passages | Relevant to claim No. |
|---|---|---|
| X | WO 02/31140 A1 (Kyowa Hakko Kogyo Co., Ltd.), 18 April, 2002 (18.04.02), & AU 200194198 A & US 2003/0115614 A1 & EP 1331266 A1 & BR 200114475 A & KR 2003081312 A & MX 2003002974 A1 | 1-27 |
| P,X | YAMANE-OHNUKI, N. et al., Establishment of FUT8 knockout Chinese hamster ovary cells: an ideal host cell line for producing completely defucosylated antibodies with enhanced antibody-dependent cellular cytotoxicity. Biotechnol.Bioeng. 05 September, 2004 (05.09.04), Vol.87, No.5, pages 614 to 622 | 1-27 |

[X] Further documents are listed in the continuation of Box C. [ ] See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 13 January, 2005 (13.01.05) | 01 February, 2005 (01.02.05) |

| Name and mailing address of the ISA/ | Authorized officer |
|---|---|
| Japanese Patent Office | |
| Facsimile No. | Telephone No. |

Form PCT/ISA/210 (second sheet) (January 2004)

**INTERNATIONAL SEARCH REPORT**

International application No.
PCT/JP2004/015315

C (Continuation). DOCUMENTS CONSIDERED TO BE RELEVANT

| Category* | Citation of document, with indication, where appropriate, of the relevant passages | Relevant to claim No. |
|---|---|---|
| P,X | WO 03/85107 A1 (Kyowa Hakko Kogyo Co., Ltd.), 16 October, 2003 (16.10.03), & AU 2003236022 A1 & US 2004/0110704 A1 | 1-27 |
| P,X | WO 03/85118 A1 (Kyowa Hakko Kogyo Co., Ltd.), 16 October, 2003 (16.10.03), & AU 2003236015 A1 & US 2004/0132140 A1 | 1-27 |

Form PCT/ISA/210 (continuation of second sheet) (January 2004)

SEQUENCE LISTING

<110> KYOWA HAKKO KOGYO CO., LTD.

<120> Protein-free medium adapted FUT8 knouck out cells

<130> 11620WO1

<150> JP2003-350166
<151> 2003-10-09

<160> 32

<170> PatentIn Ver. 2.1

<210> 1
<211> 2008
<212> DNA
<213> Cricetulus griseus

<400> 1
aacagaaact tattttcctg tgtggctaac tagaaccaga gtacaatgtt tccaattctt 60

tgagctccga gaagacagaa gggagttgaa actctgaaaa tgcgggcatg gactggttcc 120

tggcgttgga ttatgctcat tctttttgcc tgggggacct tattgtttta tataggtggt 180

catttggttc gagataatga ccaccctgac cattctagca gagaactctc caagattctt 240

gcaaagctgg agcgcttaaa acaacaaaat gaagacttga ggagaatggc tgagtctctc 300

cgaataccag aaggccctat tgatcagggg acagctacag gaagagtccg tgttttagaa 360

gaacagcttg ttaaggccaa agaacagatt gaaaattaca agaaacaagc taggaatgat 420

ctgggaaagg atcatgaaat cttaaggagg aggattgaaa atggagctaa agagctctgg 480

tttttctac aaagtgaatt gaagaaatta aagaaattag aaggaaacga actccaaaga 540

catgcagatg aaattctttt ggatttagga catcatgaaa ggtctatcat gacagatcta 600

tactacctca gtcaaacaga tggagcaggt gagtggcggg aaaaagaagc caaagatctg 660

acagagctgg tccagcggag aataacatat ctgcagaatc ccaaggactg cagcaaagcc 720

agaaagctgg tatgtaatat caacaaaggc tgtggctatg gatgtcaact ccatcatgtg 780

gtttactgct tcatgattgc ttatggcacc cagcgaacac tcatcttgga atctcagaat 840

tggcgctatg ctactggagg atgggagact gtgtttagac ctgtaagtga gacatgcaca 900

gacaggtctg gcctctccac tggacactgg tcaggtgaag tgaaggacaa aaatgttcaa 960

gtggtcgagc tccccattgt agacagcctc catcctcgtc ctccttactt acccttggct 1020

gtaccagaag accttgcaga tcgactcctg agagtccatg gtgatcctgc agtgtggtgg 1080

gtatcccagt ttgtcaaata cttgatccgt ccacaacctt ggctggaaag ggaaatagaa 1140

gaaaccacca agaagcttgg cttcaaacat ccagttattg gagtccatgt cagacgcact 1200

gacaaagtgg gaacagaagc agccttccat cccattgagg aatacatggt acacgttgaa 1260

gaacattttc agcttctcga acgcagaatg aaagtggata aaaaaagagt gtatctggcc 1320

actgatgacc cttctttgtt aaaggaggca aagacaaagt actccaatta tgaatttatt 1380

agtgataact ctatttcttg gtcagctgga ctacacaacc gatacacaga aaattcactt 1440

cggggcgtga tcctggatat acactttctc tcccaggctg acttccttgt gtgtactttt 1500

tcatcccagg tctgtagggt tgcttatgaa atcatgcaaa cactgcatcc tgatgcctct 1560

gcaaacttcc attctttaga tgacatctac tattttggag gccaaaatgc ccacaaccag 1620

attgcagttt atcctcacca acctcgaact aaagaggaaa tccccatgga acctggagat 1680

atcattggtg tggctggaaa ccattggaat ggttactcta aaggtgtcaa cagaaaacta 1740

ggaaaaacag gcctgtaccc ttcctacaaa gtccgagaga agatagaaac agtcaaatac 1800

cctacatatc ctgaagctga aaaatagaga tggagtgtaa gagattaaca acagaattta 1860

gttcagacca tctcagccaa gcagaagacc cagactaaca tatggttcat tgacagacat 1920

gctccgcacc aagagcaagt gggaaccctc agatgctgca ctggtggaac gcctctttgt 1980

gaagggctgc tgtgccctca agcccatg 2008

<210> 2
<211> 1728
<212> DNA
<213> Mus musculus

<400> 2
atgcgggcat ggactggttc ctggcgttgg attatgctca ttctttttgc ctgggggacc 60

ttgttatttt atataggtgg tcatttggtt cgagataatg accaccctga tcactccagc 120

agagaactct ccaagattct tgcaaagctt gaacgcttaa aacagcaaaa tgaagacttg 180

aggcgaatgg ctgagtctct ccgaatacca gaaggcccca ttgaccaggg gacagctaca 240

ggaagagtcc gtgttttaga agaacagctt gttaaggcca aagaacagat tgaaaattac 300

aagaaacaag ctagaaatgg tctggggaag gatcatgaaa tcttaagaag gaggattgaa 360

aatggagcta aagagctctg gttttttcta caaagcgaac tgaagaaatt aaagcattta 420
gaaggaaatg aactccaaag acatgcagat gaaattcttt tggatttagg acaccatgaa 480
aggtctatca tgacagatct atactacctc agtcaaacag atggagcagg ggattggcgt 540
gaaaaagagg ccaaagatct gacagagctg gtccagcgga gaataacata tctccagaat 600
cctaaggact gcagcaaagc caggaagctg gtgtgtaaca tcaataaagg ctgtggctat 660
ggttgtcaac tccatcacgt ggtctactgt ttcatgattg cttatggcac ccagcgaaca 720
ctcatcttgg aatctcagaa ttggcgctat gctactggtg gatgggagac tgtgtttaga 780
cctgtaagtg agacatgtac agacagatct ggcctctcca ctggacactg gtcaggtgaa 840
gtaaatgaca aaaacattca agtggtcgag ctccccattg tagacagcct ccatcctcgg 900
cctccttact taccactggc tgttccagaa gaccttgcag accgactcct aagagtccat 960
ggtgaccctg cagtgtggtg ggtgtcccag tttgtcaaat acttgattcg tccacaacct 1020
tggctggaaa aggaaataga agaagccacc aagaagcttg gcttcaaaca tccagttatt 1080
ggagtccatg tcagacgcac agacaaagtg ggaacagaag cagccttcca ccccatcgag 1140
gagtacatgg tacacgttga agaacatttt cagcttctcg cacgcagaat gcaagtggat 1200
aaaaaaagag tatatctggc tactgatgat cctactttgt taaaggaggc aaagacaaag 1260
tactccaatt atgaatttat tagtgataac tctatttctt ggtcagctgg actacacaat 1320
cggtacacag aaaattcact tcggggtgtg atcctggata tacactttct ctcacaggct 1380
gactttctag tgtgtacttt ttcatcccag gtctgtcggg ttgcttatga aatcatgcaa 1440

accctgcatc ctgatgcctc tgcgaacttc cattctttgg atgacatcta ctattttgga 1500

ggccaaaatg cccacaatca gattgctgtt tatcctcaca aacctcgaac tgaagaggaa 1560

attccaatgg aacctggaga tatcattggt gtggctggaa accattggga tggttattct 1620

aaaggtatca acagaaaact tggaaaaaca ggcttatatc cctcctacaa agtccgagag 1680

aagatagaaa cagtcaagta tcccacatat cctgaagctg aaaaatag 1728

<210> 3
<211> 3677
<212> DNA
<213> Homo sapiens (GenBank Accesion # : NM_178156)

<400> 3
cgtttagtac agaaatctca tgggagagag catccatgca tttacaaatt gttattgaat 60

tattttattg aatgatgaca cccaaactga gctagaacat aattctggct ctgctagtac 120

atcttctgtg tgatcttgga caagtcactc tactttcctt tcaattttct tttctcacag 180

ggagataatc ataaaaacga ctgtaaagta cagcacttca tagagtgctt tttgtttaaa 240

gagctgacaa taaatacgag tctcaaggtc taggaaagcc tccctcacaa cctgagctgc 300

ttgaggacaa gggattttct tttgaatcag cagtacctta tttgtgtatc tgtgatagag 360

ttcctggtac ataagaaggt ctcaataaat atgtgaattt atgaatatta ggcagattgc 420

aaccttgaca ggccactgcc tcttaaatct cctttctgtg atcttttaat atttaacatc 480

taaaaggccg ccgctacttg ctttgggata agtatccccg gtatgtactt taaaatgccc 540

aagcctagag aaatgattct tgtcttaagg gcaccatttc gctctcccac cgtaaagcgc 600

cccaggcttg ggatctgggt cccaaggcta cagggaagag tttggaacgg gaagctcatc 660

ttccggccct ctgattggcc ggctcgcact ccactcacgc ggcgcgcagc tctgattggc 720

ctcggcggca cccctcgtcc cgcgactact ttgtgtgctg gggcggcgcg ctccggtcct 780

cccgctcagc tggcggtctg ggctgctctg gggcagccct tcggtccact gctctgcatc 840

gcgggcgccg ggaattttcc gagtccgagc ggcatgtaga gcgcatgaag tacaggacaa 900

taaagcttcc tacacatatc accaggagga tctctttgaa agattcactg caggactacc 960

agagagaata atttgtctga agcatcatgt gttgaaacaa cagaagtcta ttcacctgtg 1020

cactaactag aaacagagtt acaatgtttt caattctttg agctccagga ctccagggaa 1080

gtgagttgaa aatctgaaaa tgcggccatg gactggttcc tggcgttgga ttatgctcat 1140

tctttttgcc tgggggacct tgctgtttta tataggtggt cacttggtac gagataatga 1200

ccatcctgat cactctagcc gagaactgtc caagattctg gcaaagcttg aacgcttaaa 1260

acaacagaat gaagacttga ggcgaatggc cgaatctctc cggataccag aaggccctat 1320

tgatcagggg ccagctatag gaagagtacg cgttttagaa gagcagcttg ttaaggccaa 1380

agaacagatt gaaaattaca agaaacagac cagaaatggt ctggggaagg atcatgaaat 1440

cctgaggagg aggattgaaa atggagctaa agagctctgg tttttcctac agagtgaatt 1500

gaagaaatta aagaacttag aaggaaatga actccaaaga catgcagatg aatttctttt 1560

ggatttagga catcatgaaa ggtctataat gacggatcta tactacctca gtcagacaga 1620

tggagcaggt gattggcggg aaaaagaggc caaagatctg acagaactgg ttcagcggag 1680

aataacatat cttcagaatc ccaaggactg cagcaaagcc aaaaagctgg tgtgtaatat 1740

caacaaaggc tgtggctatg gctgtcagct ccatcatgtg gtctactgct tcatgattgc 1800

atatggcacc cagcgaacac tcatcttgga atctcagaat tggcgctatg ctactggtgg 1860

atgggagact gtatttaggc ctgtaagtga gacatgcaca gacagatctg gcatctccac 1920

tggacactgg tcaggtgaag tgaaggacaa aaatgttcaa gtggtcgagc ttcccattgt 1980

agacagtctt catccccgtc ctccatattt acccttggct gtaccagaag acctcgcaga 2040

tcgacttgta cgagtgcatg gtgaccctgc agtgtggtgg gtgtctcagt ttgtcaaata 2100

cttgatccgc ccacagcctt ggctagaaaa agaaatagaa gaagccacca agaagcttgg 2160

cttcaaacat ccagttattg gagtccatgt cagacgcaca gacaaagtgg gaacagaagc 2220

tgccttccat cccattgaag agtacatggt gcatgttgaa gaacattttc agcttcttgc 2280

acgcagaatg caagtggaca aaaaaagagt gtatttggcc acagatgacc cttctttatt 2340

aaaggaggca aaaacaaagt accccaatta tgaatttatt agtgataact ctatttcctg 2400

gtcagctgga ctgcacaatc gatacacaga aaattcactt cgtggagtga tcctggatat 2460

acattttctc tctcaggcag acttcctagt gtgtactttt tcatcccagg tctgtcgagt 2520

tgcttatgaa attatgcaaa cactacatcc tgatgcctct gcaaacttcc attctttaga 2580

tgacatctac tattttgggg gccagaatgc ccacaatcaa attgccattt atgctcacca 2640

accccgaact gcagatgaaa ttcccatgga acctggagat atcattggtg tggctggaaa 2700

tcattgggat ggctattcta aaggtgtcaa caggaaattg ggaaggacgg gcctatatcc 2760

ctcctacaaa gttcgagaga agatagaaac ggtcaagtac cccacatatc ctgaggctga 2820

gaaataaagc tcagatggaa gagataaacg accaaactca gttcgaccaa actcagttca 2880

aaccatttca gccaaactgt agatgaagag ggctctgatc taacaaaata aggttatatg 2940

agtagatact ctcagcacca agagcagctg ggaactgaca taggcttcaa ttggtggaat 3000

tcctctttaa caagggctgc aatgccctca tacccatgca cagtacaata atgtactcac 3060

atataacatg caaacaggtt gtttctact ttgcccctt cagtatgtcc ccataagaca 3120

aacactgcca tattgtgtaa tttaagtgac acagacattt tgtgtgagac ttaaaacatg 3180

gtgcctatat ctgagagacc tgtgtgaact attgagaaga tcggaacagc tccttactct 3240

gaggaagttg attcttattt gatggtggta ttgtgaccac tgaattcact ccagtcaaca 3300

gattcagaat gagaatggac gtttggtttt tttttgtttt tgtttttgtt ttttccttta 3360

taaggttgtc tgttttttt ttttaaata attgcatcag ttcattgacc tcatcattaa 3420

taagtgaaga atacatcaga aaataaaata ttcactctcc attagaaaat tttgtaaaac 3480

aatgccatga acaaattctt tagtactcaa tgtttctgga cattctcttt gataacaaaa 3540

aataaatttt aaaaaggaat tttgtaaagt ttctagaatt ttatatcatt ggatgatatg 3600

ttgatcagcc ttatgtggaa gaactgtgat aaaaagagga gctttttagt tttcagctt 3660

aaaaaaaaaa aaaaaaa 3677

<210> 4

<211> 1836
<212> DNA
<213> Sus scrofa (GenBank Accesion # : D86723.1)

<400> 4
atgttttcaa ttctttgagc tctaggaagc cacgaaagtg agttgaaagt ctgaaaatgc 60

ggccatggac tggttcgtgg cgttggatta tgctcattct ttttgcctgg gggaccttgc 120

tattttacat aggtggtcac ttggtacgag ataatgacca ctctgatcac tctagccgag 180

aactgtccaa gattttggca aagctggaac gcttaaaaca acaaaatgaa gacttgagga 240

gaatggctga atctctccga ataccagaag gccccattga tcaggggcca gcttcaggaa 300

gagttcgtgc tttagaagag caatttatga aggccaaaga acagattgaa aattataaga 360

aacaaactaa aaatggtcca gggaaggatc atgaaatcct aaggaggagg attgaaaatg 420

gagctaaaga gctctggttt tttctacaaa gtgagttgaa gaaattaaag aatttagaag 480

gaaatgaact ccaaagacat gcagatgaat ttctatcaga tttgggacat catgaaaggt 540

ctataatgac ggatctatac tacctcagtc aaacagatgg ggcaggtgat tggcgtgaaa 600

aggaggccaa agatctgaca gagctggtcc agcggagaat aacatatctt cagaatccca 660

aggactgcag caaagccaag aagctagtgt gtaatatcaa caaaggctgt ggctatggct 720

gtcagctcca tcatgtagtg tactgcttta tgattgcata tggcacccag cgaacactcg 780

ccttggaatc tcacaattgg cgctacgcta ctgggggatg ggaaactgtg tttagacctg 840

taagtgagac gtgcacagac agatctggca gctccactgg acattggtca ggtgaagtaa 900

aggacaaaaa tgttcaggtg gttgagctcc ccattgtaga cagtgttcat cctcgtcctc 960

catatttacc cctggctgtc ccagaagacc ttgcagatcg acttgtacga gtccatggtg 1020
atcctgcagt gtggtgggta tcccagtttg tcaagtactt gattcgccca caaccctggc 1080
tggaaaagga aatagaagag gccaccaaga agctaggctt caaacatcca gttattggag 1140
tccatgttag acgcacagac aaagtgggag cggaagcagc cttccatccc attgaggaat 1200
acacggtgca cgttgaagaa gactttcagc ttcttgctcg cagaatgcaa gtggataaaa 1260
aaagggtgta tttggccaca gatgaccctg ctttgttaaa agaggcaaaa acaaagtacc 1320
ccagttatga atttattagt gataactcta tctcttggtc agctggacta cataatcgat 1380
atacagaaaa ttcacttcgg ggtgtgatcc tggatataca ctttctctcc caggcagact 1440
tcctagtgtg tactttttca tcgcaggtct gtagagttgc ttatgaaatc atgcaagcgc 1500
tgcatcctga tgcctctgcg aacttccgtt ctttggatga catctactat tttggaggcc 1560
caaatgccca caaccaaatt gccatttatc ctcaccaacc tcgaactgaa ggagaaatcc 1620
ccatggaacc tggagatatt attggtgtgg ctggaaatca ctgggatggc tatcctaaag 1680
gtgttaacag aaaactggga aggacgggcc tatatccctc ctacaaagtt cgagagaaga 1740
tagaaacagt caagtacccc acatatcccg aggctgacaa gtaaagcttg gacggacaga 1800
tgagaaagac aaccaaactc agttcaaacc atttga 1836

<210> 5
<211> 575
<212> PRT
<213> Cricetulus griseus

<400> 5

Met Arg Ala Trp Thr Gly Ser Trp Arg Trp Ile Met Leu Ile Leu Phe
1 5 10 15

Ala Trp Gly Thr Leu Leu Phe Tyr Ile Gly Gly His Leu Val Arg Asp
20 25 30

Asn Asp His Pro Asp His Ser Ser Arg Glu Leu Ser Lys Ile Leu Ala
35 40 45

Lys Leu Glu Arg Leu Lys Gln Gln Asn Glu Asp Leu Arg Arg Met Ala
50 55 60

Glu Ser Leu Arg Ile Pro Glu Gly Pro Ile Asp Gln Gly Thr Ala Thr
65 70 75 80

Gly Arg Val Arg Val Leu Glu Glu Gln Leu Val Lys Ala Lys Glu Gln
85 90 95

Ile Glu Asn Tyr Lys Lys Gln Ala Arg Asn Asp Leu Gly Lys Asp His
100 105 110

Glu Ile Leu Arg Arg Arg Ile Glu Asn Gly Ala Lys Glu Leu Trp Phe
115 120 125

Phe Leu Gln Ser Glu Leu Lys Lys Leu Lys Lys Leu Glu Gly Asn Glu
130 135 140

Leu Gln Arg His Ala Asp Glu Ile Leu Leu Asp Leu Gly His His Glu
145 150 155 160

Arg Ser Ile Met Thr Asp Leu Tyr Tyr Leu Ser Gln Thr Asp Gly Ala
165 170 175

Gly Glu Trp Arg Glu Lys Glu Ala Lys Asp Leu Thr Glu Leu Val Gln

180 185 190

Arg Arg Ile Thr Tyr Leu Gln Asn Pro Lys Asp Cys Ser Lys Ala Arg
195 200 205

Lys Leu Val Cys Asn Ile Asn Lys Gly Cys Gly Tyr Gly Cys Gln Leu
210 215 220

His His Val Val Tyr Cys Phe Met Ile Ala Tyr Gly Thr Gln Arg Thr
225 230 235 240

Leu Ile Leu Glu Ser Gln Asn Trp Arg Tyr Ala Thr Gly Gly Trp Glu
245 250 255

Thr Val Phe Arg Pro Val Ser Glu Thr Cys Thr Asp Arg Ser Gly Leu
260 265 270

Ser Thr Gly His Trp Ser Gly Glu Val Lys Asp Lys Asn Val Gln Val
275 280 285

Val Glu Leu Pro Ile Val Asp Ser Leu His Pro Arg Pro Pro Tyr Leu
290 295 300

Pro Leu Ala Val Pro Glu Asp Leu Ala Asp Arg Leu Leu Arg Val His
305 310 315 320

Gly Asp Pro Ala Val Trp Trp Val Ser Gln Phe Val Lys Tyr Leu Ile
325 330 335

Arg Pro Gln Pro Trp Leu Glu Arg Glu Ile Glu Glu Thr Thr Lys Lys
340 345 350

Leu Gly Phe Lys His Pro Val Ile Gly Val His Val Arg Arg Thr Asp
355 360 365

Lys Val Gly Thr Glu Ala Ala Phe His Pro Ile Glu Glu Tyr Met Val

370 375 380

His Val Glu Glu His Phe Gln Leu Leu Glu Arg Arg Met Lys Val Asp
385 390 395 400

Lys Lys Arg Val Tyr Leu Ala Thr Asp Asp Pro Ser Leu Leu Lys Glu
405 410 415

Ala Lys Thr Lys Tyr Ser Asn Tyr Glu Phe Ile Ser Asp Asn Ser Ile
420 425 430

Ser Trp Ser Ala Gly Leu His Asn Arg Tyr Thr Glu Asn Ser Leu Arg
435 440 445

Gly Val Ile Leu Asp Ile His Phe Leu Ser Gln Ala Asp Phe Leu Val
450 455 460

Cys Thr Phe Ser Ser Gln Val Cys Arg Val Ala Tyr Glu Ile Met Gln
465 470 475 480

Thr Leu His Pro Asp Ala Ser Ala Asn Phe His Ser Leu Asp Asp Ile
485 490 495

Tyr Tyr Phe Gly Gly Gln Asn Ala His Asn Gln Ile Ala Val Tyr Pro
500 505 510

His Gln Pro Arg Thr Lys Glu Glu Ile Pro Met Glu Pro Gly Asp Ile
515 520 525

Ile Gly Val Ala Gly Asn His Trp Asn Gly Tyr Ser Lys Gly Val Asn
530 535 540

Arg Lys Leu Gly Lys Thr Gly Leu Tyr Pro Ser Tyr Lys Val Arg Glu
545 550 555 560

Lys Ile Glu Thr Val Lys Tyr Pro Thr Tyr Pro Glu Ala Glu Lys

565 570 575

<210> 6
<211> 575
<212> PRT
<213> Mus musculus

<400> 6
Met Arg Ala Trp Thr Gly Ser Trp Arg Trp Ile Met Leu Ile Leu Phe
1 5 10 15

Ala Trp Gly Thr Leu Leu Phe Tyr Ile Gly Gly His Leu Val Arg Asp
20 25 30

Asn Asp His Pro Asp His Ser Ser Arg Glu Leu Ser Lys Ile Leu Ala
35 40 45

Lys Leu Glu Arg Leu Lys Gln Gln Asn Glu Asp Leu Arg Arg Met Ala
50 55 60

Glu Ser Leu Arg Ile Pro Glu Gly Pro Ile Asp Gln Gly Thr Ala Thr
65 70 75 80

Gly Arg Val Arg Val Leu Glu Glu Gln Leu Val Lys Ala Lys Glu Gln
85 90 95

Ile Glu Asn Tyr Lys Lys Gln Ala Arg Asn Gly Leu Gly Lys Asp His
100 105 110

Glu Ile Leu Arg Arg Arg Ile Glu Asn Gly Ala Lys Glu Leu Trp Phe
115 120 125

Phe Leu Gln Ser Glu Leu Lys Lys Leu Lys His Leu Glu Gly Asn Glu
130 135 140

Leu Gln Arg His Ala Asp Glu Ile Leu Leu Asp Leu Gly His His Glu
145 150 155 160

Arg Ser Ile Met Thr Asp Leu Tyr Tyr Leu Ser Gln Thr Asp Gly Ala
165 170 175

Gly Asp Trp Arg Glu Lys Glu Ala Lys Asp Leu Thr Glu Leu Val Gln
180 185 190

Arg Arg Ile Thr Tyr Leu Gln Asn Pro Lys Asp Cys Ser Lys Ala Arg
195 200 205

Lys Leu Val Cys Asn Ile Asn Lys Gly Cys Gly Tyr Gly Cys Gln Leu
210 215 220

His His Val Val Tyr Cys Phe Met Ile Ala Tyr Gly Thr Gln Arg Thr
225 230 235 240

Leu Ile Leu Glu Ser Gln Asn Trp Arg Tyr Ala Thr Gly Gly Trp Glu
245 250 255

Thr Val Phe Arg Pro Val Ser Glu Thr Cys Thr Asp Arg Ser Gly Leu
260 265 270

Ser Thr Gly His Trp Ser Gly Glu Val Asn Asp Lys Asn Ile Gln Val
275 280 285

Val Glu Leu Pro Ile Val Asp Ser Leu His Pro Arg Pro Pro Tyr Leu
290 295 300

Pro Leu Ala Val Pro Glu Asp Leu Ala Asp Arg Leu Leu Arg Val His
305 310 315 320

Gly Asp Pro Ala Val Trp Trp Val Ser Gln Phe Val Lys Tyr Leu Ile
325 330 335

Arg Pro Gln Pro Trp Leu Glu Lys Glu Ile Glu Glu Ala Thr Lys Lys
340 345 350

Leu Gly Phe Lys His Pro Val Ile Gly Val His Val Arg Arg Thr Asp
355 360 365

Lys Val Gly Thr Glu Ala Ala Phe His Pro Ile Glu Glu Tyr Met Val
370 375 380

His Val Glu Glu His Phe Gln Leu Leu Ala Arg Arg Met Gln Val Asp
385 390 395 400

Lys Lys Arg Val Tyr Leu Ala Thr Asp Asp Pro Thr Leu Leu Lys Glu
405 410 415

Ala Lys Thr Lys Tyr Ser Asn Tyr Glu Phe Ile Ser Asp Asn Ser Ile
420 425 430

Ser Trp Ser Ala Gly Leu His Asn Arg Tyr Thr Glu Asn Ser Leu Arg
435 440 445

Gly Val Ile Leu Asp Ile His Phe Leu Ser Gln Ala Asp Phe Leu Val
450 455 460

Cys Thr Phe Ser Ser Gln Val Cys Arg Val Ala Tyr Glu Ile Met Gln
465 470 475 480

Thr Leu His Pro Asp Ala Ser Ala Asn Phe His Ser Leu Asp Asp Ile
485 490 495

Tyr Tyr Phe Gly Gly Gln Asn Ala His Asn Gln Ile Ala Val Tyr Pro
500 505 510

His Lys Pro Arg Thr Glu Glu Glu Ile Pro Met Glu Pro Gly Asp Ile
515 520 525

Ile Gly Val Ala Gly Asn His Trp Asp Gly Tyr Ser Lys Gly Ile Asn
530 535 540

Arg Lys Leu Gly Lys Thr Gly Leu Tyr Pro Ser Tyr Lys Val Arg Glu
545 550 555 560

Lys Ile Glu Thr Val Lys Tyr Pro Thr Tyr Pro Glu Ala Glu Lys
565 570 575

<210> 7
<211> 446
<212> PRT
<213> Homo sapiens

<400> 7
Met Ala Ile Thr Val Ser Leu Val Asn Asn Lys Arg Lys Ile Val Val
1 5 10 15

Leu Ala Gln Pro Thr Thr Val Lys Arg Lys Arg Ile Thr Pro Tyr Lys
20 25 30

Ser Ile Met Thr Asp Leu Tyr Tyr Leu Ser Gln Thr Asp Gly Ala Gly
35 40 45

Asp Trp Arg Glu Lys Glu Ala Lys Asp Leu Thr Glu Leu Val Gln Arg
50 55 60

Arg Ile Thr Tyr Leu Gln Asn Pro Lys Asp Cys Ser Lys Ala Lys Lys
165 70 75 80

Leu Val Cys Asn Ile Asn Lys Gly Cys Gly Tyr Gly Cys Gln Leu His
85 90 95

His Val Val Tyr Cys Phe Met Ile Ala Tyr Gly Thr Gln Arg Thr Leu
100 105 110

Ile Leu Glu Ser Gln Asn Trp Arg Tyr Ala Thr Gly Gly Trp Glu Thr
115 120 125

Val Phe Arg Pro Val Ser Glu Thr Cys Thr Asp Arg Ser Gly Ile Ser
130 135 140

Thr Gly His Trp Ser Gly Glu Val Lys Asp Lys Asn Val Gln Val Val
145 150 155 160

Glu Leu Pro Ile Val Asp Ser Leu His Pro Arg Pro Pro Tyr Leu Pro
165 170 175

Leu Ala Val Pro Glu Asp Leu Ala Asp Arg Leu Val Arg Val His Gly
180 185 190

Asp Pro Ala Val Trp Trp Val Ser Gln Phe Val Lys Tyr Leu Ile Arg
195 200 205

Pro Gln Pro Trp Leu Glu Lys Glu Ile Glu Glu Ala Thr Lys Lys Leu
210 215 220

Gly Phe Lys His Pro Val Ile Gly Val His Val Arg Arg Thr Asp Lys
225 230 235 240

Val Gly Thr Glu Ala Ala Phe His Pro Ile Glu Glu Tyr Met Val His
245 250 255

Val Glu Glu His Phe Gln Leu Leu Ala Arg Arg Met Gln Val Asp Lys
260 265 270

Lys Arg Val Tyr Leu Ala Thr Asp Asp Pro Ser Leu Leu Lys Glu Ala
275 280 285

Lys Thr Lys Tyr Pro Asn Tyr Glu Phe Ile Ser Asp Asn Ser Ile Ser
290 295 300

Trp Ser Ala Gly Leu His Asn Arg Tyr Thr Glu Asn Ser Leu Arg Gly
305 310 315 320

Val Ile Leu Asp Ile His Phe Leu Ser Gln Ala Asp Phe Leu Val Cys
325 330 335

Thr Phe Ser Ser Gln Val Cys Arg Val Ala Tyr Glu Ile Met Gln Thr
340 345 350

Leu His Pro Asp Ala Ser Ala Asn Phe His Ser Leu Asp Asp Ile Tyr
355 360 365

Tyr Phe Gly Gly Gln Asn Ala His Asn Gln Ile Ala Ile Tyr Ala His
370 375 380

Gln Pro Arg Thr Ala Asp Glu Ile Pro Met Glu Pro Gly Asp Ile Ile
385 390 395 400

Gly Val Ala Gly Asn His Trp Asp Gly Tyr Ser Lys Gly Val Asn Arg
405 410 415

Lys Leu Gly Arg Thr Gly Leu Tyr Pro Ser Tyr Lys Val Arg Glu Lys
420 425 430

Ile Glu Thr Val Lys Tyr Pro Thr Tyr Pro Glu Ala Glu Lys
435 440 445

<210> 8
<211> 575
<212> PRT
<213> Sus scrofa

<400> 8
Met Arg Pro Trp Thr Gly Ser Trp Arg Trp Ile Met Leu Ile Leu Phe

1 5 10 15

Ala Trp Gly Thr Leu Leu Phe Tyr Ile Gly Gly His Leu Val Arg Asp
20 25 30

Asn Asp His Ser Asp His Ser Ser Arg Glu Leu Ser Lys Ile Leu Ala
35 40 45

Lys Leu Glu Arg Leu Lys Gln Gln Asn Glu Asp Leu Arg Arg Met Ala
50 55 60

Glu Ser Leu Arg Ile Pro Glu Gly Pro Ile Asp Gln Gly Pro Ala Ser
165 70 75 80

Gly Arg Val Arg Ala Leu Glu Glu Gln Phe Met Lys Ala Lys Glu Gln
85 90 95

Ile Glu Asn Tyr Lys Lys Gln Thr Lys Asn Gly Pro Gly Lys Asp His
100 105 110

Glu Ile Leu Arg Arg Arg Ile Glu Asn Gly Ala Lys Glu Leu Trp Phe
115 120 125

Phe Leu Gln Ser Glu Leu Lys Lys Leu Lys Asn Leu Glu Gly Asn Glu
130 135 140

Leu Gln Arg His Ala Asp Glu Phe Leu Ser Asp Leu Gly His His Glu
145 150 155 160

Arg Ser Ile Met Thr Asp Leu Tyr Tyr Leu Ser Gln Thr Asp Gly Ala
165 170 175

Gly Asp Trp Arg Glu Lys Glu Ala Lys Asp Leu Thr Glu Leu Val Gln
180 185 190

Arg Arg Ile Thr Tyr Leu Gln Asn Pro Lys Asp Cys Ser Lys Ala Lys

195 200 205

Lys Leu Val Cys Asn Ile Asn Lys Gly Cys Gly Tyr Gly Cys Gln Leu
210 215 220

His His Val Val Tyr Cys Phe Met Ile Ala Tyr Gly Thr Gln Arg Thr
225 230 235 240

Leu Ala Leu Glu Ser His Asn Trp Arg Tyr Ala Thr Gly Gly Trp Glu
245 250 255

Thr Val Phe Arg Pro Val Ser Glu Thr Cys Thr Asp Arg Ser Gly Ser
260 265 270

Ser Thr Gly His Trp Ser Gly Glu Val Lys Asp Lys Asn Val Gln Val
275 280 285

Val Glu Leu Pro Ile Val Asp Ser Val His Pro Arg Pro Pro Tyr Leu
290 295 300

Pro Leu Ala Val Pro Glu Asp Leu Ala Asp Arg Leu Val Arg Val His
305 310 315 320

Gly Asp Pro Ala Val Trp Trp Val Ser Gln Phe Val Lys Tyr Leu Ile
325 330 335

Arg Pro Gln Pro Trp Leu Glu Lys Glu Ile Glu Glu Ala Thr Lys Lys
340 345 350

Leu Gly Phe Lys His Pro Val Ile Gly Val His Val Arg Arg Thr Asp
355 360 365

Lys Val Gly Ala Glu Ala Ala Phe His Pro Ile Glu Glu Tyr Thr Val
370 375 380

His Val Glu Glu Asp Phe Gln Leu Leu Ala Arg Arg Met Gln Val Asp

385 390 395 400

Lys Lys Arg Val Tyr Leu Ala Thr Asp Asp Pro Ala Leu Leu Lys Glu
405 410 415

Ala Lys Thr Lys Tyr Pro Ser Tyr Glu Phe Ile Ser Asp Asn Ser Ile
420 425 430

Ser Trp Ser Ala Gly Leu His Asn Arg Tyr Thr Glu Asn Ser Leu Arg
435 440 445

Gly Val Ile Leu Asp Ile His Phe Leu Ser Gln Ala Asp Phe Leu Val
450 455 460

Cys Thr Phe Ser Ser Gln Val Cys Arg Val Ala Tyr Glu Ile Met Gln
465 470 475 480

Ala Leu His Pro Asp Ala Ser Ala Asn Phe Arg Ser Leu Asp Asp Ile
485 490 495

Tyr Tyr Phe Gly Gly Pro Asn Ala His Asn Gln Ile Ala Ile Tyr Pro
500 505 510

His Gln Pro Arg Thr Glu Gly Glu Ile Pro Met Glu Pro Gly Asp Ile
515 520 525

Ile Gly Val Ala Gly Asn His Trp Asp Gly Tyr Pro Lys Gly Val Asn
530 535 540

Arg Lys Leu Gly Arg Thr Gly Leu Tyr Pro Ser Tyr Lys Val Arg Glu
545 550 555 560

Lys Ile Glu Thr Val Lys Tyr Pro Thr Tyr Pro Glu Ala Asp Lys
565 570 575

<210> 9
<211> 9196
<212> DNA
<213> Cricetulus griseus

<400> 9
tctagaccag gctggtctcg aactcacaga gaaccacctg cctctgccac ctgagtgctg 60

ggattaaagg tgtgcaccac caccgcccgg cgtaaaatca tatttttgaa tattgtgata 120

atttacatta taattgtaag taaaaatttt cagcctattt tgttatacat ttttgcgtaa 180

attattcttt tttgaaagtt ttgttgtcca taatagtcta gggaaacata aagttataat 240

ttttgtctat gtatttgcat atatatctat ttaatctcct aatgtccagg aaataaatag 300

ggtatgtaat agcttcaaca tgtggtatga tagaattttt cagtgctata taagttgtta 360

cagcaaagtg ttattaattc atatgtccat atttcaattt tttatgaatt attaaattga 420

atccttaagc tgccagaact agaattttat tttaatcagg aagccccaaa tctgttcatt 480

ctttctatat atgtggaaag gtaggcctca ctaactgatt cttcacctgt tttagaacat 540

ggtccaagaa tggagttatg taaggggaat tacaagtgtg agaaaactcc tagaaaacaa 600

gatgagtctt gtgaccttag tttctttaaa aacacaaaat tcttggaatg tgttttcatg 660

ttcctcccag gtggatagga gtgagtttat ttcagattat ttattacaac tggctgttgt 720

tacttgtttc tatgtcttta tagaaaaaca tattttttt gccacatgca gcttgtcctt 780

atgattttat acttgtgtga ctcttaactc tcagagtata aattgtctga tgctatgaat 840

aaagttggct attgtatgag acttcagccc acttcaatta ttggcttcat tctctcagat 900

cccaccacct ccagagtggt aaacaacttg aaccattaaa cagactttag tctttatttg 960

aatgatagat ggggatatca gatttatagg cacagggttt tgagaaaggg agaaggtaaa 1020

cagtagagtt taacaacaac aaaaagtata ctttgtaaac gtaaaactat ttattaaagt 1080

agtagacaag acattaaata ttccttggga ttagtgcttt ttgaattttg ctttcaaata 1140

atagtcagtg agtatacccc tcccccattc tatattttag cagaaatcag aataaatggt 1200

gtttctggta cattcttttg tagagaattt attttctttg ggtttttgtg catttaaagt 1260

caataaaaat taaggttcag taatagaaaa aaaactctga tttttggaat ccccttttctt 1320

cagcttttct atttaatctc ttaatgataa tttaatttgt ggccatgtgg tcaaagtata 1380

tagccttgta tatgtaaatg ttttaaccaa cctgccttta cagtaactat ataattttat 1440

tctataatat atgacttttc ttccatagct ttagagttgc ccagtcactt taagttacat 1500

tttcatatat gttctttgtg ggaggagata attttatttc taagagaatc ctaagcatac 1560

tgattgagaa atggcaaaca aaacacataa ttaaagctga taaagaacga acatttggag 1620

tttaaaatac atagccaccc taagggttta actgttgtta gccttctttt ggaattttta 1680

ttagttcata tagaaaaatg gattttatcg tgacatttcc atatatgtat ataatatatt 1740

tacatcatat ccacctgtaa ttattagtgt ttttaaatat atttgaaaaa ataatggtct 1800

ggtttgatcc atttgaacct tttgatgttt ggtgtggttg ccaattggtt gatggttatg 1860

ataacctttg cttctctaag gttcaagtca gtttgagaat atgtcctcta aaaatgacag 1920

gttgcaagtt aagtagtgag atgacagcga gatggagtga tgagaatttg tagaaatgaa 1980

ttcacttata ctgagaactt gttttgcttt tagataatga acatattagc ctgaagtaca 2040

tagccgaatt gattaattat tcaaagatat aatcttttaa tccctataaa agaggtatta 2100

cacaacaatt caagaaagat agaattagac ttccagtatt ggagtgaacc atttgttatc 2160

aggtagaacc ctaacgtgtg tggttgactt aaagtgttta ctttttacct gatactgggt 2220

agctaattgt ctttcagcct cctggccaaa gataccatga aagtcaactt acgttgtatt 2280

ctatatctca aacaactcag ggtgtttctt actctttcca cagcatgtag agcccaggaa 2340

gcacaggaca agaaagctgc ctccttgtat caccaggaag atctttttgt aagagtcatc 2400

acagtatacc agagagacta attttgtctg aagcatcatg tgttgaaaca acagaaactt 2460

attttcctgt gtggctaact agaaccagag tacaatgttt ccaattcttt gagctccgag 2520

aagacagaag ggagttgaaa ctctgaaaat gcgggcatgg actggttcct ggcgttggat 2580

tatgctcatt ctttttgcct gggggacctt attgttttat ataggtggtc atttggttcg 2640

agataatgac caccctgacc attctagcag agaactctcc aagattcttg caaagctgga 2700

gcgcttaaaa caacaaaatg aagacttgag gagaatggct gagtctctcc ggtaggtttg 2760

aaatactcaa ggatttgatg aaatactgtg cttgaccttt aggtataggg tctcagtctg 2820

ctgttgaaaa atataatttc tacaaaccgt ctttgtaaaa ttttaagtat tgtagcagac 2880

tttttaaaag tcagtgatac atctatatag tcaatatagg tttacatagt tgcaatctta 2940

ttttgcatat gaatcagtat atagaagcag tggcatttat atgcttatgt tgcatttaca 3000

attatgttta gacgaacaca aactttatgt gatttggatt agtgctcatt aaattttttt 3060

attctatgga ctacaacaga gacataaatt ttgaaaggct tagttactct taaattctta 3120

tgatgaaaag caaaaattca ttgttaaata gaacagtgca tccggaatgt gggtaattat 3180

tgccatattt ctagtctact aaaaattgtg gcataactgt tcaaagtcat cagttgtttg 3240

gaaagccaaa gtctgattta aatggaaaac ataaacaatg atatctattt ctagatacct 3300

ttaacttgca gttactgagt ttacaagttg tctgacaact ttggattctc ttacttcata 3360

tctaagaatg atcatgtgta cagtgcttac tgtcacttta aaaaactgca gggctagaca 3420

tgcagatatg aagactttga cattagatgt ggtaattggc actaccagca agtggtatta 3480

agatacagct gaatatatta ctttttgagg aacataattc atgaatggaa agtggagcat 3540

tagagaggat gccttctggc tctcccacac cactgtttgc atccattgca tttcacactg 3600

cttttagaac tcagatgttt catatggtat attgtgtaac tcaccatcag ttttatcttt 3660

aaatgtctat ggatgataat gttgtatgtt aacactttta caaaaacaaa tgaagccata 3720

tcctcggtgt gagttgtgat ggtggtaatt gtcacaatag gattattcag caaggaacta 3780

agtcagggac aagaagtggg cgatactttg ttggattaaa tcattttact ggaagttcat 3840

cagggagggt tatgaaagtt gtggtctttg aactgaaatt atatgtgatt cattattctt 3900

gatttaggcc ttgctaatag taactatcat ttattgggaa tttgtcatat gtgccaattt 3960

gtcatgggcc agacagcgtg ttttactgaa tttctagata tctttatgag attctagtac 4020

tgttttcagc cattttacag atgaagaatc ttaaaaaatg ttaaataatt tagtttgccc 4080

aagattatac gttaacaaat ggtagaacct tctttgaatt ctggcagtat ggctacacag 4140

tccgaactct tatcttccta agctgaaaac agaaaaagca atgacccaga aaattttatt 4200

taaaagtctc aggagagact tcccatcctg agaagatctc ttttcccttt tataatttag 4260

gctcctgaat aatcactgaa ttttctccat gttccatcta tagtactgtt atttctgttt 4320

tcctttttc ttaccacaaa gtatcttgtt tttgctgtat gaaagaaaat gtgttattgt 4380

aatgtgaaat tctctgtccc tgcagggtcc cacatccgcc tcaatcccaa ataaacacac 4440

agaggctgta ttaattatga aactgttggt cagttggcta gggcttctta ttggctagct 4500

ctgtcttaat tattaaacca taactactat tgtaagtatt tccatgtggt cttatcttac 4560

caaggaaagg gtccagggac ctcttactcc tctggcgtgt tggcagtgaa gaggagagag 4620

cgatttccta tttgtctctg cttattttct gattctgctc agctatgtca cttcctgcct 4680

ggccaatcag ccaatcagtg ttttattcat tagccaataa aagaaacatt tacacagaag 4740

gacttccccc atcatgttat ttgtatgagt tcttcagaaa atcatagtat cttttaatac 4800

taatttttat aaaaaattaa ttgtattgaa aattatgtgt atatgtgtct gtgtgtcgat 4860

ttgtgctcat aagtagcatg gagtgcagaa gagggaatca gatctttttt taagggacaa 4920

agagtttatt cagattacat tttaaggtga taatgtatga ttgcaaggtt atcaacatgg 4980

cagaaatgtg aagaagctgg tcacattaca tccagagtca agagtagaga gcaatgaatt 5040

gatgcatgca ttcctgtgct cagctcactt ttcctggagc tgagctgatt gtaagccatc 5100

tgatgtcttt gctgggaact aactcaaagg caagttcaaa acctgttctt aagtataagc 5160

catctctcca gtccctcata tggtctctta agacactttc tttatattct tgtacataga 5220

aattgaattc ctaacaactg cattcaaatt acaaaatagt ttttaaaagc tgatataata 5280

aatgtaaata caatctagaa catttttata aataagcata ttaactcagt aaaaataaat 5340

gcatggttat tttccttcat tagggaagta tgtctcccca ggctgttctc tagattctac 5400

tagtaatgct gtttgtacac catccacagg ggttttattt taaagctaag acatgaatga 5460

tggacatgct tgttagcatt tagacttttt tccttactat aattgagcta gtatttttgt 5520

gctcagtttg atatctgtta attcagataa atgtaatagt aggtaatttc tttgtgataa 5580

aggcatataa attgaagttg gaaaacaaaa gcctgaaatg acagttttta agattcagaa 5640

caataatttt caaaagcagt tacccaactt tccaaataca atctgcagtt ttcttgatat 5700

gtgataaatt tagacaaaga aatagcacat tttaaaatag ctatttactc ttgatttttt 5760

tttcaaattt aggctagttc actagttgtg tgtaaggtta tggctgcaaa catctttgac 5820

tcttggttag ggaatccagg atgatttacg tgtttggcca aaatcttgtt ccattctggg 5880

tttcttctct atctaggtag ctagcacaag ttaaaggtgt ggtagtattg gaaggctctc 5940

aggtatatat ttctatattc tgtatttttt tcctctgtca tatatttgct ttctgtttta 6000

ttgatttcta ctgttagttt gatacttact ttcttacact ttctttggga tttattttgc 6060

tgttctaaga tttcttagca agttcatatc actgatttta acagttgctt cttttgtaat 6120

atagactgaa tgccccttat ttgaaatgct tgggatcaga aactcagatt tgaacttttc 6180

tttttaaata tttccatcaa gtttaccagc tgaatgtcct gatccaagaa tatgaaatct 6240

gaaatgcttt gaaatctgaa acttttagag tgataaagct tccctttaaa ttaatttgtg 6300

ttctatattt tttgacaatg tcaacctttc attgttatcc aatgagtgaa catattttca 6360

attttttgt ttgatctgtt atattttgat ctgaccatat ttataaaatt ttatttaatt 6420

tgaatgttgt gctgttactt atctttatta ttatttttgc ttattttcta gccaaatgaa 6480

attatattct gtattatttt agtttgaatt ttactttgtg gcttagtaac tgccttttgt 6540

tggtgaatgc ttaagaaaaa cgtgtggtct actgatattg gttctaatct tatatagcat 6600

gttgtttgtt aggtagttga ttatgctggt cagattgtct tgagtttatg caaatgtaaa 6660

atatttagat gcttgttttg ttgtctaaga acaaagtatg cttgctgtct cctatcggtt 6720

ctggttttc cattcatctc ttcaagctgt tttgtgtgtt gaatactaac tccgtactat 6780

cttgttttct gtgaattaac cccttttcaa aggtttcttt tcttttttt tttaagggac 6840

aacaagttta ttcagattac attttaagct gataatgtat gattgcaagg ttatcaacat 6900

ggcagaaatg tgaagaagct aggcacatta catccacatg gagtcaagag cagagagcag 6960

tgaattaatg catgcattcc tgtggtcagc tcacttttcc tattcttaga tagtctagga 7020

tcataaacct ggggaatagt gctaccacaa tgggcatatc cacttacttc agttcatgca 7080

atcaaccaag gcacatccac aggaaaaact gatttagaca acctctcatt gagactcttc 7140

ccagatgatt agactgtgtc aagttgacaa ttaaaactat cacacctgaa gccatcacta 7200

gtaaatataa tgaaaatgtt gattatcacc ataattcatc tgtatccctt tgttattgta 7260

gatttgtgga agttcctatt caagtccctg ttccttcctt aaaaacctgt tttttagtta 7320

aataggtttt ttagtgttcc tgtctgtaaa tacttttta aagttagata ttattttcaa 7380

gtatgttctc ccagtctttg gcttgtattt tcatcccttc aatacatata tttttgtaat 7440

ttattttttt tatttaaatt agaaacaaag ctgcttttac atgtcagtct cagttccctc 7500

tccctcccct cctcccctgc tccccaccta agccccaatt ccaactcctt tcttctcccc 7560

aggaagggtg aggccctcca tgggggaaat cttcaatgtc tgtcatatca tttggagcag 7620

ggcctagacc ctccccagtg tgtctaggct gagagagtat ccctctatgt ggagagggct 7680

cccaaagttc atttgtgtac taggggtaaa tactgatcca ctatcagtgg ccccatagat 7740

tgtccggacc tccaaactga cttcctcctt cagggagtct ggaacagttc tatgctggtt 7800

tcccagatat cagtctgggg tccatgagca accccttgtt caggtcagtt gtttctgtag 7860

gtttccccag cccggtcttg accccttgc tcatcacttc tccctctctg caactggatt 7920

ccagagttca gctcagtgtt tagctgtggg tgtctgcatc tgcttccatc agctactgga 7980

tgagggctct aggatggcat ataaggtagt catcagtctc attatcagag aagggctttt 8040

aaggtagcct cttgattatt gcttagattg ttagttgggg tcaaccttgt aggtctctgg 8100

acagtgacag aattctcttt aaacctataa tggctccctc tgtggtggta tccctttct 8160

tgctctcatc cgttcctccc ctgactagat cttcctgctc cctcatgtcc tcctctcccc 8220

tccccttctc cccttctctt tcttctaact ccctctcccc tccacccacg atccccatta 8280

gcttatgaga tcttgtcctt attttagcaa aacctttttg gctataaaat taattaattt 8340

aatatgctta tatcaggttt attttggcta gtatttgtat gtgtttggtt agtgttttta 8400

accttaattg acatgtatcc ttatatttag acacagattt aaatatttga agttttttt 8460

tttttttttt ttaaagattt atttattttt tatgtcttct gcctgcatgc cagaagaggg 8520

caccagatct cattcaaggt ggttgtgagc caccatgtgg ttgctgggaa ttgaactcag 8580

gacctctgga agaacagtca gtgctcttaa ccgctgagcc atctctccag cccctgaagt 8640

gtttctttta aagaggatag cagtgcatca tttttccctt tgaccaatga ctcctacctt 8700

actgaattgt tttagccatt tatatgtaat gctgttacca ggtttacatt ttcttttatc 8760

ttgctaaatt tcttccctgt ttgtctcatc tcttattttt gtctgttgga ttatataggc 8820

ttttattttt ctgtttttac agtaagttat atcaaattaa aattatttta tggaatgggt 8880

gtgttgacta catgtatgtc tgtgcaccat gtgctgacct ggtcttggcc agaagaaggt 8940

gtcatattct ctgaaactgg tattgtggat gttacgaact gccatagggt gctaggaatc 9000

aaaccccagc tcctctggaa aagcagccac tgctctgagc cactgagtcc tctcttcaag 9060

caggtgatgc caacttttaa tggttaccag tggataagag tgcttgtatc tctagcaccc 9120

atgaaaattt atgcattgct atatgggctt gtcacttcag cattgtgtga cagagacagg 9180

aggatcccaa gagctc 9196

<210> 10
<211> 28
<212> DNA
<213> Artificial Sequence

<220>
<223> Description of Artificial Sequence: Synthetic DNA

<400> 10

gagacttcag cccacttcaa ttattggc 28

<210> 11
<211> 25
<212> DNA
<213> Artificial Sequence

<220>
<223> Description of Artificial Sequence: Synthetic DNA

<400> 11
cttgtgtgac tcttaactct cagag 25

<210> 12
<211> 25
<212> DNA
<213> Artificial Sequence

<220>
<223> Description of Artificial Sequence: Synthetic DNA

<400> 12
gaggccactt gtgtagcgcc aagtg 25

<210> 13
<211> 23
<212> DNA
<213> Artificial Sequence

<220>
<223> Description of Artificial Sequence: Synthetic DNA

<400> 13

ccctcgagat aacttcgtat agc 23

<210> 14
<211> 18
<212> DNA
<213> Artificial Sequence

<220>
<223> Description of Artificial Sequence : Synthetic DNA

<400> 14
ggtaggcctc actaactg 18

<210> 15
<211> 25
<212> DNA
<213> Artificial Sequence

<220>
<223> Description of Artificial Sequence : Synthetic DNA

<400> 15
catagaaaca agtaacaaca gccag 25

<210> 16
<211> 21
<212> DNA
<213> Artificial Sequence

<220>
<223> Description of Artificial Sequence: Synthetic DNA

<400> 16

gtgagtccat ggctgtcact g 21

<210> 17
<211> 20
<212> DNA
<213> Artificial Sequence

<220>
<223> Description of Artificial Sequence: Synthetic DNA

<400> 17
cctgacttgg ctattctcag 20

<210> 18
<211> 384
<212> DNA
<213> Mus musculus

<400> 18
```
atg gat ttt cag gtg cag att atc agc ttc ctg cta atc agt gct tca    48
Met Asp Phe Gln Val Gln Ile Ile Ser Phe Leu Leu Ile Ser Ala Ser
  1               5                  10                  15
gtc ata atg tcc aga gga caa att gtt ctc tcc cag tct cca gca atc    96
Val Ile Met Ser Arg Gly Gln Ile Val Leu Ser Gln Ser Pro Ala Ile
             20                  25                  30
ctg tct gca tct cca ggg gag aag gtc aca atg act tgc agg gcc agc    144
Leu Ser Ala Ser Pro Gly Glu Lys Val Thr Met Thr Cys Arg Ala Ser
         35                  40                  45
tca agt gta agt tac atc cac tgg ttc cag cag aag cca gga tcc tcc    192
Ser Ser Val Ser Tyr Ile His Trp Phe Gln Gln Lys Pro Gly Ser Ser
     50                  55                  60
ccc aaa ccc tgg att tat gcc aca tcc aac ctg gct tct gga gtc cct    240
Pro Lys Pro Trp Ile Tyr Ala Thr Ser Asn Leu Ala Ser Gly Val Pro
 65                  70                  75                  80
```

gtt cgc ttc agt ggc agt ggg tct ggg act tct tac tct ctc acc atc 288
Val Arg Phe Ser Gly Ser Gly Ser Gly Thr Ser Tyr Ser Leu Thr Ile
85 90 95
agc aga gtg gag gct gaa gat gct gcc act tat tac tgc cag cag tgg 336
Ser Arg Val Glu Ala Glu Asp Ala Ala Thr Tyr Tyr Cys Gln Gln Trp
100 105 110
act agt aac cca ccc acg ttc gga ggg ggg acc aag ctg gaa atc aaa 384
Thr Ser Asn Pro Pro Thr Phe Gly Gly Gly Thr Lys Leu Glu Ile Lys
115 120 125

<210> 19
<211> 420
<212> DNA
<213> Mus musculus

<400> 19
atg ggt tgg agc ctc atc ttg ctc ttc ctt gtc gct gtt gct acg cgt 48
Met Gly Trp Ser Leu Ile Leu Leu Phe Leu Val Ala Val Ala Thr Arg
1 5 10 15
gtc ctg tcc cag gta caa ctg cag cag cct ggg gct gag ctg gtg aag 96
Val Leu Ser Gln Val Gln Leu Gln Gln Pro Gly Ala Glu Leu Val Lys
20 25 30
cct ggg gcc tca gtg aag atg tcc tgc aag gct tct ggc tac aca ttt 144
Pro Gly Ala Ser Val Lys Met Ser Cys Lys Ala Ser Gly Tyr Thr Phe
35 40 45
acc agt tac aat atg cac tgg gta aaa cag aca cct ggt cgg ggc ctg 192
Thr Ser Tyr Asn Met His Trp Val Lys Gln Thr Pro Gly Arg Gly Leu
50 55 60
gaa tgg att gga gct att tat ccc gga aat ggt gat act tcc tac aat 240
Glu Trp Ile Gly Ala Ile Tyr Pro Gly Asn Gly Asp Thr Ser Tyr Asn
65 70 75 80
cag aag ttc aaa ggc aag gcc aca ttg act gca gac aaa tcc tcc agc 288
Gln Lys Phe Lys Gly Lys Ala Thr Leu Thr Ala Asp Lys Ser Ser Ser
85 90 95
aca gcc tac atg cag ctc agc agc ctg aca tct gag gac tct gcg gtc 336

Thr Ala Tyr Met Gln Leu Ser Ser Leu Thr Ser Glu Asp Ser Ala Val
            100                     105                     110
tat tac tgt gca aga tcg act tac tac ggc ggt gac tgg tac ttc aat    384
Tyr Tyr Cys Ala Arg Ser Thr Tyr Tyr Gly Gly Asp Trp Tyr Phe Asn
        115                     120                     125
gtc tgg ggc gca ggg acc acg gtc acc gtc tct gca                    420
Val Trp Gly Ala Gly Thr Thr Val Thr Val Ser Ala
    130                     135                     140

<210> 20
<211> 91
<212> DNA
<213> Artificial Sequence

<220>
<223> Description of Artificial Sequence: Synthetic DNA

<400> 20
caggaaacag ctatgacgaa ttcgcctcct caaaatggat tttcaggtgc agattatcag 60
cttcctgcta atcagtgctt cagtcataat g                                91

<210> 21
<211> 91
<212> DNA
<213> Artificial Sequence

<220>
<223> Description of Artificial Sequence: Synthetic DNA

<400> 21
gtgaccttct cccctggaga tgcagacagg attgctggag actgggagag aacaatttgt 60
cctctggaca ttatgactga agcactgatt a                                91

<210> 22
<211> 90
<212> DNA
<213> Artificial Sequence

<220>
<223> Description of Artificial Sequence: Synthetic DNA

<400> 22
ctccagggga gaaggtcaca atgacttgca gggccagctc aagtgtaagt tacatccact 60
ggttccagca gaagccagga tcctccccca 90

<210> 23
<211> 89
<212> DNA
<213> Artificial Sequence

<220>
<223> Description of Artificial Sequence: Synthetic DNA

<400> 23
ccagacccac tgccactgaa gcgaacaggg actccagaag ccaggttgga tgtggcataa 60
atccagggtt tgggggagga tcctggctt 89

<210> 24
<211> 91
<212> DNA
<213> Artificial Sequence

<220>
<223> Description of Artificial Sequence: Synthetic DNA

<400> 24
tcagtggcag tgggtctggg acttcttact ctctcaccat cagcagagtg gaggctgaag 60

atgctgccac ttattactgc cagcagtgga c 91

<210> 25
<211> 90
<212> DNA
<213> Artificial Sequence

<220>
<223> Description of Artificial Sequence: Synthetic DNA

<400> 25
gttttcccag tcacgaccgt acgtttgatt tccagcttgg tcccccctcc gaacgtgggt 60
gggttactag tccactgctg gcagtaataa 90

<210> 26
<211> 99
<212> DNA
<213> Artificial Sequence

<220>
<223> Description of Artificial Sequence: Synthetic DNA

<400> 26
caggaaacag ctatgacgcg gccgcgaccc ctcaccatgg gttggagcct catcttgctc 60
ttccttgtcg ctgttgctac gcgtgtcctg tcccaggta 99

<210> 27
<211> 98
<212> DNA
<213> Artificial Sequence

<220>
<223> Description of Artificial Sequence: Synthetic DNA

<400> 27
atgtgtagcc agaagccttg caggacatct tcactgaggc cccagccttc accagctcag 60
ccccaggctg ctgcagttgt acctgggaca ggacacgc 98

<210> 28
<211> 97
<212> DNA
<213> Artificial Sequence

<220>
<223> Description of Artificial Sequence: Synthetic DNA

<400> 28
caaggcttct ggctacacat ttaccagtta caatatgcac tgggtaaaac agacacctgg 60
tcggggcctg gaatggattg gagctattta tcccgga 97

<210> 29
<211> 99
<212> DNA
<213> Artificial Sequence

<220>
<223> Description of Artificial Sequence: Synthetic DNA

<400> 29
gtaggctgtg ctggaggatt tgtctgcagt caatgtggcc ttgcctttga acttctgatt 60
gtaggaagta tcaccatttc cgggataaat agctccaat 99

<210> 30
<211> 99
<212> DNA
<213> Artificial Sequence

<220>
<223> Description of Artificial Sequence: Synthetic DNA

<400> 30
aatcctccag cacagcctac atgcagctca gcagcctgac atctgaggac tctgcggtct 60
attactgtgc aagatcgact tactacggcg gtgactggt 99

<210> 31
<211> 98
<212> DNA
<213> Artificial Sequence

<220>
<223> Description of Artificial Sequence: Synthetic DNA

<400> 31
gttttcccag tcacgacggg cccttggtgg aggctgcaga gacggtgacc gtggtccctg 60
cgccccagac attgaagtac cagtcaccgc cgtagtaa 98

<210> 32
<211> 25
<212> DNA
<213> Artificial Sequence

<220>
<223> Description of Artificial Sequence: Synthetic DNA

<400> 32
gagctggtga agcctggggc ctcag 25